# EUROPÄISCHE PATENTSCHRIFT

(11) **EP 3 016 986 B1**
(45) Veröffentlichungstag und Bekanntmachung des Hinweises auf die Patenterteilung: **03.06.2020**
(21) Anmeldenummer: 14734186.1
(22) Anmeldetag: 02.07.2014
(51) Int. Cl.: C08F 220/06, C08F 283/06, C08F 120/06, C08F 2/24, C08F 2/44, C09D 133/02, C09J 133/02, C11D 3/37, C08L 71/02, C08L 33/02

(54) **GELFÖRMIGE POLYMERZUSAMMENSETZUNG, ERHALTEN DURCH POLYMERISATION EINES SÄUREGRUPPENHALTIGEN MONOMERS IN GEGENWART EINER POLYETHERVERBINDUNG**
POLYMER COMPOSITION, OBTAINED BY POLYMERISATION OF A MONOMER CONTAINING ACID GROUPS IN THE PRESENCE OF A POLYETHER COMPOUND
COMPOSITION POLYMÈRE OBTENUE PAR POLYMÉRISATION D'UN MONOMÈRE CONTENANT DES GROUPES ACIDES EN PRÉSENCE D'UN COMPOSÉ POLYÉTHER

(30) Priorität: 03.07.2013 EP 13174938
(43) Veröffentlichungstag der Anmeldung: 11.05.2016
(73) Patentinhaber: BASF SE, 67056 Ludwigshafen am Rhein (DE)
(72) Erfinder: FUCHS, Yannick, 69469 Weinheim (DE); WITTELER, Helmut, 67157 Wachenheim (DE); WEBER, Heike, 68239 Mannheim (DE)
(86) Internationale Anmeldenummer: PCT/EP2014/064086
(87) Internationale Veröffentlichungsnummer: WO 2015/000971

(56) Entgegenhaltungen:
- WO-A1-2005/012378
- WO-A1-2012/069440
- LEV BROMBERG: "Polyether-Modified Poly(acrylic acid): Synthesis and Applications", INDUSTRIAL & ENGINEERING CHEMISTRY RESEARCH, Bd. 37, Nr. 11, 1. November 1998 (1998-11-01), Seiten 4267-4274, XP055139606, ISSN: 0888-5885, DOI: 10.1021/ie980358s
- LEV BROMBERG: "Novel Family of Thermogelling Materials via C-C Bonding between Poly(acrylic acid) and Poly(ethylene oxide)- b -poly(propylene oxide)- b -poly(ethylene oxide)", THE JOURNAL OF PHYSICAL CHEMISTRY B, Bd. 102, Nr. 11, 20. Februar 1998 (1998-02-20), Seiten 1956-1963, XP055113924, ISSN: 1520-6106, DOI: 10.1021/jp9803687

## Beschreibung

### HINTERGRUND DER ERFINDUNG

Die vorliegende Erfindung betrifft ein Verfahren zur Herstellung einer gelförmigen Polymerzusammensetzung, bei dem man eine α,β-ethylenisch ungesättigte Carbonsäure einer radikalischen Polymerisation in Gegenwart wenigstens einer Polyetherkomponente unterzieht, die nach diesem Verfahren erhältliche gelförmige Polymerzusammensetzung und deren Verwendung.

### STAND DER TECHNIK

In Haushaltsreinigern, Waschmitteln, Kosmetika und in Formulierungen für technische Anwendungen werden häufig Polyether oder Tenside zusammen mit Polyacrylsäure eingesetzt, wobei der Polyacrylsäure beispielsweise die Rolle eines Inkrustierungsinhibitors oder Dispergiermittels zukommt. Es besteht das Problem, dass Polyether und Tenside mit Polyacrylsäure in gelförmigen Formulierungen häufig nicht oder nur bedingt verträglich sind, so dass es beim Mischen zur Phasenseparation oder zu Ausfällungen kommt, was die Einsatzmöglichkeiten wenigstens einer der Komponenten stark einschränkt. Es ist insbesondere bislang nicht gelungen, transparente Gelformulierungen bereitzustellen, die Polyetherole oder Polyethergruppen-haltige Tenside in Kombination mit säuregruppenhaltigen Polymeren und speziell mit Polyacrylsäure enthalten. Gelförmige Formulierungen werden jedoch vom Verbraucher sowohl aufgrund ihrer anwendungstechnischen Eigenschaften, wie auch aus ästhetischen Gründen bevorzugt.

Für viele aktuelle Anwendungen müssen gelbildende oder filmbildende Polymerzusammensetzungen ein komplexes Anforderungsprofil erfüllen.

Aus der Literatur sind zahlreiche Verfahren zur Herstellung von Gelen aus Polyacrylsäure bekannt. Diese Gele sind zumeist aber nicht in Wasser löslich, da sie auf vernetzter Polyacylsäure basieren. Andere Gele sind zwar wasserlöslich, basieren aber auf Copolymeren der Acrylsäure mit hydrophoben Monomeren und sind deshalb als Inkrustierungsinhibitoren oder Dispergiermittel weniger leistungsfähig als reine Polyacrylsäure. Weitere Gele basieren auf hochmolekularer Polyacrylsäure, was aber für die Anwendung als Inkrustierungsinhibitor oder Dispergiermittel ebenfalls nicht vorteilhaft ist und die Probleme bei der Verarbeitung noch verstärkt.

F. E. Bailey et al. beschreiben in Polymer Preprints, American Chemical Society, Division of Polymer Chemistry, 1960, vol. 1, issue 2, p. 202 - 205 und der darin zitierten Literatur die Bildung von molekularen Assoziationskomplexen von Ethylenoxid-Polymeren, die ein sehr hohes Molekulargewicht aufweisen, mit polymeren Säuren, wie Polyacrylsäure, in wässrigen Lösungen. Zur Herstellung fester Gele ist es erforderlich, die Komponenten in Lösungsmitteln auflösen, dann zu mischen und das Lösungsmittel wieder zu entfernen, was mit zusätzlichen Prozessschritten verbunden ist, die ein solches Verfahren für den praktischen Gebrauch unwirtschaftlich machen. Die Polymerisation von Acrylsäure in Gegenwart der Ethylenoxid-Polymere ist nicht beschrieben.

In der EP 0971997 B1 wird eine flüssige Reinigerformulierung beschrieben, die ein nichtionisches Tenside und ein anionisches Polymer enthält. Bei dem nichtionischen Tensid kann es sich um einen ethoxilierten C₈-C₁₈-Alkohol und bei dem anionischen Polymer um Polyacrylsäure handeln. Das Polymer weist ein Molekulargewicht von mehr als 100 000 g/mol auf. Es wird nicht beschrieben, zur Herstellung der Formulierung wenigstens ein säuregruppenhaltiges Polymer in Gegenwart des nichtionischen Tensids zu polymerisieren.

Die EP 0 499 068 A1 beschreibt Reaktionsprodukte aus Alkoxilaten und vinylischen Monomeren, wobei diese zumindest teilweise funktionelle Gruppen tragen, die mit den OH-Gruppen der Alkoxilate in einer Kondensation reagieren können. Zur Herstellung dieser Reaktionsprodukte polymerisiert man entweder die vinylischen Monomere in Gegenwart der Alkoxilate und unterzieht das Produkt der Polymerisation anschließend einer Kondensation oder man polymerisiert zuerst die vinylischen Monomere und unterzieht anschließend das Produkt der Polymerisation einer Kondensation mit den Alkoxilaten. Im Falle das Acrylsäure als vinylisches Monomer eingesetzt wird, ist das Reaktionsprodukt somit in jedem Fall ein Ester der Polyacrylsäure. In den Ausführungsbeispielen werden als Alkoxilate ausschließlich EO-PO-Blockcopolymere mit hohem PO-Gehalt und Polytetrahydrofuran eingesetzt. Die beschriebenen Polymere dienen als Emulsionsspalter zur Schnellentwässerung von Rohölen. Ihre Herstellung erfolgt in einer ersten Variante durch Massepolymerisation, wobei das Endprodukt als erstarrte Schmelze oder nach Aufnahme in einem Lösungsmittel der Rohölemulsion zugesetzt wird. In einer zweiten Variante erfolgt die Herstellung durch Lösungspolymerisation in einem für den Einsatz als Emulsionsspalter geeigneten Lösungsmittel. Geeignete Lösungsmittel sind Aromaten, aliphatische und cycloaliphatische Kohlenwasserstoffe, Aceton, Cyclohexanon, THF und Dioxan. Gelförmige Polymere und deren Verwendung sind nicht beschrieben.

Ein ähnliches Verfahren wird in der DE 4326772 A1 beschrieben. Hier wird zusätzlich Toluol oder Xylol als Lösungsmittel verwendet. Die Verwendung von aromatischen Lösungsmitteln ist für Produkte, die in Konsumgütern eingesetzt werden sollen, unerwünscht, da die vollständige Entfernung des Lösemittels sehr zeit- und energieaufwendig ist. Die Reaktionsprodukte sind Flüssigkeiten, die als veresterte Polyacrylsäuren beschrieben werden. Esterbildung läuft der Wirkung des Polymers, beispielsweise als Inkrustierungsinhibitor zuwider und ist nicht erwünscht in Anwendungen, in denen der Einsatz möglichst reiner Polyacrylsäure gefordert ist. auch in diesem Dokument ist ein Einsatz in gelförmigen Formulierungen nicht beschrieben.

X. Li et al. beschreiben im Journal of Solid State Electrochemistry (2011), 15(6), 1271-1277) und J. H. Wu et al. beschreiben in Advanced Materials, 2007, 19, 4006-4011, Herstellungsverfahren für Polyacrylsäure-Polyethylenglykol-Gele für den Einsatz in Farbstoff-sensitivierten Solarzellen. Diese Publikationen lehren den Einsatz von N, N'-Methylenbisacrylamid als Vernetzer bei der Acrylsäurepolymerisation, so dass die erhaltenen Polymere nicht wasserlöslich sind. Außerdem wird durch den Einsatz eines Vernetzers ein sehr hohes Molekulargewicht erhalten, was die Polymere für viele Anwendungen, z.B. als Inkrustierungsinhibitoren ungeeignet macht.

In der WO 2008/139151 A1 wird ein Verfahren beschrieben, in dem Polyethylenglyol mit Acrylsäure, Isobornylacrylat und weiteren Komponente gemischt und durch UV-Belichtung zu einem festen Gel ausgehärtet ist. Aufgrund der Zusammensetzung ist für den Fachmann ersichtlich, dass das Gel nicht wasserlöslich ist. Die Gele dienen als Indikator, dass ein Datenträger, z. B. eine computerlesbare Compactdisc, nicht zuvor benutzt wurde.

In der WO 2010/026178/A2 wird auf Seite 62 und in Beispiel 19 eine Fällungspolymerisation beschrieben, bei der Acrylsäure in Gegenwart von Glycerinmonostearat und einem Alkyl-terminierten Polyethylenglycolmethacrylat polymerisiert wird. Letzteres ist ein Assoziativmonomer und kein Polyethergruppenhaltiges Tensid im Sinne der vorliegenden Erfindung. Das Verfahren erfordert zudem den Einsatz einer großen Menge an organischen Lösungsmitteln bezogen auf relativ wenig Acrylsäure und Tensid.

Lev Bromberg beschreibt im Journal of Physical Chemistry B (1998), 102, 11, 1956-1963, ein Material mit thermoreversibler Gelbildung, zu dessen Herstellung man Acrylsäure in Gegenwart eines PEO-PPO-PEO-Blockcopolymers polymerisiert. Die Reaktion erfolgt in Abwesenheit externer Lösungsmittel, um einen hohen Anteil an Verzweigung und Vernetzung in den erhaltenen Produkten zu erzielen. Diese sind weder wasserlöslich noch transparent. Als mögliche Einsatzbereiche für diese Polymere werden nur ganz allgemein die Pharmazie und die Nahrungsergänzung genannt (S. 1956, linke Spalte, "Introduction").

EP 0 639 592 A1 beschreibt Pfropfcopolymere, die durch Polymerisation einer (Meth)acrylsäure-haltigen Polymerzusammensetzung in Gegenwart einer Polyetherverbindung mit mehr als 80 Mol-% Ethylenoxideinheiten erhältlich sind. Die Polymerisation erfolgt im Wesentlichen ohne Lösungsmittel und bei Temperaturen oberhalb von 100 °C. Es wird als kritisch für die Erzielung hoher Pfropfgrade angesehen, dass der Lösungsmittelgehalt des Reaktionsgemischs keinesfalls mehr als 5 Gew.-% beträgt. Die erhaltenen Polymere dienen als Builder für Flüssigwaschmittel oder, gegebenenfalls nach einer Nachvernetzung, als wasserabsorbierende Harze.

WO 2004/099274 beschreibt ein Verfahren zur Herstellung von Polymermischungen durch Polymerisation von Monomeren vom (Meth)acrylsäuretyp in Gegenwart einer Verbindung mit einer Polyalkylenglycol-Struktur. Die erhaltenen Polymermischungen sollen Anteile von Pfropfcopolymeren enthalten, wobei auch nach längerer Lagerung die Polyalkylenglycol-Komponente und die (Meth)acrylsäure-Komponente homogen in der Mischung verbleiben. Die Polymerisation erfolgt zwingend in Gegenwart von Wasser mit einem hohen Wassergehalt in der Vorlage zu Beginn der Reaktion. Die nach dem Verfahren erhaltenen Polymermischungen eignen sich für diverse Detergenzanwendungen, speziell zur Wiederanschmutzverhinderung.

WO 2005/012378 beschreibt wässrige Dispersionen von wasserlöslichen Polymeren von anionischen Monomeren und deren Verwendung als Verdickungsmittel für wässrige Systeme. Zu ihrer Herstellung werden anionische Monomere in Gegenwart von zwei wasserlöslichen Polymeren aus verschiedenen Klassen polymerisiert, wobei es sich unter anderem auch um Polyalkylenglycole handeln kann. Beispiel 4 (Seite 19, Zeilen 14-27) betrifft die Polymerisation von Acrylsäure in Gegenwart von zwei verschiedenen Polypropylenglycolen und von Maltodextrin. Die Dispersionen werden unter Anderem in Personal care-Produkten sowie in Wasch- und Reinigungsmitteln eingesetzt. Gelförmige Produkte sind nicht beschrieben.

Der vorliegenden Erfindung liegt die Aufgabe zugrunde, eine gelförmige Polymerzusammensetzung und ein Verfahren zur ihrer Herstellung bereitzustellen, wobei die zuvor beschriebenen Nachteile des Stands der Technik vermieden werden.

Überraschenderweise wurde nun gefunden, dass diese Aufgabe gelöst wird, wenn zur Herstellung der Polymerzusammensetzung eine Monomerzusammensetzung auf Basis wenigstens einer α,β-ethylenisch ungesättigten Carbonsäure einer radikalischen Polymerisation in Gegenwart einer Polyetherkomponente unterzogen wird.

### ZUSAMMENFASSUNG DER ERFINDUNG

Ein erster Gegenstand der Erfindung ist ein Verfahren zur Herstellung einer gelförmigen Polymerzusammensetzung, bei dem man
a) eine Monomerzusammensetzung M) bereitstellt, die
   A) wenigstens eine α,β-ethylenisch ungesättigte Carbonsäure, und
   B) weniger als 0,1 Gew.-%, bezogen auf das Gesamtgewicht der Monomerzusammensetzung M) vernetzend wirkende Monomere, die zwei oder mehr als zwei polymerisierbare α,β-ethylenisch ungesättigte Doppelbindungen pro Molekül aufweisen,
   enthält,
b) die in Schritt a) bereitgestellte Monomerzusammensetzung M) einer radikalischen Polymerisation in Gegenwart wenigstens einer Polyetherkomponente PE) unterzieht, die ausgewählt ist unter Polyetherolen mit einem zahlenmittleren Molekulargewicht von wenigstens 200 g/mol und deren Mono- und Di-(C₁-C₆-alkylethern), Polyethergruppen-haltigen Tensiden und Mischungen davon, wobei, wenn die Polyetherkomponente PE) ein Polyetherol mit Propylenoxid-Wiederholungseinheiten oder einen Mono- oder Di-(C1-C6 -alkylether) eines Polyetherols mit Propylenoxid-Wiederholungseinheiten umfasst, der Anteil dieser Propylenoxid-Wiederholungseinheiten im Mittel höchstens 15 Einheiten pro Molekül beträgt, wobei die Polyetherole PE) Ethylenoxid-Homopolymere oder Ethylenoxid/Propylenoxid-Copolymere sind.

In einer speziellen Ausführungsform ist die wenigstens eine α,β-ethylenisch ungesättigte Carbonsäure A) teilweise oder vollständig durch wenigstens eine ungesättigte Sulfonsäure oder wenigstens eine ungesättigte Phosphonsäure (= Komponente C) ersetzt.

In einer speziellen Ausführungsform umfasst die Polyetherkomponente PE) wenigstens ein Polyetherol oder einen Mono- oder Di-(C₁-C₂-alkylether) davon oder besteht die Polyetherkomponente PE) aus wenigstens einem Polyetherol oder einem Mono- oder Di-(C₁-C₂-alkylether) davon, das als Alkylenoxideinheiten überwiegend oder ausschließlich Ethylenoxideinheiten eingebaut enthält.

Wenn die Polyetherkomponente PE) ein Polyetherol mit Propylenoxid-Wiederholungseinheiten oder einen Mono- oder Di-(C₁-C₆-alkylether) eines Polyetherols mit Propylenoxid-Wiederholungseinheiten umfasst, beträgt der Anteil dieser Propylenoxid-Wiederholungseinheiten im Mittel höchstens 15 Einheiten pro Molekül, bevorzugt höchstens 10 Einheiten pro Molekül.

In einer speziellen Ausführung des erfindungsgemäßen Verfahrens erfolgt die radikalische Polymerisation in Schritt b) zusätzlich in Gegenwart eines Lösungsmittels LM), das ausgewählt ist unter Wasser, C₁-C₆-Alkanolen, von PE) verschiedenen Polyolen, deren Mono- und Dialkylethern, aprotisch polaren Lösungsmitteln und Mischungen davon. In einer spezielleren Ausführung des erfindungsgemäßen Verfahrens erfolgt die radikalische Polymerisation in Schritt b) zusätzlich in Gegenwart eines Lösungsmittels LM), das ausgewählt ist unter Wasser, C₁-C₆-Alkanolen, von PE) verschiedenen Polyolen, deren Mono- und Dialkylethern und Mischungen davon.

In einer weiteren speziellen Ausführungsform unterzieht man das Reaktionsgemisch während der Polymerisation in Schritt b) und die in Schritt b) erhaltene Polymerzusammensetzung keiner Kondensation unter Entfernung eines niedermolekularen Reaktionsprodukts und/oder in Gegenwart eines Kondensationskatalysators.

In einer anderen möglichen Ausführung ist eine Kondensation während und/oder im Anschluss an die Polymerisation in Schritt b) zugelassen. Dabei erfolgt die Kondensation vorzugsweise ohne Abtrennung von Lösungsmittel. Die Polymerzusammensetzung ist auch nach der Kondensation in Form eines Gels.

Ein weiterer Gegenstand der Erfindung ist eine gelförmige Polymerzusammensetzung, erhältlich durch ein Verfahren, wie zuvor und im Folgenden definiert.

In einer bevorzugten Variante des erfindungsgemäßen Verfahrens erhält man eine Polymerzusammensetzung in Form eines transparenten Gels. Ein weiterer Gegenstand der Erfindung ist daher eine Polymerzusammensetzung, wie zuvor und im Folgenden definiert, in Form eines transparenten Gels.

Bevorzugt enthält die erfindungsgemäße gelförmige Zusammensetzung Polymerzusammensetzungen wenigstens 10 Gew.-%, bevorzugt wenigstens 15 Gew.-%, insbesondere wenigstens 20 Gew.-%, bezogen auf das Gesamtgewicht der Polymerzusammensetzung, wenigstens eines Lösungsmittels. Das Lösungsmittel ist vorzugsweise ausgewählt unter den Lösungsmitteln LM) wie zuvor und im Folgenden definiert.

Eine spezielle Ausführung der Erfindung ist daher eine gelförmige Polymerzusammensetzung, die wenigstens 10 Gew.-%, bevorzugt wenigstens 15 Gew.-%, insbesondere wenigstens 20 Gew.-%, bezogen auf das Gesamtgewicht der Polymerzusammensetzung, wenigstens eines Lösungsmittels LM) enthält, das ausgewählt ist unter Wasser, C₁-C₆-Alkanolen, von PE) verschiedenen Polyolen, deren Mono- und Dialkylethern und Mischungen davon.

Ein weiterer Gegenstand der Erfindung ist die Verwendung einer Polymerzusammensetzung, wie zuvor und im Folgenden definiert,
- in Wasch- und Reinigungsmitteln,
- in Hygieneprodukten,
- in kosmetischen Mitteln,
- in pharmazeutischen Mitteln,
- in Pflanzenschutzmitteln,
- in Netzmitteln,
- in Lacken, Beschichtungsmitteln, Klebstoffen, Lederbehandlungs- oder Textilbehandlungsmitteln, etc.,
- bei der Erschließung und/oder Ausbeutung unterirdischer Erdöl- und/oder Erdgaslagerstätten.

Ein weiterer Gegenstand der Erfindung ist ein Wasch- oder Reinigungsmittel, umfassend:
a) wenigstens eine erfindungsgemäße gelförmige Polymerzusammensetzung, wie zuvor und im Folgenden definiert oder erhältlich durch ein Verfahren, wie zuvor und im Folgenden definiert,
b) wenigstens einen Builder,
c) gegebenenfalls wenigstens ein Enzym,
d) gegebenenfalls wenigstens ein Bleichmittel,
e) gegebenenfalls Wasser,
f) gegebenenfalls wenigstens einen Verdicker, und
g) gegebenenfalls wenigstens einen weiteren Zusatzstoff, der vorzugsweise ausgewählt ist unter von a) verschiedenen Tensiden, Basen, Korrosionsinhibitoren, Entschäumern, Farbstoffen, Duftstoffen, Füllstoffen, Löslichkeitsvermittlern, organischen Lösemitteln, Elektrolyten, pH-Stellmitteln, Parfümträgern, Fluoreszenzmitteln, Hydrotropen, Antiredepositionsmitteln, optischen Aufhellern, Vergrauungsinhibitoren, Einlaufverhinderern, Knitterschutzmitteln, Farbübertragungsinhibitoren, antimikrobiellen Wirkstoffen, Antioxidantien, Korrosionsinhibitoren, Antistatika, Bügelhilfsmitteln, Phobier- und Imprägniermitteln, Quell- und Schiebefestmitteln und UV-Absorbern.

Ein weiterer Gegenstand der Erfindung ist ein Reinigungsmittel für den Sanitärbereich, das wenigstens eine erfindungsgemäße gelförmige Polymerzusammensetzung, wie zuvor und im Folgenden definiert oder erhältlich durch ein Verfahren, wie zuvor und im Folgenden definiert, und wenigstens eine organische Säure enthält.

Ein weiterer Gegenstand der Erfindung ist eine Klebstoffzusammensetzung, umfassend oder bestehend aus einer Polymerzusammensetzung, erhältlich durch ein Verfahren, wie zuvor und im Folgenden definiert. Die erfindungsgemäße Klebstoffzusammensetzung eignet sich für eine Vielzahl von Anwendungen, z.B. für beschichtete Etiketten, speziell weichmacherfreie und/oder selbstklebende und/oder wiederablösbare Papieretiketten.

### BESCHREIBUNG DER ERFINDUNG

Das erfindungsgemäße Verfahren und die danach erhaltenen Polymerzusammensetzungen weisen die folgenden Vorteile auf:
- Das erfindungsgemäße Verfahren eignet sich vorteilhaft zur Herstellung von Zusammensetzungen auf Basis von Polymeren ungesättigter Carbonsäuren und Polyethern. Dabei ist es erstmals möglich, Gele und insbesondere klare, transparente Gele auf Basis von Polyacrylsäure und Polyethern bereit zu stellen.
- Das erfindungsgemäße Verfahren ermöglicht den weitgehenden oder vollständigen Verzicht vernetzender Monomere bei der Herstellung der Polymerzusammensetzungen. Diese sind somit vorteilhafterweise wasserlöslich.
- Die nach dem erfindungsgemäßen Verfahren erhaltenen Polymerzusammensetzungen zeichnen sich sowohl durch eine hohe Verträglichkeit zwischen Polyetherkomponente und Polyacrylsäure als auch durch eine hohe Verträglichkeit mit weiteren Tensiden aus.
- Das erfindungsgemäße Verfahren eignet sich weiterhin zur Herstellung von Polymerzusammensetzungen für Klebstoffzusammensetzungen. Die Bereitstellung dieser speziellen Polymerzusammensetzungen gelingt durch Auswahl einer geeigneten Glastemperatur und/oder Polyetherkomponente PE), wie im Folgenden näher ausgeführt.

Die erfindungsgemäßen Polymerzusammensetzungen liegen unter Normalbedingungen (20 °C) als Gele vor. "Gelförmige Konsistenz" zeigen Formulierungen, die eine höhere Viskosität als eine Flüssigkeit aufweisen und die insbesondere selbsttragend sind, d. h. die eine ihnen verliehene Form ohne formstabilisierende Umhüllung behalten. Im Gegensatz zu festen Formulierungen lassen sich gelförmige Formulierungen jedoch leicht durch Erwärmen und/oder unter Anwendung von Scherkräften deformieren oder in eine fluide Form überführen. Die Viskosität der gelförmigen Polymerzusammensetzungen liegt vorzugsweise bei 20°C in einem Bereich von größer als 600 bis etwa 10 000 000 mPas, besonders bevorzugt von 1000 bis 1 000 000 mPas, insbesondere von 2000 bis 500 000 mPas. Die Viskosität bei 20°C sowie der Viskositätsverlauf in Abhängigkeit der Temperatur der zu untersuchenden Proben wurden mittels eines Rotationsrheometers (DHR-1 der Firma TA-Instruments mit Peltiersystem, Platte/Platte-Geometrie, Ø 40 mm, h =1 mm) bei Temperaturen von 20 °C bis 80 °C untersucht. Temperatur-Rampe (γ = 1 % mit *M*ₘᵢₙ = 100 µNm). Messtemperatur(en) von 80°C zu 20°C und zurück je zwei Läufe (Kühl-/Heizrate 2 K/min). Messzeit je 30 min.

Im Rahmen dieser Anmeldung werden Verbindungen, die von Acrylsäure und Methacrylsäure abgeleitet sein können teilweise durch Einfügen der Silbe "(meth)" in die von der Acrylsäure abgeleitete Verbindung bezeichnet.

Im Rahmen der vorliegenden Erfindung steht C₁-C₆-Alkyl für einen linearen oder verzweigten Alkylrest mit 1 bis 5 Kohlenstoffatomen. Beispiele hierfür sind Methyl, Ethyl, Propyl, Isopropyl, n-Butyl, sec-Butyl, Isobutyl, tert-Butyl, Pentyl und Stellungsisomere davon.

C₇-C₃₀-Alkyl steht für einen linearen oder verzweigten Alkylrest mit 6 bis 30 Kohlenstoffatomen. Beispiele hierfür sind Heptyl, Octyl, 2-Ethylhexyl, Nonyl, Decyl, 2-Propylheptyl, Undecyl, Dodecyl, Tridecyl, Tetradecyl, Pentadecyl, Hexadecyl, Heptadecyl, Octadecyl, Nonadecyl, Eicosyl, Henicosyl, Docosyl, Tricosyl, Tetracosyl und Stellungsisomere davon.

Die Herstellung der erfindungsgemäßen Polymerzusammensetzungen erfolgt durch radikalische Polymerisation der Monomerzusammensetzung M) in Gegenwart wenigstens einer Polyetherkomponente PE), die in der Regel keine copolymerisierbare Doppelbindung aufweist. Dabei werden spezielle Polymerzusammensetzungen mit vorteilhaften Eigenschaften erhalten. Ohne an eine Theorie gebunden zu sein, kann dies beispielsweise auf eine Wirkung der Polyetherkomponente PE) als Schutzkolloid oder Emulgator zurückzuführen sein. Dies kann beispielsweise auch aus einer zumindest teilweisen Pfropfung auf die Polyetherkomponente als Pfropfgrundlage resultieren. Es sind jedoch auch andere Mechanismen als eine Pfropfung vorstellbar. Die erfindungsgemäßen Polymerzusammensetzungen umfassen ganz allgemein die Verfahrensprodukte der radikalischen Polymerisation, worunter z. B. Pfropfpolymerisate, Homo- und Copolymere der in der Monomermischung M) enthaltenen Monomere, Mischungen von Pfropfpolymerisaten mit ungepfropften Verbindungen der Polyetherkomponente PE) sowie beliebige Mischungen verstanden werden.

In einer speziellen bevorzugten Ausführung werden während und im Anschluss an die Polymerisation in Schritt b) das Reaktionsgemisch und die in Schritt b) erhaltene Polymerzusammensetzung keiner Kondensation unter Entfernung eines niedermolekularen Reaktionsprodukts und/oder in Gegenwart eines Kondensationskatalysators unterzogen. Darunter wird verstanden, dass speziell keine zusätzlichen Maßnahmen durchgeführt werden mit dem Ziel, den Estergruppengehalt der nach dem erfindungsgemäßen Verfahren erhaltenen Polymerzusammensetzung zu erhöhen. In einer anderen speziellen Ausführung ist die Esterbildung während und/oder im Anschluss an die Polymerisation in Schritt b) zugelassen.

Die erfindungsgemäße Polymerzusammensetzung liegt nicht in Form eines Feststoffs vor. Feststoffförmige Zusammensetzungen sind Gegenstand von parallelen Anmeldungen. Die vorliegende Erfindung betrifft weiterhin nicht die Verwendung der gelförmigen Polymerzusammensetzung in Formulierungen für die maschinelle Geschirrreinigung (automatic dish washing, ADW).

Für den Einsatz in Form eines Gels werden vorzugsweise erfindungsgemäße Polymerzusammensetzungen mit einer Glasübergangstemperatur T_{G} im Bereich von -100 bis +150°C, vorzugsweise von -20 bis +130°C eingesetzt.

Für den Einsatz in einer Klebstoffzusammensetzung werden vorzugsweise erfindungsgemäße Polymerzusammensetzungen mit niedriger Glasübergangstemperatur eingesetzt. Vorzugsweise weisen die erfindungsgemäßen Polymerzusammensetzungen für den Einsatz in Klebstoffzusammensetzungen eine Glasübergangstemperatur T_{G} im Bereich von - 100 bis + 10°C, vorzugsweise von - 80 bis 0 °C, auf.

Die Steuerung der Glasübergangstemperatur T_{G} steuern kann prinzipiell über die Art und den Anteil der Monomere sowie das Molekulargewicht erfolgen. Eine weitere Möglichkeit zur Steuerung der Glasübergangstemperatur T_{G} liegt in der Auswahl der Polyetherkomponente PE. Die Bereitstellung einer erfindungsgemäßen Polymerzusammensetzung mit gewünschter Glasübergangstemperatur T_{G} liegt im Können des Fachmanns und kann mittels Routinemessungen überprüft werden. Die im Rahmen dieser Anmeldung beschriebenen Glasübergangstemperaturen (Tg) können mittels Differential Scanning Calorimetry (DSC) bestimmt werden. Die DSC-Messung wird an ein- und derselben Probe zweckmässigerweise ein- bis zweimal wiederholt, um eine definierte thermische Vorgeschichte der Polymerzusammensetzung sicherzustellen. Die Aufheiz- und Abkühlrate betrug dabei 20 K/min.

In einer bevorzugten Ausführung der ersten Variante liegen die erfindungsgemäßen Polymerzusammensetzungen in Form eines transparenten Gels vor. Die Transparenz eines Materials wird bestimmt durch sein Absorptions- und Streuverhalten, d.h. die durchgelassene Lichtmenge und das Erscheinungsbild in der Durchsicht. Die Gesamttransmission (Lichtdurchlässigkeit) ist das Verhältnis von durchgelassenem Licht zu einfallendem Licht. Als Maß für die Lichtdurchlässigkeit wird der Transmissionsgrad τ verwendet: Er ist der Quotient aus dem Lichtstrom Φₙ hinter und Φᵥ vor dem zu prüfenden Material und wird in Prozent angegeben. Dieser Wert enthält neben der Absorbtion auch die Streu- und Reflexionsverluste. Der Transmissionsgrad wird im allgemeinen in Luft bestimmt und wird als Funktion der Wellenlänge angegeben.

Im Rahmen der Erfindung wird die Lichtdurchlässigkeit (T_{L}) bei einer Wellenlänge von 500 nm bestimmt. Als Bezugsgröße für die maximale Lichtdurchlässigkeit (T_{L}-Wert von 100 %) dient Wasser.

Vorzugsweise weist die erfindungsgemäßen Polymerzusammensetzung in Form eines transparenten Gels einen T_{L}-Wert gemessen bei 500 nm, von mindestens 85 %, besonders bevorzugt von mindestens 90 %, bezogen auf die Lichtdurchlässigkeit von Wasser, auf.

### Monomerzusammensetzung M)

### Carbonsäuremonomer A)

Die α,β-ethylenisch ungesättigte Carbonsäure A) ist vorzugsweise ausgewählt unter Acrylsäure, Methacrylsäure, Ethacrylsäure, α-Chloracrylsäure, Crotonsäure, Maleinsäure, Itaconsäure, Citraconsäure, Mesaconsäure, Glutaconsäure, Aconitsäure, Fumarsäure und Mischungen davon. Zu den Monomeren A) zählen auch die Salze der zuvor genannten Säuren, insbesondere die Natrium-, Kalium- und Ammoniumsalze sowie die Salze mit Aminen. Die Monomere A) können als solche oder als Mischungen untereinander eingesetzt werden. Die angegebenen Gewichtsanteile beziehen sich sämtlich auf die Säureform.

Bevorzugt wird die wenigstens eine α,β-ethylenisch ungesättigte Carbonsäure in nicht neutralisierter Form zur Polymerisation eingesetzt.

Besonders bevorzugt ist die Komponente A) ausgewählt unter Acrylsäure, Methacrylsäure und Mischungen davon.

Insbesondere wird als Komponente A) ausschließlich Acrylsäure eingesetzt.

Die Komponente A) wird vorzugsweise in einer Menge von 50 bis 100 Gew.-%, besonders bevorzugt 60 bis 100 Gew.-%, bezogen auf das Gesamtgewicht der Monomerzusammensetzung M), eingesetzt.

In einer bevorzugten Ausführungsform besteht die Monomerzusammensetzung M) zu mindestens 80 Gew-%, bevorzugt zu mindestens 90 Gew.-%, insbesondere zu mindestens 95 Gew.-%, bezogen auf das Gesamtgewicht der eingesetzten Monomere, aus Acrylsäure.

### Vernetzer B)

Die erfindungsgemäße Polymerzusammensetzung enthält im Wesentlichen unvernetzte Polymere. Die zur Herstellung der erfindungsgemäßen Polymerzusammensetzung eingesetzte Monomerzusammensetzung M) enthält somit keine oder nur geringe Mengen an vernetzend wirkenden Monomeren B). Vernetzer im Sinne der Erfindung sind Verbindungen mit zwei oder mehr als zwei polymerisierbaren ethylenisch ungesättigten Doppelbindungen pro Molekül.

Vorzugsweise werden Vernetzer B) in einer Menge von 0 bis 0,1 Gew.-%, besonders bevorzugt 0 bis 0,05 Gew.-%, bezogen auf das Gesamtgewicht der Monomerzusammensetzung M), verwendet. In einer speziellen Ausführungsform enthält die Monomerzusammensetzung M) keine vernetzend wirkenden Monomere B), die zwei oder mehr als zwei polymerisierbare α,β-ethylenisch ungesättigte Doppelbindungen pro Molekül aufweisen.

Geeignete Vernetzer B) sind zum Beispiel Acrylester, Methacrylester, Allylether oder Vinylether von mindestens zweiwertigen Alkoholen. Die OH-Gruppen der zugrundeliegenden Alkohole können dabei ganz oder teilweise verethert oder verestert sein; die Vernetzer enthalten aber mindestens zwei ethylenisch ungesättigte Gruppen.

Beispiele für die zugrundeliegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglykolmonoester, 2,2-Bis(4-hydroxyphenyl)-propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10000 g/mol. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrundeliegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer B) sind die Vinylester oder die Ester einwertiger, ungesättigter Alkohole mit ethylenisch ungesättigten C₃-C₆-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellithsäure, Phthalsäure, Terephthalsäure, Zitronensäure oder Bernsteinsäure.

Weitere geeignete Vernetzer B) sind Ester ungesättigter Carbonsäuren mit den oben beschriebenen mehrwertigen Alkoholen, beispielsweise der Ölsäure, Crotonsäure, Zimtsäure oder 10-Undecensäure.

Geeignet als Vernetzer B) sind außerdem geradkettige oder verzweigte, lineare oder cyclische, aliphatische oder aromatische Kohlenwasserstoffe, die über mindestens zwei Doppelbindungen verfügen, die bei aliphatischen Kohlenwasserstoffen nicht konjugiert sein dürfen, z. B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000 g/mol.

Als Vernetzer B) sind ferner geeignet die Acrylsäureamide, Methacrylsäureamide und N-Allylamine von mindestens zweiwertigen Aminen. Solche Amine sind zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren, wie Acrylsäure, Methacrylsäure, Itaconsäure, Maleinsäure, oder mindestens zweiwertigen Carbonsäuren, wie sie oben beschrieben wurden.

Ferner sind Triallylamin und Triallylmonoalkylammoniumsalze, z. B. Triallylmethylammoniumchlorid oder -methylsulfat, als Vernetzer B) geeignet.

Geeignet sind auch N-Vinyl-Verbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z. B. N,N'-Divinylethylenharnstoff oder N,N'-Divinylpropylenharnstoff.

Weitere geeignete Vernetzer B) sind Divinyldioxan, Tetraallylsilan oder Tetravinylsilan.

### Ungesättigte Sulfon- oder Phosphonsäuren C)

Die Monomerzusammensetzung M) kann anstelle der Komponente A) wenigstens eine ungesättigte Sulfonsäure oder Phosphonsäure C) enthalten. Die Monomerzusammensetzung M) kann auch zusätzlich als Komponente C) wenigstens eine ungesättigte Sulfonsäure oder Phosphonsäure enthalten. Die Komponent C) ist vorzugsweise ausgewählt unter 2-Acrylamido-2-methylpropansulfonsäure, Vinylsulfonsäure, Allylsulfonsäure, Sulfoethylacrylat, Sulfoethylmethacrylat, Sulfopropylacrylat, Sulfopropylmethacrylat, 2-Hydroxy-3-acryloxypropylsulfonsäure, 2-Hydroxy-3-methacryloxypropylsulfonsäure, Styrolsulfonsäure, Vinylphosphonsäure, Allylphosphonsäure sowie Mischungen davon.

Bevorzugt als Komponente C) ist 2-Acrylamido-2-methylpropansulfonsäure.

Zu den Monomeren C) zählen auch die Salze der zuvor genannten Säuren, insbesondere die Natrium-, Kalium- und Ammoniumsalze sowie die Salze mit Aminen. Die Monomere C) können als solche oder als Mischungen untereinander eingesetzt werden. Die angegebenen Gewichtsanteile beziehen sich sämtlich auf die Säureform.

Bevorzugt besteht die Monomerzusammensetzung M) zu mindestens 80 Gew-%, besonders bevorzugt zu mindestens 90 Gew.-%, insbesondere zu mindestens 95 Gew.-%, bezogen auf das Gesamtgewicht der Monomerzusammensetzung M), aus Monomeren A) und C). Wenn die Monomerzusammensetzung M) wenigstens ein Monomer C) enthält, so wird dieses vorzugsweise in einer Menge von 0,1 bis 50 Gew.-%, besonders bevorzugt 1 bis 25 Gew.-%, bezogen auf das Gesamtgewicht der Monomerzusammensetzung M) eingesetzt.

### Ethergruppenhaltiges Monomer D)

Die Monomerzusammensetzung M) kann zusätzlich wenigstens ein Monomer D) enthalten, ausgewählt unter Verbindungen der allgemeinen Formeln (I.a) und (I.b) 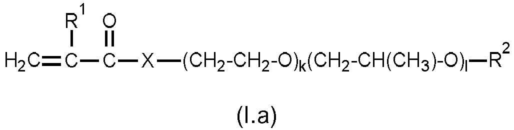 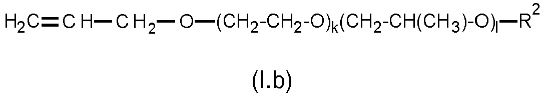 worin
die Reihenfolge der Alkylenoxideinheiten beliebig ist,
k und l unabhängig voneinander für eine ganze Zahl von 0 bis 1000 stehen, wobei die Summe aus k und l mindestens 2, bevorzugt mindestens 5, beträgt,
- R¹: für Wasserstoff oder C₁-C₈-Alkyl steht,
- R²: für Wasserstoff, C₁-C₃₀-Alkyl, C₂-C₃₀-Alkenyl oder C₅-C₈-Cycloalkyl steht,
- X: für O oder eine Gruppe der Formel NR³ steht, worin R³ für H, Alkyl, Alkenyl, Cycloalkyl, Heterocycloalkyl, Aryl oder Hetaryl steht.

In den Formeln I.a) und I.b) steht k vorzugsweise für eine ganze Zahl von 1 bis 500, besonders bevorzugt 2 bis 400, insbesondere 3 bis 250. Bevorzugt steht l für eine ganze Zahl von 0 bis 100.

Bevorzugt steht R¹ in der Formel I.a) für Wasserstoff, Methyl, Ethyl, n-Propyl, Isopropyl, n-Butyl, sec.-Butyl, tert.-Butyl, n-Pentyl oder n-Hexyl, insbesondere für Wasserstoff, Methyl oder Ethyl.

Vorzugsweise steht R² in den Formeln I.a) und I.b) für n-Octyl, 1,1,3,3-Tetramethylbutyl, Ethylhexyl, n-Nonyl, n-Decyl, n-Undecyl, Tridecyl, Myristyl, Pentadecyl, Palmityl, Heptadecyl, Octadecyl, Nonadecyl, Arrachinyl, Behenyl, Lignocerenyl, Cerotinyl, Melissinyl, Palmitoleinyl, Oleyl, Linolyl, Linolenyl, Stearyl, Lauryl.

Vorzugsweise steht X in der Formel I.a) für O oder NH, insbesondere für O.

Geeignete Polyetheracrylate I.a) sind z. B. die Polykondensationsprodukte der zuvor genannten α,β-ethylenisch ungesättigten Mono- und/oder Dicarbonsäuren und deren Säurechloriden, -amiden und -anhydriden mit Polyetherolen. Geeignete Polyetherole können leicht durch Umsetzung von Ethylenoxid, 1,2-Propylenoxid und/oder Epichlorhydrin mit einem Startermolekül, wie Wasser oder einem kurzkettigen Alkohol R²-OH hergestellt werden. Die Alkylenoxide können einzeln, alternierend nacheinander oder als Mischung eingesetzt werden. Die Polyetheracrylate I.a) können allein oder in Mischungen zur Herstellung der erfindungsgemäß eingesetzten Polymere verwendet werden.

Geeignete Allylalkoholalkoxilate I.b) sind z. B. die Veretherungsprodukte von Allylchlorid mit entsprechenden Polyetherolen. Geeignete Polyetherole können leicht durch Umsetzung von Ethylenoxid, 1,2-Propylenoxid und/oder Epichlorhydrin mit einem Starteralkohol R²-OH hergestellt werden. Die Alkylenoxide können einzeln, alternierend nacheinander oder als Mischung eingesetzt werden. Die Allylalkoholalkoxilate I.b) können allein oder in Mischungen zur Herstellung der erfindungsgemäß eingesetzten Polymere verwendet werden.

Speziell wird als Monomer D) Methyldiglycolacrylat, Methyldiglycolmethacrylat, Ethyldiglycolacrylat oder Ethyldiglycolmethacrylat eingesetzt. Bevorzugt ist Ethyldiglycolacrylat.

Die Monomerzusammensetzung M) enthält vorzugsweise 0 bis 50 Gew.-%, besonders bevorzugt 0 bis 25 Gew.-%, insbesondere 0 bis 10 Gew.-%, bezogen auf das Gesamtgewicht der Monomerzusammensetzung M), wenigstens ein ethergruppenhaltiges Monomer D). Wenn die Monomerzusammensetzung M) wenigstens ein Monomer D) enthält, so vorzugsweise in einer Menge von 0,1 bis 50 Gew.-%, besonders bevorzugt 1 bis 25 Gew.-%, insbesondere 1,5 bis 10 Gew.-%, bezogen auf das Gesamtgewicht der Monomerzusammensetzung M).

### Weitere Monomere E) bis O)

Die Monomerzusammensetzung M) kann zusätzlich wenigstens ein weiteres von den Monomeren A) bis D) verschiedenes Monomer enthalten. Bevorzugt enthält die Monomerzusammensetzung M) zusätzlich wenigstens ein Comonomer, ausgewählt unter
E) Vinylaromaten,
F) C₂-C₈ Monoolefinen, nichtaromatischen Kohlenwasserstoffen mit wenigstens zwei konjugierten Doppelbindungen,
G) Estern α,β-ethylenisch ungesättigter Mono- und Dicarbonsäuren mit C₁-C₂₀-Alkanolen,
H) Verbindungen mit einer radikalisch polymerisierbaren α,β-ethylenisch ungesättigten Doppelbindung und wenigstens einer kationogenen und/oder kationischen Gruppe pro Molekül,
I) Estern von Vinylalkohol oder Allylalkohol mit C₁-C₃₀-Monocarbonsäuren,
K) Amidgruppen-haltigen Monomeren,
L) Estern α,β-ethylenisch ungesättigter Mono- und Dicarbonsäuren mit C₂-C₃₀-Alkanediolen, Amiden α,β-ethylenisch ungesättigter Mono- und Dicarbonsäuren mit C₂-C₃₀-Aminoalkoholen mit einer primären oder sekundären Aminogruppe,
M) α,β-ethylenisch ungesättigten Nitrilen,
N) Vinylhalogeniden, Vinylidenhalogeniden,
O) Monomeren mit Harnstoffgruppen,
   und Mischungen davon.

Die Monomerzusammensetzung M) kann die weiteren Monomere E) bis O) jeweils vorzugsweise in einer Menge von 0 bis 50 Gew.-%, besonders bevorzugt 0 bis 25 Gew.-%, insbesondere 0 bis 10 Gew.-%, bezogen auf das Gesamtgewicht der Monomerzusammensetzung M), enthalten. Wenn die Monomerzusammensetzung M) wenigstens ein Monomer E) bis O) enthält, so jeweils vorzugsweise in einer Menge von 0,1 bis 50 Gew.-%, besonders bevorzugt 1 bis 25 Gew.-%, insbesondere 1,5 bis 10 Gew.-%, bezogen auf das Gesamtgewicht der Monomerzusammensetzung M). In einer speziellen Ausführung enthält die Monomerzusammensetzung M) keine weiteren Monomere E) bis O).

### Monomer E)

Bevorzugte Vinylaromaten E) sind Styrol, 2-Methylstyrol, 4-Methylstyrol, 2-(n-Butyl)styrol, 4-(n-Butyl)styrol, 4-(n-Decyl)styrol und Mischungen davon. Besonders bevorzugt sind Styrol und 2-Methylstyrol, insbesondere Styrol.

### Monomer F)

Bevorzugte Monomere F) sind Ethen, Propen, Buten, Isobuten, Diisobuten, Isopren, 1,3-Butadien und Mischungen davon.

### Monomer G)

Geeignete Ester α,β-ethylenisch ungesättigter Mono- und Dicarbonsäuren mit C₁-C₃₀-Alkanolen sind z. B. Methyl(meth)acrylat, Methylethacrylat, Ethyl(meth)acrylat, Ethylethacrylat, n-Propyl(meth)acrylat, Isopropyl(meth)acrylat, n-Butyl(meth)acrylat, tert.-Butyl(meth)acrylat, tert.-Butylethacrylat, n-Pentyl(meth)acrylat, n-Hexyl(meth)acrylat, n-Heptyl(meth)acrylat, n-Octyl(meth)acrylat,1,1,3,3-Tetramethylbutyl(meth)acrylat, Ethylhexyl(meth)acrylat, n-Nonyl(meth)acrylat, n-Decyl(meth)acrylat, n-Undecyl(meth)acrylat, Tridecyl(meth)acrylat, Myristyl(meth)acrylat, Pentadecyl(meth)acrylat, Palmityl(meth)acrylat, Heptadecyl(meth)acrylat, Nonadecyl(meth)acrylat, Arrachinyl(meth)acrylat, Behenyl(meth)acrylat, Lignocerenyl(meth)acrylat, Cerotinyl(meth)acrylat, Melissinyl(meth)acrylat, Palmitoleinyl(meth)acrylat, Oleyl(meth)acrylat, Linolyl(meth)acrylat, Linolenyl(meth)acrylat, Stearyl(meth)acrylat, Lauryl(meth)acrylat und Mischungen davon.

### Monomer H)

Bevorzugt handelt es sich bei den kationogenen und/oder kationischen Gruppen der Komponente H) um stickstoffhaltige Gruppen, wie primäre, sekundäre und tertiäre Aminogruppen sowie quaternäre Ammoniumgruppen. Vorzugsweise handelt es sich bei den stickstoffhaltigen Gruppen um tertiäre Aminogruppen oder quaternäre Ammoniumgruppen. Geladene kationische Gruppen lassen sich aus den Aminstickstoffen entweder durch Protonierung oder durch Quaternisierung mit Säuren oder Alkylierungsmitteln erzeugen. Dazu zählen z. B. Carbonsäuren, wie Milchsäure, oder Mineralsäuren, wie Phosphorsäure, Schwefelsäure und Salzsäure, oder als Alkylierungsmittel C₁-C₄-Alkylhalogenide oder -sulfate, wie Ethylchlorid, Ethylbromid, Methylchlorid, Methylbromid, Dimethylsulfat und Diethylsulfat. Eine Protonierung oder Quaternisierung kann im Allgemeinen sowohl vor als auch nach der Polymerisation erfolgen.

Vorzugsweise ist die Komponente H) ausgewählt unter Estern α,β-ethylenisch ungesättigter Mono- und Dicarbonsäuren mit Aminoalkoholen, welche am Aminstickstoff mono- oder dialkyliert sein können, Amiden α,β-ethylenisch ungesättigter Mono- und Dicarbonsäuren mit Diaminen, welche mindestens eine primäre oder sekundäre Aminogruppe aufweisen, N,N-Diallylamin, N,N-Diallyl-N-alkylaminen und deren Derivaten, vinyl- und allylsubstituierten Stickstoffheterocyclen, vinyl- und allylsubstituierten heteroaromatischen Verbindungen, und Mischungen davon.

Bevorzugte Verbindungen H) sind die Ester von α,β-ethylenisch ungesättigten Mono- und Dicarbonsäuren mit Aminoalkoholen. Bevorzugte Aminoalkohole sind C₂-C₁₂-Aminoalkohole, welche am Aminstickstoff C₁-C₈-mono- oder -dialkyliert sind. Als Säurekomponente dieser Ester eignen sich z. B. Acrylsäure, Methacrylsäure, Fumarsäure, Maleinsäure, Itaconsäure, Crotonsäure, Maleinsäureanhydrid, Monobutylmaleat und Gemische davon. Bevorzugt werden als Säurekomponente Acrylsäure, Methacrylsäure und deren Gemische eingesetzt.

Bevorzugte Monomere H) sind N-Methylaminoethyl(meth)acrylat, N-Ethylaminoethyl(meth)acrylat, N-(n-Propyl)aminoethyl(meth)acrylat, N-(tert.-Butyl)aminoethyl(meth)acrylat, N,N-Dimethylaminomethyl(meth)acrylat, N,N-Dimethylaminoethyl(meth)acrylat, N,N-Diethylaminomethyl(meth)acrylat, N,N-Diethylaminoethyl(meth)acrylat, N,N-Dimethylaminopropyl(meth)acrylat, N,N-Diethylaminopropyl(meth)acrylat und N,N-Dimethylaminocyclohexyl(meth)acrylat.

Geeignete Monomere H) sind weiterhin die Amide der zuvor genannten α,β-ethylenisch ungesättigten Mono- und Dicarbonsäuren mit Diaminen, welche mindestens eine primäre oder sekundäre Aminogruppe aufweisen. Bevorzugt sind Diamine, die eine tertiäre und eine primäre oder sekundäre Aminogruppe aufweisen.

Bevorzugt als Monomere H) sind z. B. N-[tert.-Butylaminoethyl](meth)acrylamid, N-[2-(Dimethylamino)ethyl]acrylamid, N-[2-(Dimethylamino)ethyl]methacrylamid, N-[3-(Dimethylamino)propyl]acrylamid, N-[3-(Dimethylamino)propyl]methacrylamid, N-[4-(Dimethylamino)butyl]acrylamid, N-[4-(Dimethylamino)butyl]methacrylamid, N-[2-(Diethylamino)ethyl]acrylamid, N-[4-(Dimethylamino)cyclohexyl]acrylamid und N-[4-(Dimethylamino)cyclohexyl]methacrylamid.

In einer geeigneten Ausführungsform umfasst die Komponente H) als vinylsubstituierte heteroaromatische Verbindung wenigstens eine N-Vinylimidazol-Verbindung. In einer speziellen Ausführungsform ist die Komponente H) ausgewählt unter N-Vinylimidazol-Verbindungen und Mischungen, die wenigstens eine N-Vinylimidazol-Verbindung enthalten.

Geeignete N-Vinylimidazol-Verbindungen sind Verbindungen der Formel (III) 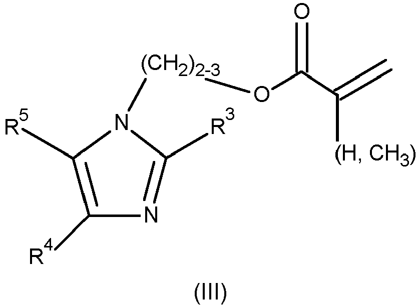 worin R³ bis R⁵ unabhängig voneinander für Wasserstoff, C₁-C₄-Alkyl oder Phenyl stehen. Bevorzugt stehen R¹ bis R³ für Wasserstoff.

Geeignet sind weiterhin N-Vinylimidazol-Verbindungen der allgemeinen Formel (IV) 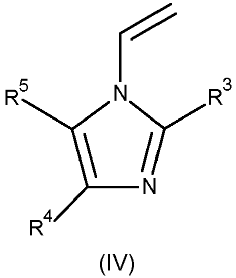 worin R³ bis R⁵ unabhängig voneinander für Wasserstoff, C₁-C₄-Alkyl oder Phenyl stehen.

Beispiele für Verbindungen der allgemeinen Formel (IV) sind folgender Tabelle zu entnehmen:

**Tabelle**

| R³ | R⁴ | R⁵ |
|---|---|---|
| H | H | H |
| Me | H | H |
| H | Me | H |
| H | H | Me |
| Me | Me | H |
| H | Me | Me |
| Me | H | Me |
| Ph | H | H |
| H | Ph | H |
| H | H | Ph |
| Ph | Me | H |
| Ph | H | Me |
| Me | Ph | H |
| H | Ph | Me |
| H | Me | Ph |
| Me | H | Ph |

| | | |
|---|---|---|
| Me = Methyl Ph = Phenyl | | |

Bevorzugt als Monomer H) ist 1-Vinylimidazol (N-Vinylimidazol) und sind Mischungen, die N-Vinylimidazol enthalten.

Geeignete Monomere H) sind auch die durch Protonierung oder Quaternisierung der zuvor genannten N-Vinylimidazolverbindungen erhältlichen Verbindungen. Beispiele für solche geladenen Monomere H) sind quaternisierte Vinylimidazole, insbesondere 3-Methyl-1-vinylimidazoliumchlorid, -methosulfat und -ethosulfat. Geeignete Säuren und Alkylierungsmittel sind die zuvor aufgeführten. Bevorzugt erfolgt eine Protonierung oder Quaternisierung nach der Polymerisation.

Geeignete Monomere H) sind weiterhin von Vinylimidazolen verschiedene vinyl- und allylsubstituierte Stickstoffheterocyclen, wie 2- und 4-Vinylpyridin, 2- und 4-Allylpyridin, und die Salze davon.

### Monomer I)

Geeignete Ester von Vinylalkohol mit C₁-C₃₀-Monocarbonsäuren sind z. B. Methylvinylester, Ethylvinylester, n-Propylvinylester, Isopropylvinylester, n-Butylvinylester,
tert.-Butylvinylester, n-Pentylvinylester, n-Hexylvinylester, n-Heptylvinylester, n-Octylvinylester, 1,1,3,3-Tetramethylbutylvinylester, Ethylhexylvinylester, n-Nonylvinylester, n-Decylvinylester, n-Undecylvinylester, Tridecylvinylester, Myristylvinylester, Pentadecylvinylester, Palmitylvinylester, Heptadecylvinylester, Octadecylvinylester, Nonadecylvinylester, Arrachinylvinylester, Behenylvinylester, Lignocerenylvinylester, Cerotinylvinylester, Melissinylvinylester, Palmitoleinylvinylester, Oleylvinylester, Linolylvinylester, Linolenylvinylester, Stearylvinylester, Laurylvinylester und Mischungen davon.

### Monomer K)

Geeignete amidgruppenhaltige Monomere K) sind Verbindungen der allgemeinen Formel (V) 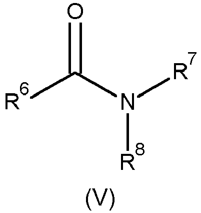 wobei
einer der Reste R⁶ bis R⁸ für eine Gruppe der Formel CH₂=CR⁹- mit R⁹ = H oder C₁-C₄-Alkyl steht und die übrigen Reste R⁶ bis R⁸ unabhängig voneinander für H, Alkyl, Cycloalkyl, Heterocycloalkyl, Aryl oder Hetaryl stehen,
wobei R⁶ und R⁷ gemeinsam mit der Amidgruppe, an die sie gebunden sind, auch für ein Lactam mit 5 bis 8 Ringatomen stehen können,
wobei R⁷ und R⁸ gemeinsam mit dem Stickstoffatom, an das sie gebunden sind, auch für einen fünf- bis siebengliedrigen Heterocyclus stehen können.

Bevorzugt sind die Verbindungen der Komponente K) ausgewählt unter primären Amiden α,β-ethylenisch ungesättigter Monocarbonsäuren, N-Vinylamiden gesättigter Monocarbonsäuren, N-Vinyllactamen, N-Alkyl- und N,N-Dialkylamiden α,β-ethylenisch ungesättigter Monocarbonsäuren und Mischungen davon.

Bevorzugte Monomere K) sind N-Vinyllactame und deren Derivate, die z. B. einen oder mehrere C₁-C₆-Alkylsubstituenten, wie Methyl, Ethyl, n-Propyl, Isopropyl, n-Butyl, sec.-Butyl, tert.-Butyl, etc. aufweisen können. Dazu zählen z. B. N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam etc.

Besonders bevorzugt werden N-Vinylpyrrolidon und/oder N-Vinylcaprolactam eingesetzt.

Geeignete Monomere K) sind weiterhin Acrylsäureamid und Methacrylsäureamid.

Geeignete N-Alkyl- und N,N-Dialkylamide α,β-ethylenisch ungesättigter Monocarbonsäuren sind beispielsweise Methyl(meth)acrylamid, Methylethacrylamid, Ethyl(meth)acrylamid, Ethylethacrylamid, n-Propyl(meth)acrylamid, Isopropyl(meth)-acrylamid, n-Butyl(meth)acrylamid, tert.-Butyl(meth)acrylamid, tert.-Butylethacrylamid, n-Pentyl(meth)acrylamid, n-Hexyl(meth)acrylamid, n-Heptyl(meth)acrylamid, n-Octyl(meth)acrylamid,1,1,3,3-Tetramethylbutyl(meth)acrylamid, Ethylhexyl(meth)acrylamid, n-Nonyl(meth)acrylamid, n-Decyl(meth)acrylamid, n-Undecyl(meth)acrylamid, Tridecyl(meth)acrylamid, Myristyl(meth)acrylamid, Pentadecyl(meth)acrylamid, Palmityl(meth)acrylamid, Heptadecyl(meth)acrylamid, Nonadecyl(meth)acrylamid, Arrachinyl(meth)acrylamid, Behenyl(meth)acrylamid, Lignocerenyl(meth)acrylamid, Cerotinyl(meth)acrylamid, Melissinyl(meth)acrylamid, Palmitoleinyl(meth)acrylamid, Oleyl(meth)acrylamid, Linolyl(meth)acrylamid, Linolenyl(meth)acrylamid, Stearyl(meth)acrylamid, Lauryl(meth)acrylamid, N-Methyl-N-(n-octyl)(meth)acrylamid, N,N-Di-(n-octyl)(meth)acrylamid und Mischungen davon.

Als Monomere K) geeignete offenkettige N-Vinylamidverbindungen sind beispielsweise N-Vinylformamid, N-Vinyl-N-methylformamid, N-Vinylacetamid, N-Vinyl-N-methylacetamid, N-Vinyl-N-ethylacetamid, N-Vinylpropionamid, N-Vinyl-N-methylpropionamid, N-Vinyl-butyramid und Mischungen davon. Bevorzugt wird N-Vinylformamid eingesetzt.

### Monomer L)

Geeignete Ester α,β-ethylenisch ungesättigter Mono- und Dicarbonsäuren mit C₂-C₃₀-Alkandiolen sind 2-Hydroxyethylacrylat, 2-Hydroxyethylmethacrylat, 2-Hydroxyethylethacrylat, 2-Hydroxypropylacrylat, 2-Hydroxypropylmethacrylat, 3-Hydroxypropylacrylat, 3-Hydroxypropylmethacrylat, 3-Hydroxybutylacrylat, 3-Hydroxybutylmethacrylat, 4-Hydroxybutylacrylat, 4-Hydroxybutylmethacrylat, 6-Hydroxyhexylacrylat, 6-Hydroxyhexylmethacrylat, 3-Hydroxy-2-ethylhexylacrylat, 3-Hydroxy-2-ethylhexylmethacrylat, etc.

Geeignete Amide α,β-ethylenisch ungesättigter Mono- und Dicarbonsäuren mit C₂-C₃₀-Aminoalkoholen mit einer primären oder sekundären Aminogruppe sind 2-Hydroxyethylacrylamid, 2-Hydroxyethylmethacrylamid, 2-Hydroxyethylethacrylamid, 2-Hydroxypropylacrylamid, 2-Hydroxypropylmethacrylamid, 3-Hydroxypropylacrylamid, 3-Hydroxypropylmethacrylamid, 3-Hydroxybutylacrylamid, 3-Hydroxybutylmethacrylamid, 4-Hydroxybutylacrylamid, 4-Hydroxybutylmethacrylamid, 6-Hydroxyhexylacrylamid, 6-Hydroxyhexylmethacrylamid, 3-Hydroxy-2-ethylhexylacrylamid und 3-Hydroxy-2-ethylhexyl-methacrylamid.

### Monomer M)

Geeignete α,β-ethylenisch ungesättigte Nitrile sind Acrylnitril oder Methacrylnitril.

### Monomer N)

Geeignete Vinylhalogenide und Vinylidenhalogeniden sind Vinylchlorid, Vinylidenchlorid, Vinylfluorid, Vinylidenfluorid und Mischungen davon.

### Monomer O)

Geeignete Monomere O) mit Harnstoffgruppen sind N-Vinylharnstoff, N-Allylharnstoff oder Derivative von Imidazolidin-2-on. Diese umfassen N-Vinyl- und N-Allylimidazolidin-2-on, N-Vinyloxyethylimidazolidin-2-on, N-(2-(meth)acrylamidoethyl)imidazolidin-2-on, N-(2-(meth)acryloxyethyl)imidazolidin-2-on (d.h., 2-Ureido(meth)acrylat), N-[2-((meth)acryloxyacetamido)ethyl]imidazolidin-2-on, etc.

Bevorzugt wird in Schritt a) eine Monomerzusammensetzung M) bereitgestellt, die
- Acrylsäure und/oder Methacrylsäure (= Monomer A),
- gegebenenfalls wenigstens ein ethergruppenhaltiges Monomer, vorzugsweise ausgewählt unter Methyldiglycolacrylat, Methyldiglycolmethacrylat, Ethyldiglycolacrylat, Ethyldiglycolmethacrylat und Mischungen davon (= Monomer D),
- gegebenenfalls Acrylsäureamid und/oder Methacrylsäureamid (= Monomer K),
- gegebenenfalls wenigstens einen Ester der (Meth)acrylsäure mit einem C₂-C₃₀-Alkandiol, vorzugsweise ausgewählt unter 2-Hydroxyethylacrylat, 2-Hydroxyethylmethacrylat, 2-Hydroxyethylethacrylat, 2-Hydroxypropylacrylat, 2-Hydroxypropylmethacrylat, 3-Hydroxypropylacrylat, 3-Hydroxypropylmethacrylat, 3-Hydroxybutylacrylat, 3-Hydroxybutylmethacrylat, 4-Hydroxybutylacrylat, 4-Hydroxybutylmethacrylat, 6-Hydroxyhexylacrylat, 6-Hydroxyhexylmethacrylat, 3-Hydroxy-2-ethylhexylacrylat, 3-Hydroxy-2-ethylhexylmethacrylat und Mischungen davon (= Monomer L)
enthält.

In einer bevorzugten Ausführung wird in Schritt a) eine Monomerzusammensetzung M) bereitgestellt, die aus Acrylsäure und/oder Methacrylsäure besteht.

In einer weiteren bevorzugten Ausführung wird in Schritt a) eine Monomerzusammensetzung M) bereitgestellt, die aus
- Acrylsäure und/oder Methacrylsäure, und
- wenigstens einem ethergruppenhaltigen Monomer, ausgewählt unter Methyldiglycolacrylat, Methyldiglycolmethacrylat, Ethyldiglycolacrylat, Ethyldiglycolmethacrylat und Mischungen davon,
   besteht.

In einer weiteren bevorzugten Ausführung wird in Schritt a) eine Monomerzusammensetzung M) bereitgestellt, die aus
- Acrylsäure und/oder Methacrylsäure, und
- einem Ester der (Meth)acrylsäure mit einem C₂-C₃₀-Alkandiol, ausgewählt unter 2-Hydroxyethylacrylat, 2-Hydroxyethylmethacrylat, 2-Hydroxyethylethacrylat, 2-Hydroxypropylacrylat, 2-Hydroxypropylmethacrylat, 3-Hydroxypropylacrylat, 3-Hydroxypropylmethacrylat, 3-Hydroxybutylacrylat, 3-Hydroxybutylmethacrylat, 4-Hydroxybutylacrylat, 4-Hydroxybutylmethacrylat, 6-Hydroxyhexylacrylat, 6-Hydroxyhexylmethacrylat, 3-Hydroxy-2-ethylhexylacrylat, 3-Hydroxy-2-ethylhexylmethacrylat und Mischungen davon,
   besteht.

In einer weiteren bevorzugten Ausführung wird in Schritt a) eine Monomerzusammensetzung M) bereitgestellt, die aus
- Acrylsäure und/oder Methacrylsäure, und
- Acrylsäureamid und/oder Methacrylsäureamid,
besteht.

### Polyetherkomponente PE)

Geeignet als Polyetherkomponente PE) sind Polyetherole mit einem zahlenmittleren Molekulargewicht von wenigstens 200 g/mol und deren Mono- und Di-(C₁-C₆-alkylether), davon, wobei, wenn die Polyetherkomponente PE) ein Polyetherol mit Propylenoxid-Wiederholungseinheiten oder einen Mono- oder Di-(C1-C6 -alkylether) eines Polyetherols mit Propylenoxid-Wiederholungseinheiten umfasst, der Anteil dieser Propylenoxid-Wiederholungseinheiten im Mittel höchstens 15 Einheiten pro Molekül beträgt und die Polyetherole PE Ethylenoxid-Homopolymere oder Ethylenoxid/Propylenoxid-Copolymere sind.

Geeignete Polyetherole und deren Mono- und Di-(C₁-C₆-alkylether) können linear oder verzweigt, vorzugsweise linear sein. Geeignete Polyetherole und deren Mono- und Di-(C₁-C₆-alkylether)weisen im Allgemeinen ein zahlenmittleres Molekulargewicht im Bereich von etwa 200 bis 100000, bevorzugt 300 bis 50000, besonders bevorzugt 500 bis 40000, auf. Geeignete Polyetherole sind beispielsweise wasserlösliche oder wasserdispergierbare nichtionische Polymere, die Alkylenoxid-Wiederholungseinheiten aufweisen. Vorzugsweise beträgt der Anteil an Alkylenoxid-Wiederholungseinheiten mindestens 30 Gew.-%, bezogen auf das Gesamtgewicht der Verbindung. Geeignete Polyetherole sind Polyalkylenglycole, wie Polyethylenglycole, Polypropylenglycole, Polytetrahydrofurane und Alkylenoxidcopolymere. Geeignete Alkylenoxide zur Herstellung von Alkylenoxidcopolymeren sind z. B. Ethylenoxid, Propylenoxid, Epichlorhydrin, 1,2- und 2,3-Butylenoxid. Geeignet sind beispielsweise Copolymerisate aus Ethylenoxid und Propylenoxid, Copolymerisate aus Ethylenoxid und Butylenoxid sowie Copolymerisate aus Ethylenoxid, Propylenoxid und mindestens einem Butylenoxid. Die Alkylenoxidcopolymere können die Alkylenoxideinheiten statistisch verteilt oder in Form von Blöcken einpolymerisiert enthalten. Vorzugsweise beträgt in den Ethylenoxid/Propylenoxid-Copolymeren der Anteil an von Ethylenoxid abgeleiteten Wiederholungseinheiten 40 bis 99 Gew.-%. Besonders bevorzugt als Polyetherkomponente PE) sind Ethylenoxid-Homopolymere und Ethylenoxid/Propylenoxid-Copolymere.

Geeignet als Polyetherkomponente PE) sind weiterhin die Mono- und Di-(C₁-C₂-alkylether) der zuvor beschriebenen Polyetherole. Bevorzugt sind Polyalkylenglycolmonomethylether und Polyalkylenglycoldimethylether.

Geeignet als Polyetherkomponente PE) sind weiterhin Polyethergruppen-haltige Tenside. Geeignet sind allgemein nichtionische und ionische Tenside, die wenigstens eine unpolare und wenigstens eine polare Gruppe aufweisen und die eine Polyethergruppe umfassen.

Die Polyethergruppen-haltigen Tenside PE) sind vorzugsweise ausgewählt unter Alkylpolyoxyalkylenether, Arylpolyoxyalkylenether, Alkylarylpolyoxyalkylenether, alkoxylierte tierische und/oder pflanzliche Fette und/oder Öle, Fettaminalkoxylate, Fettsäureamidalkoxylate, Fettsäurediethanolamidalkoxylate, Polyoxyethylensorbitanfettsäureester, Alkylpolyethersulfate, Arylpolyethersulfate, Alkylarylpolyethersulfate, Alkylpolyethersulfonate, Arylpolyethersulfonate, Alkylarylpolyethersulfonate, Alkylpolyetherphosphateate, Arylpolyetherphosphate, Alkylarylpolyetherphosphate, Glycerinethersulfonate, Glycerinethersulfate, Monoglycerid(ether)sulfate, Fettsäureamidethersulfate, Polyoxyalkylensorbitanfettsäureestern und Mischungen davon.

Zu den bevorzugten nichtionischen Tensiden Polyethergruppen-haltigen Tenside PE) gehören beispielsweise:
- Alkylpolyoxyalkylenether, die sich von niedermolekularen C₃-C₆-Alkoholen oder von C₇-C₃₀-Fettalkoholen ableiten. Dabei kann die Etherkomponente aus Ethylenoxideinheiten, Propylenoxideinheiten, 1,2-Butylenoxideinheiten, 1,4-Butylenoxideinheiten und statistischen Copolymeren und Blockcopolymeren davon abgeleitet sein. Geeignete nichtionische Tenside umfassen unter anderem Tenside der allgemeinen Formel (VI)

   R¹⁰-O-(CH₂CH₂O)ₓ-(CHR¹¹CH₂O)_{y}-R¹² (VI),

   worin R¹⁰ ein linearer oder verzweigter Alkylrest mit 6 bis 22 C-Atomen ist,
   R¹¹ und R¹² unabhängig voneinander Wasserstoff oder ein linearer oder verzweigter Alkylrest mit 1 bis 10 C-Atomen oder H sind, wobei R¹² bevorzugt Methyl ist, und
   x und y unabhängig voneinander 0 bis 300 sind. Bevorzugt ist x = 1 bis 100 und y = 0 bis 30.

Dazu zählen speziell auch Fettalkoholalkoxylate und Oxoalkoholalkoxylate, wie iso-Tridecylalkohol- und Oleylalkoholpolyoxyethylenether.
- Hydroxylgruppen-haltige Tenside der allgemeinen Formel (VII)

   R¹³-O-(CH₂CH₂O)ₛ-(CH₂CH₂CH₂O)ₜ-(CH₂CH₂CH₂CH₂O)ᵤ-(CH₂CHR¹⁴O)ᵥ-CH₂CH(OH)R¹⁵ (VII)

   wobei
   in den Verbindungen der Formel (VII) die Reihenfolge der Alkylenoxideinheiten beliebig ist,
   s, t, u und v unabhängig voneinander für eine ganze Zahl von 0 bis 500 stehen, wobei die Summe aus s, t, u und v > 0 ist,
   R¹³ und R¹⁵ unabhängig voneinander für einen geradkettigen oder verzweigten, gesättigten C₁-C₄₀-Alkylrest oder einen ein- oder mehrfach ungesättigten C₂-C₄₀-Alkenylrest stehen, und
   R¹⁴ ausgewählt ist unter Methyl, Ethyl, n-Propyl, Isopropyl oder n-Butyl.

In den Verbindungen der allgemeinen Formel (VII) steht die Summe aus s, t, u und v vorzugsweise für einen Wert von 10 bis 300, besonders bevorzugt von 15 bis 200 und insbesondere von 20 bis 150.

Bevorzugt stehen t und u für 0. Dann steht die Summe aus s und v vorzugsweise für einen Wert von 10 bis 300, besonders bevorzugt von 15 bis 200 und insbesondere von 20 bis 150.

In den Verbindungen der allgemeinen Formel (VII) stehen vorzugsweise R¹³ und R¹⁵ unabhängig voneinander für einen geradkettigen oder verzweigten, gesättigten C₂-C₃₀-Alkylrest. Dabei können R¹³ und R¹⁵ auch für Mischungen verschiedener Alkylreste stehen.

In den Verbindungen der allgemeinen Formel (VII) steht R¹⁴ vorzugsweise für Methyl oder Ethyl, insbesondere für Methyl.

Eine bevorzugte Ausführung sind Hydroxylgruppen-haltige Tenside der allgemeinen Formel (VII.1)

R¹³-O-(CH₂CH₂O)ₛ-(CH₂CH(CH₃)O)ᵥ-CH₂CH(OH)R¹⁵ (VII.1)

wobei
die Reihenfolge der -(CH₂CH₂O)- und der (CH₂CH(CH₃)O)-Einheiten beliebig ist,
s und v unabhängig voneinander für eine ganze Zahl von 0 bis 500 stehen, wobei die Summe aus s und v > 0 ist, und
R¹³ und R¹⁵ unabhängig voneinander für einen geradkettigen, gesättigten C₁-C₃₀-Alkylrest oder einen verzweigten, gesättigten C₃-C₃₀-Alkylrest oder einen ein- oder mehrfach ungesättigten C₂-C₃₀-Alkenylrest stehen.

In den Verbindungen der allgemeinen Formel (VII.1) steht die Summe aus s und v vorzugsweise für einen Wert von 10 bis 300, besonders bevorzugt von 15 bis 200 und insbesondere von 20 bis 150.

Zur Gruppe dieser nichtionischen Tenside zählen beispielsweise Hydroxymischether der allgemeinen Formel (C₆₋₂₂-Alkyl)-CH(OH)CH₂O-(EO)₂₀₋₁₂₀-(C₂₋₂₆-Alkyl).
- Alkoholpolyoxyalkylenester der allgemeinen Formel (VIII)

   R¹⁶-O-(CH₂CH₂O)ₚ-(CH₂CHR¹⁷O)_{q}-C(=O)R¹⁸ (VIII)

   wobei
   in den Verbindungen der Formel (VIII) die Reihenfolge der Alkylenoxideinheiten beliebig ist,
   p und q unabhängig voneinander für eine ganze Zahl von 0 bis 500 stehen, wobei die Summe aus p und q > 0 ist,
   R¹⁶ und R¹⁸ unabhängig voneinander für einen geradkettigen oder verzweigten, gesättigten C₁-C₄₀-Alkylrest oder einen ein- oder mehrfach ungesättigten C₂-C₄₀-Alkenylrest stehen, und
   R¹⁷ ausgewählt ist unter Methyl, Ethyl, n-Propyl, Isopropyl oder n-Butyl.

In den Verbindungen der allgemeinen Formel (VIII) steht die Summe aus p und q vorzugsweise für einen Wert von 10 bis 300, besonders bevorzugt von 15 bis 200 und insbesondere von 20 bis 150.

In den Verbindungen der allgemeinen Formel (VIII) stehen vorzugsweise R¹⁶ und R¹⁸ unabhängig voneinander für einen geradkettigen oder verzweigten, gesättigten C₄-C₃₀-Alkylrest. Dabei können R¹⁶ und R¹⁸ auch für Mischungen verschiedener Alkylreste stehen.

In den Verbindungen der allgemeinen Formel (VIII) steht R¹⁷ vorzugsweise für Methyl oder Ethyl, insbesondere für Methyl.

Dazu zählt beispielsweise Laurylalkoholpolyoxyethylenacetat.
- Alkylarylalkoholpolyoxyethylenether, z. B. Octylphenol-polyoxyethylenether,
- alkoxylierte tierische und/oder pflanzliche Fette und/oder Öle, beispielsweise Maisölethoxylate, Rizinusölethoxylate, Talgfettethoxylate,
- Alkylphenolalkoxylate, wie beispielsweise ethoxyliertes iso-Octyl-, Octyl- oder Nonylphenol, Tributylphenolpolyoxyethylenether,
- Fettaminalkoxylate, Fettsäureamid- und Fettsäurediethanolamidalkoxylate, insbesondere deren Ethoxylate,
- Polyoxyalkylensorbitanfettsäureester.

Ein Beispiel für ein Alkylpolyethersulfat ist Natriumdodecylpoly(oxyethylen)sulfat (Natriumlaurylethersulfat, SLES). Ein bevorzugter kommerziell erhältlicher modifizierter Fettalkoholpolyglycolether ist ein beidseitig endständig CₓH₂ₓ₊₁/C_{y}H_{2y+1}-terminiertes Polyethylenoxid mit einer freien OH-Gruppe und x, y = 6 - 14.

### Lösungsmittel LM)

Die radikalische Polymerisation in Schritt b) kann in Gegenwart eines Lösungsmittels LM) erfolgen, das ausgewählt ist unter Wasser, C₁-C₆-Alkanolen, von PE) verschiedenen Polyolen, deren Mono- und Dialkylethern, aprotisch polaren Lösungsmitteln und Mischungen davon.

Geeignete aprotisch polare Lösungsmitttel sind Pyrrolidone und Pyrrolidonderivate. Dazu zählen speziell 2-Pyrrolidon (γ-Butyrolactam) und N-Methylpyrrolidon.

Bevorzugt erfolgt die radikalische Polymerisation in Schritt b) in Gegenwart eines Lösungsmittels LM), das ausgewählt ist unter Wasser, C₁-C₆-Alkanolen, von PE) verschiedenen Polyolen, deren Mono- und Dialkylethern und Mischungen davon.

Geeignete Polyole und deren Mono- und Dialkylether umfassen auch Alkylenglycolmono(C₁-C₄-alkyl)ether, Alkylenglycoldi(C₁-C₄-alkyl)ether, Oligoalkylenglycole mit einem zahlenmittleren Molekulargewicht von weniger als 200 g/mol und deren Mono(C₁-C₄-alkyl)ether und Di(C₁-C₄-alkyl)ether.

Das Lösungsmittel LM) ist vorzugsweise ausgewählt unter Wasser, Methanol, Ethanol, n-Propanol, Isopropanol, n-Butanol, Ethylenglycol, Ethylenglycolmono(C₁-C₄-alkyl)ethern, Ethylenglycoldi(C₁-C₄-alkyl)ethern, 1,2-Propylenglycol, 1,2-Propylenglycolmono(C₁-C₄-alkyl)ethern, 1,2-Propylenglycoldi(C₁-C₄-alkyl)ethern, Glycerin, Polyglycerinen, 2-Pyrrolidon, N-Methylpyrrolidon, Oligoalkylenglycolen mit einem zahlenmittleren Molekulargewicht von weniger als 200 g/mol und Mischungen davon.

Geeignete Oligoethylenglycole sind unter den CTFA-Bezeichnungen PEG-6, PEG-8, PEG-12, PEG-6-32, PEG-20, PEG-150, PEG-7M, PEG-12M and PEG-115M kommerziell erhältlich. Dazu zählen speziell die Pluriol E ® Marken der BASF SE. Geeignete Alkylpolyalkylenglycole sind die entsprechenden Pluriol A ...E ® Marken der BASF SE. Bevorzugt sind die isomeren Dipropylenglycole, wie 1,1'-Oxydi-2-propanol, 2,2'-Oxydi-1-propanol, 2(2-Hydroxypropoxy)-1-propanol und Mischungen davon.

Das Lösungsmittel LM) ist besonders bevorzugt ausgewählt unter Wasser, Ethanol, n-Propanol, Isopropanol, Ethylenglycol, Diethylenglycol, 1,2-Propylenglycol, 1,2-Dipropylenglycol und Mischungen davon.

In einer speziellen Ausführungsform wird als Lösungsmittel LM) Wasser oder ein Gemisch aus Wasser und wenigstens einem von Wasser verschiedenen Lösungsmittel LM) eingesetzt, ausgewählt unter Ethanol, n-Propanol, Isopropanol, Ethylenglycol, Diethylenglycol, 1,2-Propylenglycol, 1,2-Dipropylenglycol und Mischungen davon.

In einer speziellen Ausführungsform erfolgt die radikalische Polymerisation in Schritt c) in Gegenwart eines Lösungsmittels LM), das zu wenigstens 50 Gew.-%, vorzugsweise zu wenigstens 75 Gew.-%, speziell zu wenigstens 90 Gew.-%, bezogen auf das Gesamtgewicht des Lösungsmittels LM), aus Wasser besteht. Insbesondere erfolgt die radikalische Polymerisation in Schritt c) in Gegenwart eines Lösungsmittels LM), dass vollständig aus Wasser besteht.

Bevorzugt enthält die gelförmige Polymerzusammensetzung wenigstens 10 Gew.-%, bevorzugt wenigstens 15 Gew.-%, insbesondere wenigstens 20 Gew.-%, bezogen auf das Gesamtgewicht der Polymerzusammensetzung, wenigstens eines Lösungsmittels LM).

Bevorzugt enthält die gelförmige Polymerzusammensetzung 10 bis 90 Gew.-%, bevorzugt 15 bis 80 Gew.-%, insbesondere 20 bis 70 Gew.-%, bezogen auf das Gesamtgewicht der Polymerzusammensetzung, wenigstens eines Lösungsmittels LM).

### Herstellung der erfindungsgemäßen Polymerzusammensetzungen

Die Herstellung der erfindungsgemäßen Polymerzusammensetzungen umfasst eine radikalische Polymerisation der Monomerzusammensetzung M) in Gegenwart wenigstens einer Polyetherkomponente PE). Sie wird vorzugsweise im Zulaufverfahren durchgeführt. Dabei werden im Allgemeinen zumindest die Monomere in flüssiger Form dem Reaktionsansatz zudosiert. Unter den Dosierbedingungen flüssige Monomere können dem Reaktionsansatz ohne Zugabe eines Lösungsmittels LM) zugeführt werden, ansonsten werden die Monomere als Lösung in einem geeigneten Lösungsmittel LM) eingesetzt.

Bevorzugt erfolgt die radikalische Polymerisation in Schritt b) in Zulauf-Fahrweise, wobei Zuläufe, die wenigstens eine α,β-ethylenisch ungesättigte Carbonsäure enthalten, kein Lösungsmittel LM) enthalten.

Die Dosierraten des Monomerzulaufs / der Monomerzuläufe sowie etwaiger weiterer Zuläufe (Initiator, Regler, etc.) werden vorzugsweise so gewählt, dass die Polymerisation mit der gewünschten Umsatzrate aufrechterhalten wird. Die Zugabe der einzelnen Zuläufe kann dabei kontinuierlich, periodisch, mit konstanter oder wechselnder Dosierrate, im Wesentlichen zeitgleich oder zeitversetzt erfolgen. Vorzugsweise erfolgt die Zugabe aller Zuläufe zum Reaktionsansatz kontinuierlich.

Das Gewichtsverhältnis der Monomermischung M) zur Komponente PE) liegt vorzugsweise im Bereich von 1 : 10 bis 10 : 1 liegt.

Sofern zur Herstellung der Polymerzusammensetzung ein Lösungsmittel LM) eingesetzt wird, liegt das Gewichtsverhältnis der Komponente PE) zur Komponente LM) vorzugsweise im Bereich von 0,3 : 1 bis 5 : 1, besonders bevorzugt von 0,5 : 1 bis 3 : 1.

Bevorzugt erfolgt die radikalische Polymerisation in Schritt b) bei einer Temperatur im Bereich von 20 bis 90°C, besonders bevorzugt von 30 bis 85°C, insbesondere von 40 bis 80°C.

### Bevorzugt umfasst die radikalische Polymerisation in Schritt b) des erfindungsgemäßen Verfahrens

b1) Bereitstellung einer Vorlage, die wenigstens einen Teil der Polyetherkomponente PE), gegebenenfalls wenigstens einen Teil des Reglers R) und, falls die Polymerisation in Gegenwart eines Lösungsmittels LM) erfolgt, gegebenenfalls wenigstens einen Teil von LM) enthält;
b2) Zugabe der Monomerzusammensetzung M) in einem Zulauf oder in mehreren Zuläufe sowie Zugabe eines Zulaufs, der den Radikalstarter S), gelöst in einem Teil der wenigstens einen Polyetherkomponente PE) und/oder des Lösungsmittels LM) enthält und gegebenenfalls Zugabe eines Zulaufs der die Menge des Reglers R) enthält, die nicht in der Vorlage eingesetzt wird,
b3) gegebenenfalls Nachpolymerisierung der in Schritt b2) erhaltenen Reaktionsmischung.

Üblicherweise wird die Vorlage vor der Zugabe der Zuläufe unter Rühren auf die Polymerisationstemperatur erwärmt.

Bevorzugt werden die einzelnen Reaktanden simultan in getrennten Zuläufen zugegeben, wobei die Flussraten der Zuläufe in der Regel über den Zeitraum der Zugabe möglichst konstant gehalten werden.

Die Zugabe der Zuläufe in Schritt b2) erfolgt über einen Zeitraum der in vorteilhafter Weise so gewählt wird, dass die bei der exothermen Polymerisationsreaktion entstehende Reaktionswärme ohne größeren technischen Aufwand, z.B. ohne die Verwendung eines Rückflusskühlers, abgeführt werden kann. Üblicherweise erfolgt die Zugabe der Zuläufe über einen Zeitraum von 1 bis 10 Stunden. Bevorzugt erfolgt die Zugabe der Zuläufe über einen Zeitraum von 2 bis 8 Stunden, besonders bevorzugt über 3 bis 5 Stunden.

Während der radikalischen Polymerisation werden in der Regel das optional eingesetzte Lösungsmittel und/oder etwaig entstehende Kondensationsprodukte nicht abgeführt. D. h. während der Polymerisation findet üblicherweise kein oder ein im Rahmen der technischen Möglichkeiten nur sehr geringer Stoffaustausch mit der Umgebung statt.

Die Polymerisation kann in der Regel bei Umgebungsdruck oder vermindertem oder erhöhtem Druck erfolgen. Bevorzugt wird die Polymerisation bei Umgebungsdruck durchgeführt.

Die Polymerisation erfolgt in der Regel bei konstanter Temperatur, kann aber auch bei Bedarf während der Polymerisation variiert werden. Bevorzugt wird die Polymerisationstemperatur über den gesamten Reaktionszeitraum, d.h. der Schritte b2) und b3), möglichst konstant gehalten. Je nachdem welche Einsatzstoffe im erfindungsgemäßen Verfahren eingesetzt werden, bewegt sich die Polymerisationstemperatur üblicherweise im Bereich von 20 bis 90°C. Bevorzugt bewegt sich die Polymerisationstemperatur im Bereich von 30 bis 85°C und insbesondere im Bereich von 40 bis 80°C. Sofern die Polymerisation nicht unter erhöhtem Druck durchgeführt wird und der Reaktionsmischung wenigstens ein optionales Lösungsmittel LM) zugesetzt wurde, bestimmt das Lösungsmittel bzw. Lösungsmittelgemisch durch die entsprechenden Siedetemperaturen die maximale Reaktionstemperatur.

Die Polymerisation kann in Abwesenheit oder in Gegenwart eines Inertgases erfolgen. Üblicherweise wird die Polymerisation in Anwesenheit eines Inertgases durchgeführt. Unter Inertgas wird in der Regel ein Gas verstanden, welches unter den gegebenen Reaktionsbedingungen keine Reaktion mit den an den Reaktion beteiligten Edukten, Reagenzien, Lösungsmitteln oder den entstehenden Produkten eingeht.

Falls die Polymerisation in Gegenwart eines Lösungsmittels durchgeführt wird, so ist dieses ausgewählt unter den zuvor beschriebenen Lösungsmitteln LM).

Zur Herstellung der Polymerisate können die Monomeren mit Hilfe von Radikale bildenden Initiatoren, im Nachfolgenden auch als Radikalstarter oder Starter bezeichnet, polymerisiert werden. Als Radikalstarter (Initiatoren) für die radikalische Polymerisation kommen grundsätzlich alle Radikalstarter in Frage, die im Reaktionsmedium, wie es zum Zeitpunkt ihrer Zugabe vorherrscht, im Wesentlichen löslich sind und bei den gegebenen Reaktionstemperaturen eine ausreichende Aktivität besitzen, um die Polymerisation zu starten. In das erfindungsgemäße Verfahren kann ein einzelner Radikalstarter oder eine Kombination wenigstens zweier Radikalstarter eingesetzt werden. Im letzteren Fall können die wenigstens zwei Radikalstarter im Gemisch oder vorzugsweise getrennt, gleichzeitig oder nacheinander, z.B. zu unterschiedlichen Zeitpunkten im Reaktionsverlauf, eingesetzt werden.

Als Radikalstarter für die radikalische Polymerisation können die hierfür üblichen Peroxo- und/oder Azo-Verbindungen eingesetzt werden, beispielsweise Alkali- oder Ammoniumperoxidisulfate, Diacetylperoxid, Dibenzoylperoxid, Succinylperoxid, Di-tert.-butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat, Bis-(o-toluoyl)-peroxid, Didecanoylperoxid, Dioctanoylperoxid, tert.-Butylperoctoat, Dilauroylperoxid, tert.-Butylperisobutyrat, tert.-Butylperacetat, Di-tert.-amylperoxid, tert.-Butylhydroperoxid, 2,2'-Azo-bis-isobutyronitril, Azo-bis-(2-amidinopropan)dihydrochlorid, Azobis(2,4-dimethylvaleronitril) oder 2,2'-Azo-bis-(2-methyl-butyronitril).

Geeignet sind auch Initiatormischungen oder Redox-Initiator-Systeme, wie z. B. Ascorbinsäure/Eisen(II)sulfat/Natriumperoxodisulfat, tert.-Butylhydroperoxid/Natriumdisulfit, tert.-Butylhydroperoxid/Natriumhydroxymethansulfinat,
H₂O₂/Cu^{I}.

In dem erfindungsgemäßen Verfahren bewegt sich die Menge an eingesetztem Initiatorsystem (Starter) im Bereich von 0,01 bis 10 Gew.-%, bevorzugt im Bereich von 0,1 bis 5 Gew.-%, besonders bevorzugt im Bereich von 0,2 bis 2 Gew.-% und insbesondere im Bereich von 0,3 bis 1,5 Gew.-%.

Im erfindungsgemäßen Verfahren wird der Radikalstarter in der Regel als Lösung in einem Lösungsmittel bereitgestellt, das wenigstens eines der zuvor genannten Lösungsmittel LM) umfasst.

Die Menge an Regler, die üblicherweise in dem erfindungsgemäßen Verfahren eingesetzt wird, beträgt 1 bis 40 pphm ("parts per hundred monomer", d.h. Gewichtsteile bezogen auf hundert Gewichtsteile Monomerzusammensetzung). Bevorzugt liegt die in dem erfindungsgemäßen Verfahren eingesetzte Menge an Regler im Bereich von 3 bis 30 pphm, besonders bevorzugt im Bereich von 5 bis 25 pphm.

Üblicherweise wird der Regler in Schritt b2) vollständig über einen der Zuläufe kontinuierlich zur Polymerisationsmischung zugegeben. Es ist jedoch auch möglich, den Regler entweder vollständig zur Vorlage, d.h. vor der eigentlichen Polymerisation, zuzugeben oder es wird nur ein Teil des Reglers in der Vorlage vorgelegt und der Rest in Schritt b2) über einen der Zuläufe kontinuierlich zur Polymerisationsmischung zugegeben. Die Zugabe des Reglers kann dabei jeweils ohne oder mit Lösungsmittel LM) erfolgen.

Die Menge des Reglers und die Art wie dieser zur Reaktionsmischung zugegeben wird, haben einen starken Einfluss auf das mittlere Molekulargewicht der Polymerzusammensetzung. Wenn weniger Regler eingesetzt wird und/oder wenn die Zugabe überwiegend vor der Polymerisation erfolgt, führt dies in der Regel zu höheren mittleren Molekulargewichten des gebildeten Polymers. Werden hingegen größere Menge Regler verwendet und/oder findet die Zugabe des Reglers größtenteils während der Polymerisation statt (Schritt b2)), führt dies in der Regel zu einem kleineren mittleren Molekulargewicht.

### Herstellung transparenter Gele

Das erfindungsgemäße Verfahren dient in einer bevorzugten Ausführungsform zur Herstellung einer klaren Gelzusammensetzung.

Bevorzugt beträgt die Menge an Polyetherkomponente PE) in der Vorlage (Schritt b1)) 10 bis 100 Gew.-%, besonders bevorzugt 25 bis 100 Gew.-% und insbesondere 30 bis 100 Gew.-% bezogen auf das Gesamtgewicht der zur Polymerisation eingesetzten Polyetherkomponente PE).

In der 1. Variante liegt das Mengenverhältnis der zur Herstellung der Polymerzusammensetzung verwendeten Polyetherkomponente PE) zur eingesetzten Monomerzusammensetzung M) üblicherweise im Bereich von 1,0 : 10 bis 10 : 1.

Bevorzugt liegt der Gehalt an Lösungsmittel in der Vorlage bei 10 bis 90 Gew.-%, bezogen auf das Gesamtgewicht aller in der Vorlage befindlichen Einsatzstoffe. Bevorzugt liegt der Gehalt an Lösungsmittel in der Vorlage bei 15 bis 85 Gew.-%, insbesondere bei 20 bis 80 Gew.-%, bezogen auf das Gesamtgewicht aller in der Vorlage befindlichen Einsatzstoffe.

Üblicherweise werden Lösungsmittel LM) verwendet, die einen Siedepunkt bei Normaldruck (1 bar) von unter 240°C aufweisen. In der Regel wird als Lösungsmittel LM in dieser ersten bevorzugten Ausführungsform Wasser, Ethanol, n-Propanol, Isopropanol, n-Butanol, Ethylenglycol, Ethylenglycolmono(C₁-C₄-alkyl)ethern, Ethylenglycoldi(C₁-C₄-alkyl)ethern, 1,2-Propylenglycol, 1,2-Propylenglycolmono(C₁-C₄-alkyl)ethern, 1,2-Propylenglycoldi(C₁-C₄-alkyl)ethern, Dipropylenglycoleoder Mischungen davon verwendet. Bevorzugt wird als Lösungsmittel LM) in dieser ersten bevorzugten Ausführungsform Wasser, Ethanol, n-Propanol, Isopropanol, n-Butanol oder Mischungen davon verwendet.

In dieser ersten bevorzugten Ausführungsform werden die nach Beendigung der Polymerisation (Schritt b3)) erhaltenen Polymerzusammensetzungen in ein geeignetes Gefäß transferiert und gegebenenfalls direkt auf Umgebungstemperatur (20 °C) abgekühlt.

In der Regel besitzt die nach dem zuvor beschriebenen Verfahren erhaltene Polymerzusammensetzung bereits eine gelartige Konsistenz. Gewünschtenfalls können die rheologischen Eigenschaften der Polymerzusammensetzung beispielsweise durch die Zugabe von Lösungsmitteln und/oder Verdickern eingestellt werden. Geeignete Verdicker sind im Folgenden unter der Komponente e) beschrieben.

Die erfindungsgemäß verwendete Polymerzusammensetzung ist vorzugsweise transparent, d. h. sie weist eine Lichtdurchlässigkeit (T_{L}-Wert) von mindestens 85 %, insbesondere von mindestens 90 %, bezogen auf die Lichtdurchlässigkeit von Wasser, auf.

Die erfindungsgemäß verwendete Polymerzusammensetzung weist vorzugsweise eine Viskosität bei 20°C in einem Bereich von größer als 600 bis etwa 10 000 000 mPas, besonders bevorzugt von 1000 bis 1 000 000 mPas, insbesondere von 2000 bis 500 000 mPas, auf. Die Viskosität bei 20°C sowie der Viskositätsverlauf in Abhängigkeit der Temperatur der zu untersuchenden Proben wurden mittels eines Rotationsrheometers (DHR-1 der Firma TA-Instruments mit Peltiersystem, Platte/Platte-Geometrie, Ø 40 mm, h = 1 mm) bei Temperaturen von 20 °C bis 80 °C untersucht. Temperatur-Rampe (γ = 1 % mit *M*ₘᵢₙ = 100 µNm). Messtemperatur(en) von 80 °C zu 20 °C und zurück je zwei Läufe (Kühl-/Heizrate 2 K/min). Messzeit je 30 min.

Die Säuregruppen der erfindungsgemäßen Polymerzusammensetzung können nicht, teilweise oder vollständig neutralisiert werden. Bevorzugt werden die Säuregruppen der erfindungsgemäßen Polymerzusammensetzung nicht oder nur teilweise neutralisiert.

Die Säuregruppen der erfindungsgemäßen Polymerzusammensetzung liegen vorzugsweise in nicht neutralisierter Form vor.

Als Base für die Neutralisation können Alkalimetallbasen wie Natronlauge, Kalilauge, Soda, Natriumhydrogencarbonat, Kaliumcarbonat oder Kaliumhydrogencarbonat und Erdalkalimetallbasen wie Calciumhydroxid, Calciumoxid, Magnesiumhydroxid oder Magnesiumcarbonat sowie Ammoniak oder Amine verwendet werden. Geeignete Amine sind z. B. C₁-C₆-Alkylamine, bevorzugt n-Propylamin und n-Butylamin, Dialkylamine, bevorzugt Diethylpropylamin und Dipropylmethylamin, Trialkylamine, bevorzugt Triethylamin und Triisopropylamin. Bevorzugt sind Aminoalkohole, z. B. Trialkanolamine, wie Triethanolamin, Alkyldialkanolamine, wie Methyl- oder Ethyldiethanolamin und Dialkylalkanolamine, wie Dimethylethanolamin sowie 2-Amino-2-methyl-1-propanol. Die Neutralisation der Säuregruppen kann auch mit Hilfe von Mischungen mehrerer Basen vorgenommen werden. Besonders bevorzugt ist die Base ausgewählt unter NaOH, KOH, 2-Amino-2-methyl-1-propanol, Triethylamin, Diethylaminopropylamin, Diethanolamin, Triethanolamin, Triisopropanolamin und Mischungen davon.

### Charakterisierung der Polymerzusammensetzung

Die erfindungsgemäße Polymerzusammensetzung weist vorzugsweise einen Gehalt an Säuregruppen von mehr als 1 mmol/g, besonders bevorzugt von mehr als 1.3 mmol/g auf. Die erfindungsgemäße Polymerzusammensetzung weist vorzugsweise einen Gehalt an Säuregruppen von höchstens 15 mmol/g auf. Die erfindungsgemäße Polymerzusammensetzung weist insbesondere einen Gehalt an Säuregruppen von 1.5 mmol/g bis 15 mmol/g auf.

Eine Polymerzusammensetzung in Form eines transparenten Gels weist vorzugsweise einen Gehalt an Säuregruppen von 1.5 mmol/g bis 15 mmol/g, bevorzugt von 4 mmol/g bis 7 mmol/g, auf.

In einer zweiten bevorzugten Ausführungsform liegen die Säuregruppen der erfindungsgemäßen Polymerzusammensetzung in nicht neutralisierter Form vor.

Vorzugsweise weist die Polymerzusammensetzung eine Löslichkeit in Wasser bei 40 °C und einem pH-Wert von 8 von mindesten 5 g/L auf.

Das gewichtsmittlere Molekulargewicht M_{w} der erfindungsgemäßen Polymerzusammensetzung, bestimmt mittels Gelpermeationschromatographie (GPC) unter Verwendung von neutralisierter Polyacrylsäure als Polymerstandard beträgt vorzugsweise 1000 bis 150000 Dalton.

### Tensidhaltige Zusammensetzung

Die erfindungsgemäße Polymerzusammensetzung eignet sich besonders vorteilhaft zur Formulierung tensidhaltiger Zusammensetzungen. Insbesondere handelt es sich dabei um wässrige tensidhaltige Zusammensetzungen. Die erfindungsgemäße Polymerzusammensetzung zeichnet sich durch ihre gute Verträglichkeit mit einer Vielzahl Tensiden aus.

Tensidhaltigen Zusammensetzungen, die die erfindungsgemäße Polymerzusammensetzung enthalten, weisen vorzugsweise einen Gesamttensidgehalt von 0,1 bis 75 Gew.-%, besonders bevorzugt von 0,5 bis 60 Gew.-%, insbesondere von 1 bis 50 Gew.-%, bezogen auf das Gesamtgewicht der tensidhaltigen Zusammensetzung, auf.

Geeignete zusätzliche Tenside sind anionische Tenside, nichtionische Tenside, kationische Tenside, amphotere Tenside und Mischungen davon.

Typische Beispiele für anionische Tenside sind Seifen, Alkylsulfonate, Alkylbenzolsulfonate, Olefinsulfonate, Methylestersulfonate, Sulfofettsäuren, Alkylsulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Alkylglucosecarboxylate, Proteinfettsäurekondensate und Alkyl(ether)phosphate.

Geeignete Seifen sind z. B. Alkali-, Erdalkali- und Ammoniumsalze von Fettsäuren, wie Kaliumstearat.

Geeignete Olefinsulfonate werden z. B. durch Anlagerung von SO₃ an Olefine der Formel R¹⁵-CH=CH-R¹⁶ und nachfolgende Hydrolyse und Neutralisation erhalten, wobei R¹⁵ und R¹⁶ unabhängig voneinander für H oder Alkylreste mit 1 bis 20 Kohlenstoffatomen stehen, mit der Maßgabe, dass R¹⁵ und R¹⁶ zusammen mindestens 6 und vorzugsweise 8 bis 20, speziell 10 bis 16, Kohlenstoffatome aufweisen. Hinsichtlich Herstellung und Verwendung sei auf den Übersichtsartikel "J. Am. Oil. Chem. Soc.", 55, 70 (1978) verwiesen. Die Olefinsulfonate können als Alkali-, Erdalkali-, Ammonium-, Alkylammonium-, Alkanolammonium- oder Glucammoniumsalze vorliegen. Vorzugsweise liegen die Olefinsulfonate als Natriumsalze vor. Das hydrolysierte alpha-Olefinsulfonierungsprodukt, d. h. die alpha-Olefinsulfonate, setzen sich aus ca. 60 Gew.-% Alkansulfonaten und ca. 40 Gew.-% Hydroxyalkansulfonaten zusammen; hiervon sind etwa 80 bis 85 Gew.-% Mono- und 15 bis 20 Gew.-% Disulfonate.

Bevorzugte Methylestersulfonate (MES) werden durch Sulfonierung der Fettsäuremethylester von pflanzlichen oder tierischen Fetten oder Ölen gewonnen. Bevorzugt sind Methylestersulfonate aus pflanzlichen Fetten und Ölen, z. B. aus Rapsöl, Sonnenblumenöl, Sojaöl, Palmöl, Kokosfett, etc.

Bevorzugte Alkylsulfate sind Sulfate von Fettalkoholen der allgemeinen Formel R¹⁷-O-SO₃Y, worin R¹⁷ für einen linearen oder verzweigten, gesättigten oder ungesättigten Kohlenwasserstoffrest mit 6 bis 22 Kohlenstoffatomen und Y für ein Alkalimetall, das einwertige Ladungsäquivalent eines Erdalkalimetalls, Ammonium-, Mono-, Di-, Tri- oder Tetraalkylammonium, Alkanolammonium oder Glucammonium, steht. Geeignete Fettalkoholsulfate werden vorzugsweise durch Sulfatierung von nativen Fettalkoholen oder synthetischen Oxoalkoholen und nachfolgender Neutralisation erhalten werden. Typische Beispiele für Fettalkoholsulfate sind die Sulfatierungsprodukte des Capronalkohols, Caprylalkohols, 2-Ethylhexylalkohols, Caprinalkohols, Laurylalkohols, Isotridecylalkohols, Myristylalkohols, Cetylalkohols, Palmoleylalkohols, Stearylalkohols, Isostearylalkohols, Oleylalkohols, Elaidylalkohols, Petroselinylalkohols, Linolylalkohols, Linolenylalkohols, Behenylalkohols und Elaeostearylalkohols sowie die Salze und Mischungen davon. Bevorzugte Salze der Fettalkoholsulfate sind die Natrium- und Kaliumsalze, insbesondere die Natriumsalze. Bevorzugte Mischungen der Fettalkoholsulfate basieren auf technischen Alkoholmischungen, die z. B. bei der Hochdruckhydrierung von technischen Methylestern auf Basis von Fetten und Ölen oder bei der Hydrierung von Aldehyden aus der Oxosynthese oder bei der Dimerisierung von ungesättigten Fettalkoholen anfallen. Vorzugsweise werden zur Herstellung von Alkylsulfaten Fettalkohole und Fettalkoholgemische mit 12 bis 18 Kohlenstoffatomen und insbesondere 16 bis 18 Kohlenstoffatomen eingesetzt. Typische Beispiele hierfür sind technische Alkoholsulfate auf Basis von pflanzlichen Rohstoffen.

Bevorzugte Sarkosinate sind Natriumlauroylsarkosinat oder Natriumstearoylsarkosinat.

Bevorzugte Proteinfettsäurekondensate sind pflanzliche Produkte auf Weizenbasis.

Bevorzugte Alkylphosphate sind Mono- und Diphosphorsäurealkylester.

Geeignete Acylglutamate sind Verbindungen der Formel (I) 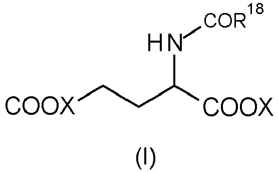 worin COR¹⁸ für einen linearen oder verzweigten Acylrest mit 6 bis 22 Kohlenstoffatomen und 0, 1, 2 oder 3 Doppelbindungen und X für Wasserstoff, ein Alkalimetall, das einwertige Ladungsäquivalent eines Erdalkalimetalls, Ammonium, Alkylammonium, Alkanolammonium oder Glucammonium steht. Die Herstellung von Acylglutamaten erfolgt beispielsweise durch Schotten-Baumann-Acylierung von Glutaminsäure mit Fettsäuren, Fettsäureestern oder Fettsäurehalogeniden. Acylglutamate sind kommerziell beispielsweise von der Clariant AG, Frankfurt/DE, oder der Ajinomoto Co. Inc., Tokio/JP, erhältlich. Eine Übersicht zu Herstellung und Eigenschaften der Acylglutamate findet sich von M. Takehara et al. in J. Am. Oil Chem. Soc. 49 (1972) 143. Typische als Komponente b) geeignete Acylglutamate leiten sich bevorzugt von Fettsäuren mit 6 bis 22 und besonders bevorzugt 12 bis 18 Kohlenstoffatomen ab. Es werden insbesondere die Mono- oder Dialkalimetallsalze des Acylglutamats eingesetzt. Dazu zählen z. B. (Handelsnamen der Fa. Ajinomoto, USA in Klammern): Natriumcocoylglutamat (Amisoft CS-11), Dinatriumcocoylglutamat (Amisoft ECS-22SB), Triethanolammoniumcocoylglutamat (Amisoft CT-12), Triethanolammoniumlauroylglutamat (Amisoft LT-12), Natriummyristoylglutamat (Amisoft MS-11), Natriumstearoylglutamat (Amisoft HS-11 P) und Mischungen davon.

Zu den zusätzlichen nichtionischen Tensiden gehören beispielsweise:
- Glycerinester, wie beispielsweise Glycerinmonostearat,
- Zuckertenside, Sorbitester, wie beispielsweise Sorbitanfettsäureester (Sorbitanmonooleat, Sorbitantristearat), Polyoxyethylensorbitanfettsäureester, Alkylpolyglycoside, N-Alkylgluconamide,
- Alkylmethylsulfoxide,
- Alkyldimethylphosphinoxide, wie beispielsweise Tetradecyldimethylphosphinoxid.

Geeignete amphotere Tenside sind z. B. Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate. Beispielsweise können Cocodimethylsulfopropylbetain, Laurylbetain, Cocamidopropylbetain, Natriumcocamphopropionat oder Tetradecyldimethylaminoxid eingesetzt werden.

Zu den kationischen Tensiden gehören beispielsweise quaternierte Ammoniumverbindungen, insbesondere Alkyltrimethylammonium- und Dialkyldimethylammonium-Halogenide und -alkylsulfate sowie Pyridin- und Imidazolin-Derivate, insbesondere Alkylpyridinium-Halogenide. Beispielsweise können Behenyl- oder Cetyltrimethylammoniumchlorid eingesetzt werden.

### Wasch- und Reinigungsmittel

Die erfindungsgemäße Polymerzusammensetzung eignet sich vorteilhaft für die Verwendung in Wasch- und Reinigungsmitteln.

Unter Waschmitteln versteht man dabei im Rahmen der vorliegenden Erfindung solche Mittel, die zum Reinigen von flexiblen Materialien mit hoher Saugfähigkeit verwendet werden, z.B. von Materialien mit einem textilen Charakter, während man unter Reinigungsmitteln im Rahmen der vorliegenden Erfindung solche Mittel versteht, die zum Reinigen von Materialien mit geschlossener Oberfläche, d.h. mit einer Oberfläche, die keine oder nur wenige und kleine Poren hat und infolgedessen keine oder nur eine geringe Saugfähigkeit aufweist, eingesetzt werden.

Beispiele für flexible Materialien mit hoher Saugfähigkeit sind solche, die natürliche, synthetische oder halbsynthetische Fasermaterialien enthalten oder daraus bestehen und die demzufolge in der Regel zumindest teilweise einen textilen Charakter aufweisen. Die faserhaltigen oder aus Fasern bestehenden Materialien können prinzipiell in jeder im Gebrauch oder der Herstellung und Verarbeitung vorkommenden Form vorliegen. Beispielsweise können Fasern ungeordnet in Form von Flocke oder Haufwerk, geordnet in Form von Fäden, Garnen, Zwirnen, oder in Form von Flächengebilden wie Vliesen, Lodenstoffen oder Filz, Geweben, Gewirken in allen denkbaren Bindungsarten vorliegen. Bei den Fasern kann es sich um Rohfasern oder um Fasern in beliebigen Verarbeitungsstadien handeln. Beispiele sind natürliche Eiweiß- oder Zellulosefasern, wie Wolle, Seide, Baumwolle, Sisal, Hanf oder Kokosfasern, oder synthetische Fasern, wie beispielsweise Polyester-, Polyamid- oder Polyacrylnitrilfasern.

Beispiele für Materialien, die keine oder nur wenige und kleine Poren haben und keine oder nur eine geringe Saugfähigkeit aufweisen, sind Metall, Glas, Email oder Keramik. Typische Objekte aus diesen Materialien sind z.B. metallene Spülbecken, Badewannen, Waschbecken, Kacheln, Fliesen, ausgehärtete Kunstharze, wie z.B. dekorative Melaminharzoberflächen auf Küchenmöbeln oder lackierte Metallflächen wie z.B. Kühlschränke und Autokarosserien, gedruckten Schaltungen (printed circuit boards), Mikrochips, versiegelte oder lackierte Hölzer, z.B. Parkett oder Wandverkleidungen, Fensterrahmen, Türen, Kunststoffbeläge wie Fußbodenbeläge aus PVC oder Hartgummi, oder Hart- oder Weichschaumstoffe mit weitgehend geschlossenen Oberflächen.

Beispiele für Reinigungsmittel, die die erfindungsgemäße Polymerzusammensetzung enthalten, umfassen Wasch- und Reinigungsmittel, Metallentfetter, Glasreiniger, Fußbodenreiniger, Allzweckreiniger, Hochdruckreiniger, Neutralreiniger, alkalische Reiniger, saure Reiniger, Spritzentfetter, Molkereireiniger, Großküchenreiniger, Apparatereiniger in der Industrie, insbesondere der chemischen Industrie, Reiniger für der Autowäsche und auch Haushalts-Allzweckreiniger.

Bevorzugt sind die die Wasch- und Reinigungsmittel, die die erfindungsgemäße Polymerzusammensetzung enthalten, bei Raumtemperatur (20 °C) gelförmig, flüssig oder in Anteilen gelförmig.

Die erfindungsgemäße Polymerzusammensetzung zeichnet sich insbesondere durch eine hervorragende Wirkung als Cobuilder, Tensid oder soil release Polymer aus. Die Bestandteile der Polymerzusammensetzung unterstützen weiterhin die Reinigungsleistung der Gesamtformulierung, indem sie den Schmutz ablösen und dispergieren.

Das erfindungsgemäße Wasch- und Reinigungsmittel umfasst vorzugsweise folgende Bestandteile:
a) wenigstens eine erfindungsgemäße gelförmige Polymerzusammensetzung,
b) wenigstens einen Builder (auch als Sequestrierungsmittel, Gerüststoff, Komplexbildner, Chelator, Chelatisierungsmittel oder Enthärter bezeichnet),
c) gegebenenfalls wenigstens ein Enzym,
d) gegebenenfalls wenigstens ein Bleichmittel,
e) gegebenenfalls Wasser,
f) gegebenenfalls wenigstens einen Verdicker, und
g) gegebenenfalls wenigstens einen weiteren Zusatzstoff, der vorzugsweise ausgewählt ist unter von a) verschiedenen Tensiden, Basen, Korrosionsinhibitoren, Entschäumern, Farbstoffen, Duftstoffen, Füllstoffen, Löslichkeitsvermittlern, organischen Lösemitteln, Elektrolyten, pH-Stellmitteln, Parfümträgern, Fluoreszenzmitteln, Hydrotropen, Antiredepositionsmitteln, optischen Aufhellern, Vergrauungsinhibitoren, Einlaufverhinderern, Knitterschutzmitteln, Farbübertragungsinhibitoren, antimikrobiellen Wirkstoffen, Antioxidantien, Korrosionsinhibitoren, Antistatika, Bügelhilfsmitteln, Phobier- und Imprägniermitteln, Quell- und Schiebefestmitteln und UV-Absorbern.

Vorzugsweise enthalten die erfindungsgemäßen Wasch- und Reinigungsmittel:
a) 0,1 bis 90 Gew.-% wenigstens einer erfindungsgemäßen gelförmigen Polymerzusammensetzung,
b) 5 bis 90 Gew.-%;wenigstens eines Builders,
c) 0 bis 8 Gew.-% wenigstens eines Enzyms,
d) 0 bis 30 Gew.-% wenigstens eines Bleichmittels,
e) 0 bis 90 Gew.-% Wasser,
f) 0 bis 8 Gew.-% wenigstens eines Verdickers,
g) 0 bis 50 Gew.-% wenigstens eines weiteren Zusatzstoffs,
mit der Maßgabe, dass sich die Gewichtsmengen der Komponenten a) bis g) zu 100 Gew.-% ergänzen.

Eine bevorzugte Ausführungsform sind gelförmige Wasch- und Reinigungsmittel. Unter gelförmigen Wasch- und Reinigungsmitteln versteht man fluide Mittel, die bei Raumtemperatur (20 °C) eine Viskosität aufweisen, die höher ist als die von Wasser, die aber noch soweit fließfähig sind, dass sie mit üblichen Dosierhilfsmitteln problemlos dosiert werden können. Vorzugsweise weisen die erfindungsgemäßen gelförmigen Wasch- und Reinigungsmittel eine Viskosität von 0,5 bis 100 Pa·s, besonders bevorzugt von 0,5 bis 50 Pa·s und insbesondere von 1 bis 30 Pa·s bei 20 °C auf.

Vorzugsweise enthalten die gelförmigen Wasch- und Reinigungsmittel:
a) 0,1 bis 90 Gew.-% wenigstens einer erfindungsgemäßen gelförmigen Polymerzusammensetzung,
b) 5 bis 80 Gew.-%;wenigstens eines Builders,
c) 0 bis 8 Gew.-% wenigstens eines Enzyms,
d) 0 bis 30 Gew.-% wenigstens eines Bleichmittels,
e) 0,1 bis 90 Gew.-% Wasser,
f) 0,1 bis 8 Gew.-% wenigstens eines Verdickers,
g) 0 bis 25 Gew.-% wenigstens eines weiteren Zusatzstoffs,
mit der Maßgabe, dass sich die Gewichtsmengen der Komponenten a) bis g) zu 100 Gew.-% ergänzen.

Besonders bevorzugt umfasst das erfindungsgemäße Wasch- und Reinigungsmittel wenigstens ein Enzym.

Des Weiteren bevorzugt umfasst das erfindungsgemäße Wasch- und Reinigungsmittel wenigstens ein Bleichmittel.

### Komponente a)

Bezüglich als Komponente a) geeignete und bevorzugte erfindungsgemäße Polymerzusammensetzungen wird auf die vorstehenden Ausführungen verwiesen.

### Komponente b)

Die erfindungsgemäßen Wasch- und Reinigungsmittel enthalten vorzugsweise wenigstens einen Builder.

Builder, die teilweise auch als Sequestrierungsmittel, Gerüststoff, Komplexbildner, Chelator, Chelatisierungsmittel oder Enthärter bezeichnet werden, binden Erdalkalimetalle und andere wasserlösliche Metallsalze, ohne zu präzipitieren. Sie helfen Schmutz aufzubrechen, dispergieren Schmutzteilen, helfen Schmutz abzulösen und haben teilweise eine eigene Waschwirkung. Außerdem halten sie, wenn sie fest sind und in pulverförmigen Formulierungen eingesetzt werden, das Pulver rieselfähig.

Geeignete Builder können sowohl organischer als auch anorganischer Natur sein. Beispiele sind Alumosilikate, Carbonate, Phosphate und Polyphosphate, Polycarbonsäuren, Polycarboxylate, Hydroxycarbonsäuren, Phosphonsäuren, z.B. Hydroxyalkylphosphonsäuren, Phosphonate, Aminopolycarbonsäuren und deren Salze und polymere carbonsäuregruppenhaltige Verbindungen und deren Salze.

Geeignete anorganische Builder sind beispielsweise kristalline oder amorphe Alumosilikate mit ionenaustauschenden Eigenschaften, wie Zeolithe. Verschiedene Typen von Zeolithen sind geeignet, insbesondere Zeolithe A, X, B, P, MAP und HS in ihrer Na-Form oder in Formen, in denen Na teilweise gegen andere Kationen wie Li, K, Ca, Mg oder Ammonium ausgetauscht ist. Geeignete Zeolithe sind beispielsweise in der US-A-4604224 beschrieben. Als Builder geeignete kristalline Silikate sind beispielsweise Disilikate oder Schichtsilikate, z.B. 5-Na₂Si₂O₅ oder B-Na₂Si₂O₅ (SKS 6 bzw. SKS 7). Die Silikate können in Form ihrer Alkalimetall-, Erdalkalimetall- oder Ammoniumsalze eingesetzt werden, vorzugsweise als Na-, Li- und Mg-Silikate. Amorphe Silikate wie beispielsweise Natriummetasilikat, welches eine polymere Struktur aufweist, oder amorphes Disilikat (Britesil® H 20 Hersteller: Akzo) sind ebenfalls verwendbar. Bevorzugt ist hierunter Natriumdisilikat.

Geeignete anorganische Buildersubstanzen auf Carbonat-Basis sind Carbonate und Hydrogencarbonate. Diese können in Form ihrer Alkalimetall-, Erdalkalimetall- oder Ammoniumsalze eingesetzt werden. Vorzugsweise werden Na-, Li- und Mg-Carbonate bzw. -Hydrogencarbonate, insbesondere Natriumcarbonat und/oder Natriumhydrogencarbonat, eingesetzt.

Übliche, als anorganische Builder eingesetzte Phosphate sind Alkalimetallorthophosphate und/oder -polyphosphate wie z.B. Pentanatriumtriphosphat.

Geeignete organische Builder sind beispielsweise C₄-C₃₀-Di-, -Tri- und -Tetracarbonsäuren, wie z.B. Bernsteinsäure, Propantricarbonsäure, Butantetracarbonsäure, Cyclopentantetracarbonsäure und Alkyl- und Alkenylbernsteinsäuren mit C₂-C₂₀-Alkyl- bzw. -Alkenyl-Resten.

Geeignete organische Builder sind weiterhin Hydroxycarbonsäuren und Polyhydroxycarbonsäuren (Zuckersäuren). Dazu zählen C₄-C₂₀-Hydroxycarbonsäuren wie z.B. Äpfelsäure, Weinsäure, Gluconsäure, Schleimsäure, Milchsäure, Glutarsäure, Zitronensäure, Tartronsäure, Glucoheptonsäure, Lactobionsäure und Saccharosemono-, -di- und -tricarbonsäure. Bevorzugt sind hierunter Zitronensäure und ihre Salze.

Geeignete organische Builder sind weiterhin Phosphonsäuren, wie z.B. Hydroxyalkylphosphonsäuren, Aminophosphonsäuren und die Salze davon. Dazu zählen z.B. Phosphonobutantricarbonsäure, Aminotris-methylenphosphonsäure, Ethylendiamintetraethylenphosphonsäure, Hexamethylendiamintetramethylenphosphonsäure, Diethylentriaminpentamethylenphosphonsäure, Morpholino-methandiphosphonsäure, 1-Hydroxy-C₁-bis C₁₀-alkyl-1,1-diphosphonsäuren wie 1-Hydroxyethan-1,1-diphosphonsäure. Bevorzugt ist hierunter 1-Hydroxyethan-1,1-diphosphonsäure und deren Salze. Geeignete organische Builder sind weiterhin Aminopolycarbonsäuren, wie Nitrilotriessigsäure (NTA), Nitrilomonoessigdipropionsäure, Nitrilotripropionsäure, β-Alanindiessigsäure (β-ADA), Ethylendiamintetraessigsäure (EDTA), Diethylentriaminpentaessigsäure, 1,3-Propylendiamintetraessigsäure, 1,2-Propylendiamintetraessigsäure, N-(Alkyl)-ethylendiamintriessigsäure, N-(Hydroxyalkyl)-ethylendiamintriessigsäure, Ethylendiamintriessigsäure, Cyclohexylen-1,2-diamintetraessigsäure, Iminodibernsteinsäure, Ethylendiamindibernsteinsäure, Serindiessigsäure, Isoserindiessigsäure, L-Asparagindiessigsäure, L-Glutamindiessigsäure, Methylglycindiessigsäure (MGDA) und die Salze der zuvorgenannten Aminopolycarbonsäuren. Bevorzugt sind hierunter L-Glutamindiessigsäure, Methylglycindiessigsäure und deren Salze.

Geeignete organische Builder sind weiterhin polymere carbonsäuregruppenhaltige Verbindungen, wie Acrylsäure-Homopolymere. Diese weisen vorzugsweise ein zahlenmittleres Molekulargewicht im Bereich von 800 bis 70000 g/mol, besonders bevorzugt von 900 bis 50000 g/mol, insbesondere von 1000 bis 20000 g/mol, speziell 1000 bis 10000 g/mol, auf. Der Begriff Acrylsäure-Homopolymer umfasst dabei auch Polymere, in denen die Carbonsäuregruppen teilweise oder vollständig neutralisiert vorliegen. Dazu zählen Acrylsäure-Homopolymere, in denen die Carbonsäuregruppen teilweise oder vollständig in Form von Alkalimetallsalzen oder Ammoniumsalzen vorliegen. Bevorzugt sind Acrylsäure-Homopolymere, in denen die Carbonsäuregruppen protoniert sind oder in denen die Carbonsäuregruppen teilweise oder vollständig in Form von Natriumsalzen vorliegen.

Geeignete polymere carbonsäuregruppenhaltige Verbindungen sind auch Oligomaleinsäuren, wie sie beispielsweise in EP-A 451 508 und EP-A 396 303 beschrieben sind.

Geeignete polymere carbonsäuregruppenhaltige Verbindungen sind auch Terpolymere ungesättigter C₄-C₈-Dicarbonsäuren, wobei als Comonomere monoethylenisch ungesättigte Monomere aus der unten angegebenen Gruppe (i) in Mengen von bis zu 95 Gew.-%, aus der Gruppe (ii) in Mengen von bis zu 60 Gew.-% und aus der Gruppe (iii) in Mengen von bis zu 20 Gew.-% einpolymerisiert sein können. Als ungesättigte C₄-C₈-Dicarbonsäuren sind hierbei beispielsweise Maleinsäure, Fumarsäure, Itaconsäure und Citraconsäure geeignet. Bevorzugt wird Maleinsäure. Die Gruppe (i) umfasst monoethylenisch ungesättigte C₃-C₈-Monocarbonsäuren wie z.B. Acrylsäure, Methacrylsäure, Crotonsäure und Vinylessigsäure. Bevorzugt werden aus der Gruppe (i) Acrylsäure und Methacrylsäure eingesetzt. Die Gruppe (ii) umfasst monoethylenisch ungesättigte C₂-C₂₂-Olefine, Vinylalkylether mit C₁-C₈-Alkylgruppen, Styrol, Vinylester von C₁-C₈-Carbonsäuren, (Meth)acrylamid und Vinylpyrrolidon. Bevorzugt werden aus der Gruppe (ii) C₂-C₆-Olefine, Vinylalkylether mit C₁-C₄-Alkylgruppen, Vinylacetat und Vinylpropionat eingesetzt. Falls die Polymeren der Gruppe (ii) Vinylester einpolymerisiert enthalten, können diese auch teilweise oder vollständig zu Vinylalkohol-Struktureinheiten hydrolysiert vorliegen. Geeignete Co- und Terpolymere sind beispielsweise aus US-A 3887806 sowie DE-A 4313909 bekannt. Die Gruppe (iii) umfasst (Meth)acrylester von C₁-C₈-Alkoholen, (Meth)acrylnitril, (Meth)acrylamide von C₁-C₈-Aminen, N-Vinylformamid und N-Vinylimidazol.

Geeignete polymere carbonsäuregruppenhaltige Verbindungen sind auch Homopolymere der monoethylenisch ungesättigten C₃-C₈-Monocarbonsäuren wie z.B. Acrylsäure, Methacrylsäure, Crotonsäure und Vinylessigsäure, insbesondere der Acrylsäure und Methacrylsäure, Copolymere von Dicarbonsäuren, wie z.B. Copolymere von Maleinsäure und Acrylsäure im Gewichtsverhältnis 10:90 bis 95:5, besonders bevorzugt solche im Gewichtsverhältnis 30:70 bis 90:10 mit Molmassen von 1000 bis 150000; Terpolymere aus Maleinsäure, Acrylsäure und einem Vinylester einer C₁-C₃- Carbonsäure im Gewichtsverhältnis 10 (Maleinsäure) :90 (Acrylsäure + Vinylester) bis 95 (Maleinsäure) :10 (Acrylsäure + Vinylester), wobei das Gew.-Verhältnis von Acrylsäure zum Vinylester im Bereich von 30:70 bis 70:30 variieren kann; Copolymere von Maleinsäure mit C₂-C₈-Olefinen im Molverhältnis 40:60 bis 80:20, wobei Copolymere von Maleinsäure mit Ethylen, Propylen oder Isobuten im Molverhältnis 50:50 besonders bevorzugt sind.

Geeignete polymere carbonsäuregruppenhaltige Verbindungen sind weiterhin Copolymere von 50 bis 98 Gew.-% ethylenisch ungesättigter schwacher Carbonsäuren mit 2 bis 50 Gew.-% ethylenisch ungesättigter Sulfonsäuren, wie sie beispielsweise in der EP-A-0877002 beschrieben sind. Geeignete schwache ethylenisch ungesättigte Carbonsäuren sind insbesondere C₃-C₆-Monocarbonsäuren, wie Acrylsäure und Methacrylsäure. Geeignete ethylenisch ungesättigte Sulfonsäuren sind 2-Acetylamidomethyl-1-propansulfonsäure, 2-Methacrylsäureamido-2-methyl-1-propansulfonsäure, 2-Methacrylamindo-2-hydroxypropansulfonsäure, Allylsulfonsäure, Methallylsulfonsäure, Allyloxybenzolsulfonsäure, Methallyloxybenzolsulfonsäure, 2-Hydroxy-3-(2-propenyloxy)propansulfonsäure, 2-Methyl-2-propen-1-sulfonsäure, Styrolsulfonsäure, Vinylsulfonsäure, 3-Sulfopropylacrylat, 3-Sulfopropylmethacrylat, Sulfomethylacrylamid, Sulfomethylmethacrylamid und Salze dieser Säuren. Die Copolymere können weiterhin 0 bis 30 Gew.-% ethylenisch ungesättigter C₄-C₈-Dicarbonsäuren, wie Maleinsäure, sowie 0 bis 30 Gew.-% wenigstens eines Monomers, das mit den zuvor genannten Monomeren copolymerisierbar ist, einpolymerisiert enthalten. Bei letzterem handelt es sich beispielsweise um C₁-C₄-Alkylester von (Meth)Acrylsäure, C₁-C₄-Hydroxyalkylester von (Meth)Acrylsäure, Acrylamid, Alkyl-substituiertes Acrylamid, N,N-Dialkyl-substituiertes Acrylamid, Vinylphosphonsäure, Vinylacetat, Allylalkohole, sulfonierte Allylalkohole, Styrol und andere Vinylaromaten, Acrylonitril, N-Vinylpyrrolidon, N-Vinylformamid, N-Vinylimidazol oder N-Vinylpyridin. Das gewichtsmittlere Molekulargewicht dieser Copolymere liegt im Bereich von 3000 bis 50000 Dalton. Besonders geeignet sind Copolymere mit etwa 77 Gew.-% wenigstens einer ethylenisch ungesättigten C₃-C₆-Monocarbonsäure und etwa 23 Gew.-% wenigstens einer ethylenisch ungesättigten Sulfonsäure.

Pfropfpolymere ungesättigter Carbonsäuren auf niedermolekulare Kohlenhydrate oder hydrierte Kohlenhydrate, vgl. US-A 5227446, DE-A 4415623 und DE-A 4313909, eignen sich ebenfalls. Geeignete ungesättigte Carbonsäuren sind hierbei beispielsweise Maleinsäure, Fumarsäure, Itaconsäure, Citraconsäure, Acrylsäure, Methacrylsäure, Crotonsäure und Vinylessigsäure sowie Mischungen aus Acrylsäure und Maleinsäure, die in Mengen von 40 bis 95 Gew.-%, bezogen auf die zu pfropfende Komponente, aufgepfropft werden. Zur Modifizierung können zusätzlich bis zu 30 Gew.-%, bezogen auf die zu pfropfende Komponente, weitere monoethylenisch ungesättigte Monomere einpolymerisiert vorliegen. Geeignete modifizierende Monomere sind die oben genannten Monomere der Gruppen (ii) und (iii). Als Pfropfgrundlage sind abgebaute Polysaccharide wie z.B. sauer oder enzymatisch abgebaute Stärken, Inuline oder Zellulose, Eiweisshydrolysate und reduzierte (hydrierte oder hydrierend aminierte) abgebaute Polysaccharide wie z.B. Mannit, Sorbit, Aminosorbit und N-Alkylglucamin geeignet sowie auch Polyalkylenglykole mit Molmassen mit bis zu M_{w} = 5000 wie z. B. Polyethylenglykole, Ethylenoxid/Propylenoxidbzw. Ethylenoxid/Butylenoxid bzw. Ethylenoxid/Propylenoxid/Butylenoxid-Blockcopolymere und alkoxylierte ein- oder mehrwertige C₁-C₂₂-Alkohole.(vgl. US-A-5756456).

Ebenfalls geeignet sind Polyglyoxylsäuren, wie sie beispielsweise in EP-B-001004, US-A-5399286, DE-A-4106355 und EP-A-656914 beschrieben sind. Die Endgruppen der Polyglyoxylsäuren können unterschiedliche Strukturen aufweisen.

Weiterhin sind Polyamidocarbonsäuren und modifizierte Polyamidocarbonsäuren geeignet; diese sind beispielsweise aus EP-A-454126, EP-B-511037, WO-A94/01486 und EP-A-581452 bekannt.

Auch Polyasparaginsäuren oder Cokondensate der Asparaginsäure mit weiteren Aminosäuren, C₄-C₂₅-Mono- oder -Dicarbonsäuren und/oder C₄-C₂₅-Mono- oder - Diaminen können als polymere carbonsäuregruppenhaltige Verbindungen eingesetzt werden. Besonders bevorzugt werden in phosphorhaltigen Säuren hergestellte, mit C₆-C₂₂-Mono- oder -Dicarbonsäuren bzw. mit C₆-C₂₂-Mono- oder -Diaminen modifizierte Polyasparaginsäuren eingesetzt.

Unter den polymeren carbonsäuregruppenhaltigen Verbindungen sind Polyacrylsäuren auch in teilweise oder vollständig neutralisierter Form, bevorzugt.

Als organische Builder eignen sich weiterhin Iminodibernsteinsäure, Oxydibernsteinsäure, Aminopolycarboxylate, Alkylpolyaminocarboxylate, Aminopolyalkylenphosphonate, Polyglutamate, hydrophob modifizierte Citronensäure wie z.B. Agaricinsäure, Poly-[alpha]-hydroxyacrylsäure, N-Acylethylendiamintriacetate wie Lauroylethylendiamintriacetat und Alkylamide der Ethylendiamintetraessigsäure wie EDTA-Talgamid.

Weiterhin können auch oxidierte Stärken als organische Builder verwendet werden.

Bevorzugt wird als Komponente b) ein Gemisch verschiedener Builder eingesetzt.

Bevorzugt enthält das Gemisch verschiedener Builder wenigstens zwei der folgenden Bestandteile: wenigstens ein Carbonat (z.B. Natriumcarbonat), wenigstens ein Silikat (z.B. Natriumdisilkat), wenigstens eine polymere carbonsäuregruppenhaltige Verbindung oder wenigstens eine polymere carbonsäuregruppenhaltige Verbindung, in der die Carbonsäuregruppen teilweise oder vollständig neutralisiert vorliegen (z.B. Polyacrylsäure), wenigstens eine (Poly)Hydroxycarbonsäure oder ein Salz davon (z.B. Zitronensäure oder ein Citrat), wenigstens eine Aminopolycarbonsäure oder ein Salz davon (z.B. Methylglycindiessigsäure oder ein Salz davon, z.B. ein Natriumsalz davon), wenigstens eine Phosphonsäure (z.B. 1-Hydroxyethan1-(1,1-diphosphonsäure); HEDP), wenigstens ein Phosphat. Besonders bevorzugt enthält das Gemisch wenigstens ein Carbonat, wenigstens ein Silikat und wenigstens eine polymere gegebenenfalls (teil)neutralisierte carbonsäuregruppenhaltige Verbindung sowie optional wenigstens einen der folgenden Bestandteile: wenigstens eine (Poly)Hydroxycarbonsäure oder ein Salz davon, wenigstens eine Phosphonsäure, wenigstens ein Phosphat. Speziell enthält das Gemisch wenigstens ein Carbonat, wenigstens ein Silikat, wenigstens eine polymere gegebenenfalls (teil)neutralisierte carbonsäuregruppenhaltige Verbindung, wenigstens eine (Poly)Hydroxycarbonsäure oder ein Salz davon und wenigstens eine Phosphonsäure, sowie optional wenigstens ein Phosphat.

In einem solchen Gemisch sind die Bestandteile vorzugsweise in folgenden Mengen enthalten:
b1) wenigstens ein Carbonat: 10 bis 50 Gew.-%;
b2) wenigstens ein Silikat: 1 bis 10 Gew.-%;
b3) wenigstens eine polymere gegebenenfalls (teil)neutralisierte carbonsäuregruppenhaltige Verbindung: 5 bis 20 Gew.-%;
b4) wenigstens eine (Poly)Hydroxycarbonsäure oder ein Salz davon: 0 bis 50 Gew.-%;
b5) wenigstens eine Aminopolycarbonsäure oder ein Salz davon: 0 bis 60 Gew.-%; b6) wenigstens eine Phosphonsäure: 0,2 bis 1 Gew.-%;
b7) wenigstens ein Phosphat: 0 bis 60 Gew.-%.

Die Gew.-%-Angaben beziehen sich dabei auf das Gesamtgewicht des Builders. Die Gewichtsmengen von b1) bis b7) ergänzen sich zu 100 Gew.-%.

### Komponente c)

Die erfindungsgemäßen Wasch- und Reinigungsmittel enthalten vorzugsweise wenigstens ein Enzym.

Geeignete Enzyme sind solche, wie sie üblicherweise als industrielle Enzyme eingesetzt werden. Dazu zählen sowohl Enzyme mit optimaler Aktivität im neutralen bis alkalischen pH-Bereich, als auch Enzyme mit optimaler Aktivität im sauren pH-Bereich.

Die Enzyme sind vorzugsweise ausgewählt unter Aminopeptidasen, Amylasen, Arabinasen, Carbohydrasen, Carboxypeptidasen, Catalasen, Cellulasen, Chitinasen, Cutinasen, Cyclodextringlycosyltransferasen, Deoxyribonucleasen, Esterasen, Galactanasen, Alpha-Galactosidasen, Beta-Galactosidasen, Glucanasen, Glucoamylasen, Alpha-Glucosidasen, Beta-Glucosidasen, Haloperoxidasen, Hydrolaseinvertasen, Isomerasen, Keratinasen, Laccasen, Lipasen, Mannanasen, Mannosidasen, Oxidasen, pectinolytischen Enzymen, Peptidoglutaminasen, Peroxidasen, Peroxygenasen, Phytasen, Polyphenoloxidasn, proteolytischen Enzymen, Ribonucleasen, Transglutaminasen, Transferasen, Xylanasen und Mischungen davon

Die Enzyme sind speziell ausgewählt unter Hydrolasen, wie Proteasen, Esterasen, Glucosidasen, Lipasen, Amylasen, Cellulasen, Mannanasen, anderen Glykosylhydrolasen und Gemischen der zuvor genannten Enzyme. Alle diese Hydrolasen tragen zur Schmutzauflösung und -entfernung von protein-, fett- oder stärkehaltigen Verschmutzungen bei. Zur Bleiche können auch Oxireduktasen eingesetzt werden. Besonders gut geeignet sind aus Bakterienstämmen oder Pilzen wie Bacillus subtilis, Bacillus licheniformis, Streptomyceus griseus und Humicola insolens gewonnene enzymatische Wirkstoffe.

Bevorzugte Enzyme werden im Folgenden genauer beschrieben:

### Proteasen:

Geeignete proteolytische Enzyme (Proteasen) können grundsätzlich tierischen, pflanzlichen oder mikrobiellen Ursprungs sein. Bevorzugt sind proteolytische Enzyme mikrobiellen Ursprungs. Dazu zählen auch chemisch oder genetisch modifizierte Mutanten.

Bevorzugte Proteasen sind Serinproteasen, Metalloproteasen oder trypsinartige Proteasen. Vorzugsweise wird eine alkalische mikrobielle Protease eingesetzt. Beispiele für alkalische Proteasen sind Subtilisine, speziell solche die von *Bacillus* abgeleitet sind, z.B. Subtilisin Novo, Subtilisin Carlsberg, Subtilisin 309, Subtilisin 147 und Subtilisin 168 (beschrieben in WO 89/06279). Beispiele für trypsinartige Proteasen sind Trypsin (z.B. aus Schwein oder Rind) und die z.B. in der WO 89/06270 beschriebene *Fusarium* Protease.

Bevorzugte kommerziell erhältliche Proteasen umfassen die unter folgenden Markenbezeichnungen erhältlichen Proteasen: Alcalase™, Savinase™, Primase™, Durazym™, Esperase™, Neutrase™ von Novozymes A/S (Dänemark), die von DuPont/Genencor unter den Markennamen Maxatase™, Maxacal™, Maxapem™, , PREFERENZ™ P, EXCELLENZ™ P Properase™, Purafect™ und Purafect™ OXP vertriebenen Produkte, und die von Solvay Enzymes unter den Markennamen Opticlean™ und Optimase™ vertriebenen Produkte.

Die erfindungsgemäßen Zusammensetzungen enthalten vorzugsweise wenigstens eine Protease in einer Menge von 0.00001 Gew.-% bis 30 Gew.-% Enzymprotein, besonders bevorzugt von 0.0001 Gew.-% bis 15 Gew.-% Enzymprotein, insbesondere 0.001 Gew.-% bis 10 Gew.-% Enzymprotein, spezieller 0.001 Gew.-% bis 5 Gew.-% Enzymprotein, bezogen auf das Gesamtgewicht der erfindungsgemäßen Polymerzusammensetzung.

### Lipasen:

Geeignete Lipasen können grundsätzlich von Bakterien oder Pilzen stammen. Dazu zählen auch chemisch oder genetisch modifizierte Mutanten.

Beispiele für geeignete Lipasen sind *Humicola lanuginosa* Lipase, wie z.B. beschrieben in EP 258 068 und EP 305 216, *Rhizomucor miehei* Lipase, wie z.B. beschrieben in EP 238 023, *Candida* Lipase, wie C. *antarctica* Lipase, z.B. die C. *antarctica* Lipasen A or B wie beschrieben in EP 214 761, *Pseudomonas Lipase, wie* P. *alcaligenes* und P. *pseudoal- caligenes* Lipase, wie z.B. beschrieben in EP 218 272, P. *cepacia* Lipase, wie z.B. beschrieben in EP 331 376, *P. stutzeri* Lipase, wie z.B. beschrieben in GB 1,372,034, *P. fluorescens* Lipase, *Bacillus Lipase,* z.B. eine *B. subtilis* Lipase (Dartois et al., (1993), Biochemica et Biophysica acta 1131, 253-260), *B. stearo- thermophilus* Lipase (JP 64/744992) und *B. pumilus* Lipase (WO 91/16422).

Weiterhin ist eine Anzahl geklonter Lipasen geeignet, umfassend *Penicillium camembertii* Lipase beschrieben von Yamaguchi et al., (1991), Gene 103, 61-67), *Geotricum candidum* Lipase (Schimada, Y. et al., (1989), J. Biochem., 106, 383-388), und verschiedene *Rhizopus* Lipasen, wie *R. delemar* Lipase (Hass, M. J et al., (1991), Gene 109, 117-113), *R. niveus* Lipase (Kugimiya et al., (1992), Biosci. Biotech. Biochem. 56, 716-719) und *R. oryzae* Lipase.

Weiterhin können andere Typen lipolytischer Enzyme eingesetzt werden, wie Cutinasen, z.B. eine von *Pseudomonas mendocina* abgeleitete Cutinase wie z.B. beschrieben in WO 88/09367, oder eine von *Fusarium solani pisi* abgeleitete cutinase (wie z.B. beschrieben in WO 90/09446).

Speziell geeignete Lipasen sind MI Lipase™, Luma Fast™ und Lipo-max™ (Genencor), Lipoclean™, Lipex™, Lipolex™ Lipolase™ und Lipolase Ultra™ (Novozymes A/S), und Lipase P "Amano" (Amano Pharmaceutical Co. Ltd.).

Die erfindungsgemäßen Zusammensetzungen enthalten vorzugsweise wenigstens eine Lipase in einer Menge von 0.00001 Gew.-% bis 30 Gew.-% Enzymprotein, besonders bevorzugt von 0.0001 Gew.-% bis 15 Gew.-% Enzymprotein, insbesondere 0.001 Gew.-% bis 10 Gew.-% Enzymprotein, spezieller 0.001 Gew.-% bis 5 Gew.-% Enzymprotein, bezogen auf das Gesamtgewicht der erfindungsgemäßen Polymerzusammensetzung.

### Amylasen:

Grundsätzlich sind alle α- und/oder β-Amylasen geeignet. Geeignete Amylasen können grundsätzlich von Bakterien oder Pilzen stammen. Dazu zählen auch chemisch oder genetisch modifizierte Mutanten.

Beispiele für geeignete Amylasen sind α-Amylasen erhalten aus einem speziellen Stamm von B. amyloliquefaciens oder B. *licheniformis,* detailliert beschrieben in GB 1,296,839. Weitere geeignete Amylasen umfassen Rückgrate, bie bekannt sind als SP707 aus *Bacillus* sp, AP1378, BSG *B. stearothermophilus* alpha-Amylase, SP690, SP722, und AA560 aus *Bacillus* sp. Geeignete kommerziell erhältliche Amylasen sind Stainzyme™, Stainzyme Plus™, Natalase™, Duramyl™, Termamyl™, Fungamyl™ and BAN™ (erhältlich von Novozymes A/S), und Rapidase™ PREFERENZ™ S, EXCELLENZ™ S und Maxamyl P™ (erhältlich von Genencor).

Die erfindungsgemäßen Zusammensetzungen enthalten vorzugsweise wenigstens eine Amylase in einer Menge von 0.00001 Gew.-% bis 30 Gew.-% Enzymprotein, besonders bevorzugt von 0.0001 Gew.-% bis 15 Gew.-% Enzymprotein, insbesondere 0.001 Gew.-% bis 10 Gew.-% Enzymprotein, spezieller 0.001 Gew.-% bis 5 Gew.-% Enzymprotein, bezogen auf das Gesamtgewicht der erfindungsgemäßen Polymerzusammensetzung.

### Cellulasen:

Grundsätzlich sind alle Cellullasen geeignet. Geeignete Cellulasen können grundsätzlich von Bakterien oder Pilzen stammen. Dazu zählen auch chemisch oder genetisch modifizierte Mutanten.

Geeignete Cellulasen sind in der in US 4,435,307 beschrieben. Dabei handelt es sich um pilzliche Cellulasen, hergestellt aus *Humicola insolens.* Speziell geeignet sind Cellulasen mit farbpflegenden Eigenschaften. Beispiele solcher Cellulasen sind in der EP 0 495 257 beschrieben.

Geeignete kommerziell erhältliche Cellulasen umfassen Celluzyme™ hergestellt aus einem Stamm von *Humicola insolens,* Carezyme™, Celluclean™, Endolase™, Whitezyme™ (Novozymes A/S), REVITALENZ™ (Dupont), Biotouch C™ (AB Enzymes) und KAC-500(B)™ (Kao Corporation).

Die erfindungsgemäßen Zusammensetzungen enthalten vorzugsweise wenigstens eine Cellulase in einer Menge von 0.00001 Gew.-% bis 30 Gew.-% Enzymprotein, besonders bevorzugt von 0.0001 Gew.-% bis 15 Gew.-% Enzymprotein, insbesondere 0.001 Gew.-% bis 10 Gew.-% Enzymprotein, spezieller 0.001 Gew.-% bis 5 Gew.-% Enzymprotein, bezogen auf das Gesamtgewicht der erfindungsgemäßen Polymerzusammensetzung.

### Peroxidasen / Oxidasen:

Geeignete Peroxidasen / Oxidasen können grundsätzlich von Pflanzen, Bakterien oder Pilzen stammen. Dazu zählen auch chemisch oder genetisch modifizierte Mutanten.

Peroxidase-Enzyme werden in Kombination mit Wasserstoffperoxid oder einer Wasserstoffperoxidquelle (z.B. einem Percarbonat, Perborat oder Persulfat) eingesetzt. Oxidase-Enzyme werden in Kombination mit Sauerstoff eingesetzt. Beide Enzymtypen werden zur "Lösungsbleiche" eingesetzt, um die Farbstoffübertragung von einem gefärbten Gewebe auf ein anderes Gewebe zu vermeiden, wenn diese gemeinsam in einer Flotte gewaschen werden. Der Einsatz kann vorzugsweise mit Wirkverbesserern erfolgen, die z.B. in der WO 94/12621 und WO 95/01426 beschrieben sind.

Die erfindungsgemäßen Zusammensetzungen enthalten vorzugsweise wenigstens eine Peroxidase oder Oxidase in einer Menge von 0.00001 Gew.-% bis 30 Gew.-% Enzymprotein, besonders bevorzugt von 0.0001 Gew.-% bis 15 Gew.-% Enzymprotein, insbesondere 0.001 Gew.-% bis 10 Gew.-% Enzymprotein, spezieller 0.001 Gew.-% bis 5 Gew.-% Enzymprotein, bezogen auf das Gesamtgewicht der erfindungsgemäßen Polymerzusammensetzung.

### Lyasen:

Grundsätzlich sind alle Lyasen geeignet. Geeignete Lyasen können grundsätzlich von Bakterien oder Pilzen stammen. Dazu zählen auch chemisch oder genetisch modifizierte Mutanten.

Geeignete Lyasen sind Pectatlyasen oder Pectinlyasen. Geeignete kommerziell erhältliche Lyasen umfassen XPect™ (Novozymes A/S) und PREFERENZ™ F (Dupont).

Die erfindungsgemäßen Zusammensetzungen enthalten vorzugsweise wenigstens eine Lyase in einer Menge von 0.00001 Gew.-% bis 30 Gew.-% Enzymprotein, besonders bevorzugt von 0.0001 Gew.-% bis 15 Gew.-% Enzymprotein, insbesondere 0.001 Gew.-% bis 10 Gew.-% Enzymprotein, spezieller 0.001 Gew.-% bis 5 Gew.-% Enzymprotein, bezogen auf das Gesamtgewicht der erfindungsgemäßen Polymerzusammensetzung.

Erfindungsgemäße Mittel können weitere Enzyme enthalten, die unter dem Begriff Hemicellulasen zusammengefaßt werden. Hierzu gehören beispielsweise Mannanasen, Xanthanlyasen, Pektinylasen (= Pektinasen), Pektinesterasen, Xyloglucanasen (=Xylanasen), Pullulanasen und β-Glucanasen.

Die erfindungsgemäßen Zusammensetzungen enthalten vorzugsweise wenigstens eine Hemicellulase in einer Menge von 0.00001 Gew.-% bis 30 Gew.-% Enzymprotein, besonders bevorzugt von 0.0001 Gew.-% bis 15 Gew.-% Enzymprotein, insbesondere 0.001 Gew.-% bis 10 Gew.-% Enzymprotein, spezieller 0.001 Gew.-% bis 5 Gew.-% Enzymprotein, bezogen auf das Gesamtgewicht der erfindungsgemäßen Polymerzusammensetzung.

Vorzugsweise enthält das erfindungsgemäße Wasch- und Reinigungsmittel wenigstens ein Enzym, das ausgewählt ist unter Proteasen, Amylasen, Mannanasen, Cellulasen, Lipasen, Pektinylasen und Mischungen davon.

Vorzugsweise enthält das erfindungsgemäße Wasch- und Reinigungsmittel wenigstens eine Protease und/oder Amylase.

Vorzugsweise enthält das erfindungsgemäße Wasch- und Reinigungsmittel eine Enzymmischung. Bevorzugt sind beispielsweise Enzymmischungen, die folgende Enzyme enthalten oder aus ihnen bestehen:
- Protease und Amylase,
- Protease und Lipase (bzw. lipolytisch wirkenden Enzymen),
- Protease und Cellulase,
- Amylase, Cellulase und Lipase (bzw. lipolytisch wirkenden Enzymen),
- Protease, Amylase und Lipase (bzw. lipolytisch wirkenden Enzymen)
- Protease, Lipase (bzw. lipolytisch wirkenden Enzymen) und Cellulase.

Die Enzyme können an Trägerstoffe adsorbiert sein, um sie gegen vorzeitige Zersetzung zu schützen.

Gegebenenfalls kann das erfindungsgemäße Wasch- und Reinigungsmittel noch Enzymstabilisatoren enthalten. Dazu zählen z.B. Calciumpropionat, Natriumformiat, Borsäuren, Boronsäuren und deren Salze, wie 4-Formylphenylboronsäure, Peptide und Peptidderivate, wie z.B. Peptidaldehyde, Polyole, wie 1,2-Propandiol, und Mischungen davon.

### Komponente d)

Bei den Bleichmitteln d) handelt es sich vorzugsweise um Bleichsysteme, die neben Bleichmitteln gegebenenfalls noch Bleichaktivatoren, Bleichkatalysatoren und/oder Bleichstabilisatoren enthalten.

Geeignete Bleichmittel sind beispielsweise Percarbonsäuren, z.B. Diperoxododecandicarbonsäure, Phthalimidopercapronsäure oder Monoperoxophthalsäure oder-terephthalsäure, Salze der Percarbonsäuren, z.B. Natriumpercarbonat, Addukte von Wasserstoffperoxid an anorganische Salze, z.B. Natriumperborat-Monohydrat, Natriumperborat-Tetrahydrat, Natriumcarbonat-Perhydrat oder Natriumphosphat-Perhydrat, Addukte von Wasserstoffperoxid an organische Verbindungen, z.B. Harnstoff-Perhydrat, oder von anorganischen Peroxosalzen, z.B. Alkalimetallpersulfaten, oder -peroxodisulfaten.

Als Bleichaktivatoren eignen sich beispielsweise polyacylierte Zucker, z.B. Pentaacetylglucose; Acyloxybenzolsulfonsäuren und deren Alkali- und Erdalkaiimetallsalze, z.B. Natrium-p-nonanoyloxybenzolsulfonat oder Natrium-p-benzoyloxybenzol sulfonat;
- N,N-diacylierte und N,N,N',N'-tetraacylierte Amine, z.B. N,N,N',N'-Tetraacetylmethylendiamin und -ethylendiamin (TAED), N,N-Diacetylanilin, N,N-Diacetyl-ptoluidin oder 1,3-diacylierte Hydantoine wie 1,3-Diacetyl-5,5-dimethylhydantoin; N-Alkyl-N-sulfonylcarbonamide, z.B. N-Methyl-N-mesylacetamid oder N-MethylN-mesylbenzamid; N-acylierte cyclische Hydrazide, acylierte Triazole oder Urazole, z.B. Monoacetylmaleinsäurehydrazid; O,N,N-trisubstituierte Hydroxylamine, z.B. O-Benzoyl-N,N-succinylhydroxylamin, O-Acetyl-N,N-succinylhydroxylamin oder O, N, N- Triacetylhydroxylamin; N,N'-Diacylsulfurylamide, z.B. N,N'-Dimethyl-N,N'-diacetylsulfurylamid oder N,N'- Diethyl-N,N'-dipropionylsulfurylamid; acylierte Lactame wie beispielsweise Acetylcaprolactam, Octanoylcaprolactam, Benzoylcaprolactam oder Carbonylbiscaprolactam; Anthranilderivate wie z.B. 2-Methylanthranil oder 2-Phenylanthranil; Triacylcyanurate, z.B. Triacetylcyanurat oder Tribenzoylcyanurat; Oximester und Bisoximester wie z.B. O-Acetylacetonoxim oder Bisisopropyliminocarbonat; Carbonsäureanhydride, z.B. Essigsäureanhydrid, Benzoesäureanhydrid, m-Chlorbenzoesäureanhydrid oder Phthalsäureanhydrid; Enolester wie z.B. Isopropenylacetat; 1,3-Diacyl-4,5-diacyloxy-imidazoline, z.B. 1,3-Diacetyl-4,5-diacetoxyimidazolin; Tetraacetylglycoluril und Tetrapropionylglycoluril; diacylierte 2,5-Diketopiperazine, z.B. 1,4-Diacetyl-2,5-diketopiperazin; ammoniumsubstituierte Nitrile wie z.B. N-Methylmorpholiniumacetonitrilmethylsulfat; Acylierungsprodukte von Propylendiharnstoff und 2,2-Dimethyl-propylendihamstoff, z.B. Tetraacetylpropylendihamstoff; a-Acyloxypolyacylmalonamide, z.B. a-Acetoxy-N,N'-diacetylmalonamid; Diacyl-dioxohexahydro-1 ,3,5-triazine, z.B. 1,5-Diacetyl-2,4-dioxohexahydro1 ,3,5-triazin; Benz-(4H)1,3-oxazin-4-one mit Alkylresten, z.B. Methyl, oder aromatischen Resten z.B. Phenyl, in der 2-Position.

Ein Bleichsystem aus Bleichmitteln und Bleichaktivatoren kann gegebenenfalls noch Bleichkatalysatoren enthalten. Geeignete Bleichkatalysatoren sind beispielsweise quaternierte Imine und Sulfonimine, die beispielsweise in US-A 5 360 569 und EP-A 453 003 beschrieben sind. Besonders wirksame Bleichkatalysatoren sind Mangankomplexe, die beispielsweise in der WO-A 94/21777 beschrieben sind. Solche Verbindungen werden im Falle ihres Einsatzes in den Wasch- und Reinigungsmitteln höchstens in Mengen bis 1 ,5 Gew.-%, insbesondere bis 0,5 Gew.-%, im Falle von sehr aktiven Mangankomplexen in Mengen bis zu 0,1 Gew.-%, eingearbeitet. Neben dem beschriebenen Bleichsystem aus Bleichmitteln, Bleichaktivatoren und gegebenenfalls Bleichkatalysatoren ist für die erfindungsgemäßen Wasch- und Reinigungsmittel auch die Verwendung von Systemen mit enzymatischer Peroxidfreisetzung oder von photoaktivierten Bleichsystemen möglich.

### Komponente f)

Die erfindungsgemäß verwendeten Polymerzusammensetzungen eignen sich alleine bereits zur Modifizierung der rheologischen Eigenschaften im Sinne einer Verdickung.

Um der erfindungsgemäßen Formulierung die erwünschte Viskosität zu verleihen, kann zusätzlich wenigstens ein Verdicker f) eingesetzt werden.

Geeignet sind grundsätzlich jedwede bekannten Verdicker (Rheologiemodifizierungsmittel), sofern sie keinen negativen Einfluss auf die Wirkung des Wasch- und Reinigungsmittels ausüben. Geeignete Verdicker können sowohl natürlichen Ursprungs als auch synthetischer Natur sein.

Beispiele für Verdicker natürlichen Ursprungs sind Xanthan, Johannisbrotkernmehl, Guarmehl, Carrageen, Agar, Tragant, Gummi arabicum, Alginate, modifizierte Stärken, wie Hydroxyethylstärke, Stärkephosphatester oder Stärkeacetate, Dextrine, Pektine und Cellulosederivate, wie Carboxymethylcellulose, Hydroxyethylcellulose, Hydroxypropylcellulose, Hydroxypropylmethylcellulose, Methylcellulose und dergleichen.

Verdicker natürlichen Ursprungs sind auch anorganische Verdicker, wie Polykieselsäuren und Tonmineralien, z.B. Schichtsilikate, wie auch die bei den Buildern genannten Silikate.

Beispiele für synthetische Verdicker sind Polyacryl- und Polymethacrylverbindungen, wie (teil)vernetzte Homopolymere der Acrylsäure, beispielsweise mit einem Allylether von Saccharose oder Pentaerythrit oder mit Propylen vernetzte Homopolymere der Acrylsäure (Carbomer), z.B. die Carbopol®-Marken von BF Goodridge (z.B. Carbopol® 676, 940, 941, 934 und dgl.) oder die Polygel®-Marken von 3V Sigma (z.B. Polygel® DA), Copolymere ethylenisch ungesättigter Mono- oder Dicarbonsäuren, beispielsweise Terpolymere von Acrylsäure, Methacrylsäure oder Maleinsäure mit Methyl- oder Ethylacrylat und einem (Meth)Acrylat, das sich von langkettigen ethoxylierten Alkoholen ableitet, beispielsweise die Acusol®-Marken von Rohm & Haas (z.B. Acusol® 820 oder 1206A), Copolymere von zwei oder mehr Monomeren, die ausgewählt sind unter Acrylsäure, Methacrylsäure und ihren C₁-C₄-Alkylestern, z.B. Copolymere von Methacrylsäure, Butylacrylat und Methylmethacrylat oder von Butylacrylat und Methylmethacrylat, z.B. die Aculyn®- und Acusol®-Marken von Rohm & Haas (z.B. Aculyn® 22, 28 oder 33 und Acusol® 810, 823 und 830), oder vernetzte hochmolekulare Acrylsäurecopolymere, beispielsweise mit einem Allylether von Saccharose oder Pentaerythrit vernetzte Copolymere von C₁₀-C₃₀-Alkylacrylaten mit einem oder mehreren Comonomeren, die ausgewählt sind unter Acrylsäure, Methacrylsäure und ihren C₁-C₄-Alkylestern (z.B. Carbopol® ETD 2623, Carbopol® 1382 oder Carbopol® AQUA 30 von Rohm & Haas).

Beispiele für synthetische Verdicker sind weiterhin Umsetzungsprodukte von Maleinsäurepolymeren mit ethoxylierten langkettigen Alkoholen, z.B. die Surfonic L-Serie von Texaco Chemical Co. oder Gantrez AN-119 von ISP; Polyethylenglykole, Polyamide, Polyimine und Polycarbonsäuren.

Geeignet sind auch Gemische der o.g. Verdicker.

Bevorzugte Verdicker sind Xanthane und die oben genannten Polyacryl- und Polymethacrylverbindungen.

### Komponente g)

Geeignete zusätzliche Tenside g), die von Komponente a) verschieden sind, können kationisch, anionisch, zwitterionisch oder nichtionisch sein. Geeignete Tenside sind die zuvor bei den Tensidsystemen genannte.

Geeignete organische Lösungsmittel g) sind ausgewählt unter ein- oder mehrwertigen Alkoholen, Alkanolaminen oder Glykolethern. Vorzugsweise sind sie ausgewählt unter Ethanol, n- oder i-Propanol, Butanolen, Glykol, Propan- oder Butandiol, Glycerin, Diglykol, Propyl- oder Butyldiglykol, Hexylenglycol, Ethylenglykolmethylether, Ethylenglykolethylether, Ethylenglykolpropylether, Ethylenglykolmono-n-butylether, Diethylenglykol-methylether, Diethylenglykolethylether, Propylenglykolmethyl-, -ethyl- oder -propylether, Dipropylenglykolmonomethyl- oder - ethylether, Di-isopropylenglykolmonomethyl- oder -ethylether, Methoxy-, Ethoxy- oder Butoxytriglykol, i-Butoxy-ethoxy-2-propanol, 3-Methyl-3-methoxybutanol, Propylen-glykol-t-butylether sowie Mischungen dieser Lösemittel.

Als Schauminhibitoren oder Entschäumer der Komponente g) kommen beispielsweise Seifen, Paraffine oder Silikonöle in Betracht, die gegebenenfalls auf Trägermaterialien aufgebracht sein können.

Geeignete Basen der Komponente g) sind Alkalimetallhydroxide, Erdalkalimetallhydroxide, Alkalimetallcarbonate, Erdalkalimetallcarbonate, Ammoniumcarbonat Alkalimetallhydrogencarbonate, Erdalkalimetallhydrogencarbonate, Ammoniumhydrogencarbonat und Mischungen davon. Vorzugsweise werden Na-, Li- und Mg-Carbonate bzw. -Hydrogencarbonate, insbesondere Natriumcarbonat und/oder Natriumhydrogencarbonat, eingesetzt.

Zusätzlich können die gelförmigen Wasch- oder Reinigungsmittel weitere Inhaltsstoffe g) enthalten, die die anwendungstechnischen und/oder ästhetischen Eigenschaften des gelförmigen Wasch- oder Reinigungsmittels weiter verbessern. In der Regel enthalten bevorzugte Mittel zusätzlich zu den zuvor genannten Komponenten wenigstens einen weiteren Zusatzstoff, der ausgewählt ist unter Elektrolyten, pH-Stellmitteln, Parfümträgern, Fluoreszenzmitteln, Hydrotropen, Antiredepositionsmitteln, optischen Aufhellern, Vergrauungsinhibitoren, Einlaufverhinderern, Knitterschutzmitteln, Farbübertragungsinhibitoren, antimikrobiellen Wirkstoffen, Antioxidantien, Korrosionsinhibitoren, Antistatika, Bügelhilfsmitteln, Phobier- und Imprägniermitteln, Quell- und Schiebefestmitteln sowie UV-Absorbern.

Um den ästhetischen Eindruck der gelförmigen Wasch- oder Reinigungsmittel zu verbessern, können sie mit geeigneten Farbstoffen eingefärbt werden. Bevorzugte Farbstoffe, deren Auswahl dem Fachmann keinerlei Schwierigkeit bereitet, besitzen eine hohe Lagerstabilität und Unempfindlichkeit gegenüber den übrigen Inhaltsstoffen der Mittel und gegen Licht sowie keine ausgeprägte Substantivität gegenüber Textilfasern, um diese nicht anzufärben.

### I & I-Reiniger

Die erfindungsgemäßen Polymerzusammensetzungen eignen sich speziell für industrielle und institutionelle Reiniger (I & I-Reiniger). Industrielle und institutionelle Reiniger sind typischerweise Waschmittel, Allzweckreiniger, Schaumreiniger, Gelreiniger, CIP-Reiniger (cleaning in place-Reiniger) für professionelle und in der Regel automatisierte Reinigungsvorgänge, z. B. in Großwäschereien, Molkereien, Brauereien, der Nahrungsmittel- oder Getränkeindustrie, der Pharmaindustrie bzw. der Galenik oder Sanitärreiniger.

Die Reiniger können stark basisch mit hohem Elektrolytgehalt sein und bei Bedarf Bleichmittel (wie Wasserstoffperoxid, Natriumhypochlorit) oder Desinfektionsmittel sowie Entschäumer enthalten (z. B. in der Flaschenreinigung). Auch die gängigen zuvor genannten Enzyme können in den industriellen und institutionellen Reinigern enthalten sein. Hinsichtlich der Reinigungsarten, für die sich die erfindungsgemäßen Formulierungen eignen, herrscht große Vielfalt. Beispielhaft seien Reinigungsbäder (stationär oder bewegt), Sprühreinigung, Ultraschall-, Dampfstrahl- und Hochdruckreinigung erwähnt, gegebenenfalls in Kombination mit mechanischer Reinigung, z. B. durch rotierende Bürsten.

Die genannten Formulierungen für die Reinigung schließen solche für die Industrie, das Verkehrswesen, Handel und Gewerbe und für den privaten Sektor ein. Im einzelnen seien beispielhaft genannt: Professionelle Wäschereien, professionelles Reinigungsgewerbe, rzaufbereitende Industrie, Metall- und metallverarbeitende Industrie, Automobil- und Automobilzulieferindustrie, Elektroindustrie, Elektronikindustrie, Photoindustrie und -gewerbe, Freizeitindustrie und -gewerbe, Baustoffindustrie, Brauindustrie und -gewerbe; Nahrungsmittelindustrie (z. B. Verarbeitung oder Herstellung von Fleisch-, Geflügel-Milch- Fischprodukten) Tiernahrungsmittelindustrie, Kosmetika-Industrie, Pharmaindustrie, Agroindustrie, Gastronomie, Gesundheitswesen, Handwerksbetriebe, und öffentliches Verkehrswesen. Beispiele für zu reinigende Objekte sind Anstaltswäsche, Klinikwäsche, Wäsche aus Wäschereiannahmen, Gebäude mit Wohn-, Büro- oder Geschäftsräumen der verschiedensten Art sowie Sanitärräumen, Lagerhäuser, Brauereien, Einzelhandelsgeschäfte, wie Bäckereien, Metzgereien und Supermärkte; Krankenhäuser, Pflegeheime, Altersheime, Verwaltungsgebäude, Fabrikgebäude, Arztpraxen; weiterhin Kraftfahrzeuge (PKW und LKW), Autobusse, Straßentankfahrzeuge (innen und außen), Eisenbahnkesselwagen, Personen- und Güterwagen sowie Luftfahrzeuge und Schiffe; ferner Gebäudefassaden, gekachelte oder gestrichene Wände, Fußböden aus Holz (Parkett, Dielen) mit Estrich oder textilen oder Kunststoffbelägen, Signal- und Beleuchtungseinrichtungen, Möbel, Geländer, Schildbrücken, Schilder, Warnbaken, Begrenzungspfähle, Kessel, Geschirr, Glasscheiben, Straßen und Wege, Hofbefestigungen, Straßen- und Eisenbahntunnel.

### Saurer Sanitärreiniger

Die erfindungsgemäße Polymerzusammensetzung eignet sich weiterhin vorteilhaft zur Herstellung von Reinigern für den Sanitärbereich, die wenigstens eine organische Säure enthalten. Saure Sanitärreiniger eignen sich insbesondere zur WC-Reinigung, zur Reini¬ gung von Waschbecken, Duschkabinen, Duschwannen und Schwimmbecken sowie Spülbecken im Küchenbereich. Sie sind beispielsweise wirksam bei der Entfernung von Kalk- und Urinsteinablagerungen und bei der Entfernung von Bakterien die sich üblicherweise auf den Kalk- und Urinsteinablagerungen bilden. Sie sorgen für Sauberkeit und beugen schlechten Gerüchen wirksam vor. Überraschenderweise wurde nun gefunden, dass die erfindungsgemäße gelförmige Polymerzusammensetzung eine hohe Verträglichkeit mit sauren Tensidzusammensetzungen aufweist und sich besonders vorteilhaft für den Einsatz in sauren Sanitärreinigern eignet.

Ein bevorzugter saurer Sanitärreiniger umfasst vorzugsweise folgende Bestandteile:
- wenigstens eine erfindungsgemäße gelförmige Polymerzusammensetzung,
- wenigstens eine organische Säure,
- gegebenenfalls Wasser,
- gegebenenfalls wenigstens einen Verdicker, und
   gegebenenfalls wenigstens einen weiteren Zusatzstoff,

Der saure Sanitärreiniger liegt vorzugsweise flüssig oder speziell gelförmig vor.

Geeignete organische Säuren sind Ameisensäure, Essigsäure, Citronensäure oder Methansulfonsäure. Besonders bevorzugt sind Essigsäure oder Citronensäure. Der erfindungsgemäße Reiniger enthält die organische Säure vorzugsweise in einer Menge von 1 bis 40 Gew.-%, insbesondere von 5 bis 15 Gew.-%, bezogen auf das Gesamtgewicht des Reinigers.

Der erfindungsgemäße Reiniger enthält vorzugsweise wenigstens ein Tensid in einer Menge von 0,5 bis 50 Gew.-%, insbesondere von 1 bis 15 Gew.-%, bezogen auf das Gesamtgewicht des Reinigers.

Geeignete Verdicker sind die zuvor als Komponente f) genannten, auf die hier in vollem Umfang Bezug genommen wird.

Geeignete Zusatzstoffe sind die zuvor als Komponente g) genannten, auf die hier in vollem Umfang Bezug genommen wird. Dazu zählen speziell Stabilisatoren, Farbstoffe und Duftstoffe.

### Klebstoffzusammensetzung

Die erfindungsgemäße Polymerzusammensetzung eingnet sich zur Herstellung von Klebstoffzusammensetzung die adhäsiv sind, d.h. sie sind in der Lage, Substrate zu verbinden, ohne dass die Substrate selbst merklich verändert werden, wobei der Zusammenhalt der verbundenen Substrate durch Adhäsionskräfte (Anziehungskräfte zwischen Adhäsiv und Substrat) und Kohäsion (innerer Zusammenhalt des Adhäsive) bestimmt wird.

Polymerzusammensetzungen zur Herstellung einer Klebstoffzusammensetzung werden erhalten durch radikalische Polymerisation einer Monomerzusammensetzung M), die wenigstens eine α,β-ethylenisch ungesättigte Carbonsäure A), wie zuvor definiert, enthält. Bevorzugte Monomere A) sind ausgewählt unter Acrylsäure, Methacrylsäure, Itaconsäure, Maleinsäure oder Fumarsäure. Die Säuregruppen können in protonierter Form oder in Form ihrer Salze vorliegen. Bevorzugt enthält die Monomerzusammensetzung 0,5 bis 15 Gew.-%, besonders bevorzugt 1 bis 10 Gew.-%, bezogen auf das Gesamtgewicht der Monomerzusammensetzung, wenigstens einer α,β-ethylenisch ungesättigte Carbonsäure A). Besonders bevorzugt ist Acrylsäure.

Geeignete Comonomere sind z.B. (Meth)acrylsäurealkylester mit einem C₁-C_{1O}-Alkylrest, wie Methylmethacrylat, Methylacrylat, n-Butylacrylat, Ethylacrylat und Ethylhexylacrylat. Insbesondere sind auch Mischungen der (Meth)acrylsäurealkylester geeignet. Vinylester von Carbonsäuren mit 1 bis 20 C-Atomen sind z.B. Vinyllaurat, Vinylstearat, Vinylpropionat, Versaticsäurevinylester und Vinylacetat. Als vinylaromatische Verbindungen kommen Vinyltoluol, α- und p-Methylstyrol, α-Butylstyrol, 4-n-Butylstyrol, 4-n-Decylstyrol und vorzugsweise Styrol in Betracht. Beispiele für Nitrile sind Acrylnitril und Methacrylnitril. Die Vinylhalogenide sind mit Chlor, Fluor oder Brom substituierte ethylenisch ungesättigte Verbindungen, bevorzugt Vinylchlorid und Vinylidenchlorid. Als Vinylether zu nennen sind z.B. Vinylmethylether oder Vinylisobutylether. Bevorzugt sind Vinylether von 1 bis 4 C-Atome enthaltenden Alkoholen. Geeignete Kohlenwasserstoffe mit 4 bis 8 C- Atomen und zwei olefinischen Doppelbindungen sind zum Beispiel Butadien, Isopren und Chloropren. Weitere Monomere sind z.B. auch Hydroxylgruppen enthaltende Monomere, insbesondere C₁-C_{1O}-Hydroxyalkyl(meth)acrylate oder (Meth)acrylamid. Als weitere Monomere seien darüber hinaus Phenyloxyethylglykolmono-(meth)acrylat, Glycidyl(meth)acrylat, Aminoalkyl(meth)acrylate wie z.B. 2-Aminoethyl-(meth)acrylat genannt. Alkylgruppen weisen vorzugsweise von 1 bis 20 C-Atome auf. Als weitere Monomere seien auch vernetzende Monomere genannt.

Bevorzugt als Polyetherkomponente PE) sind die zuvor genannten Polyetherole und speziell Polyethylenglycole.

Die Art und Menge der Monomere bzw. die Verhältnisse verschiedener Comonomere zueinander sind dabei derart, dass die Glasübergangstemperatur in dem zuvor genannten Bereich liegt.

Die erfindungsgemäßen Klebstoffzusammensetzungen können wenigstens ein Lösungs- bzw. Dispergiermittel enthalten. Das Lösungs- bzw. Dispergiermittel der Klebstoffzusammensetzung kann sowohl nur aus Wasser, als auch aus Mischungen aus Wasser und damit mischbaren Flüssigkeiten, wie Methanol oder Ethanol, bestehen. Vorzugsweise wird nur Wasser verwendet. Der pH-Wert der Klebstoffzusammensetzung wird vorzugsweise auf pH größer 4,5, insbesondere auf einen pH-Wert zwischen 5 und 9,5 eingestellt.

Die Klebstoffzusammensetzungen können allein aus der gelförmigen Polymerzusammensetzung bestehen. Die Klebstoffzusammensetzung kann jedoch auch noch weitere Zusatzstoffe enthalten, z.B. Füllstoffe, Farbstoffe, Verlaufsmittel, Verdicker (vorzugsweise Assoziativverdicker), Entschäumer, Pigmente, Netzmittel oder Tackifier (klebrigmachende Harze). Für eine bessere Benetzung von Oberflächen können die Klebstoffe Benetzungshilfsmittel, z. B. Fettalkoholethoxylate, Alkylphenolethoxylate, Nonylphenolethoxylate, Polyoxyethylene, Polyoxypropylene oder Natriumdodecylsulfonate enthalten. Die Menge an Zusatzstoffen beträgt im allgemeinen 0,05 bis 5 Gew.-Teile, insbesondere 0,1 bis 3 Gew.-Teile auf 100 Gew.-Teile Polymerzusammensetzung.

In einer Ausführungsform ist die Klebstoffzusammensetzung im Wesentlichen frei von Weichmachern. Weichmacher sind Zusätze, welche die Klebekraft herabsetzen. Im Wesentlichen frei von Weichmachern bedeutet, dass die Zusammensetzungen weniger als 1 Gew.-%, vorzugsweise weniger als 0,5 Gew.%, bezogen auf die Gesamtzusammensetzung an Weichmachern, besonders bevorzugt keinen Weichmacher enthalten.

Die erfindungsgemäße Klebstoffzusammensetzung ist vorzugsweise ein Haftklebstoff. Ein Haftklebstoff ist ein viskoelastischer Klebstoff, dessen abgebundener Film bei Raumtemperatur (20°C) in trockenem Zustand permanent klebrig und klebfähig bleibt. Die Klebung auf Substraten erfolgt sofort durch leichten Anpressdruck.

Die erfindungsgemäße Klebstoffzusammensetzung kann zur Verbindung wenigstens zweier Substrate oder zur Herstellung von selbstklebenden Artikeln verwendet werden. Die zu verbindenden Substrate oder selbstklebenden Artikel sind zumindest teilweise mit dem Haftklebstoff beschichtet. Vorzugsweise sind die selbstklebenden Artikel nach der Verklebung wiederabziehbar. Bei den selbstklebenden Artikeln kann es sich z.B. um Folien, Bänder oder Etiketten handeln. Geeignete Trägermaterialien sind z.B. Papier, Kunststofffolien und Metallfolien. Bei erfindungsgemäßen selbstklebenden Bändern kann es sich um einseitig oder beidseitig beschichtete Bänder aus den obigen Substanzen handeln. Bei erfindungsgemäßen selbstklebenden Etiketten kann es sich um Etiketten aus Papier oder einer thermoplastischen Folie handeln. Als thermoplastische Folie kommen z.B. Folien aus Polyolefinen (z.B. Polyethylen, Polypropylen), Polyole- fincopolymeren, Folien aus Polyestern (z.B. Polyethylenterephtalat) oder Polyacetat in Betracht. Die Oberflächen der thermoplastischen Polymerfolien sind vorzugweise coronabehandelt. Die Etiketten sind einseitig mit Klebstoff beschichtet. Bevorzugte Substrate für die selbstklebenden Artikel sind Papier und Polymerfolien. Bevorzugte selbstklebende Artikel sind Papieretiketten Die Artikel sind auf mindestens einer Oberfläche zumindest teilweise mit einer erfindungsgemäßen Klebstoffzusammensetzung beschichtet. Der Klebstoff kann nach üblichen Methoden wie Rakeln oder Streichen auf die Artikel aufgetragen werden. Die Auftragsmenge beträgt bevorzugt 0,1 bis 20 g, besonders bevorzugt 2 bis 15 g Feststoff pro m². Nach dem Auftragen kann ein Trocknungsschritt zur Entfernung des Wasser bzw. der Lösungsmittel folgen. Bei den Substraten, die miteinander verbunden werden oder auf die die selbstklebenden Artikel vorteilhaft aufgebracht werden können, kann es sich z.B. um Metall, Holz, Glas, Papier oder Kunststoff handeln. Die selbstklebenden Artikel eignen sich insbesondere zum Verkleben auf Verpackungsoberflächen, Kartons, Kunststoffverpackungen, Bücher, Fenster, Kraftfahrzeugkarosserien oder Karosserieteilen. Die selbstklebenden Artikel spezieller Ausführungsformen können von den Gegenständen von Hand wieder abgezogen werden, ohne dass ein Klebstoffrückstand auf dem Gegenstand verbleibt. Die Haftung auf den Gegenständen ist gut, trotzdem lassen sich die Folien, Bänder und Etiketten leicht abziehen.

### Kosmetische und pharmazeutische Mittel

Die erfindungsgemäßen Polymerzusammensetzungen eignen sich vorzugsweise zur Formulierung von kosmetischen und pharmazeutischen Produkten, speziell von wässrigen kosmetischen und pharmazeutischen Produkten.

Ein weiterer Gegenstand der Erfindung ist eine kosmetische oder pharmazeutische Zusammensetzung, enthaltend
a) wenigstens eine erfindungsgemäße gelförmige Polymerzusammensetzung, wie zuvor definiert,
b) wenigstens einen kosmetisch oder pharmazeutisch akzeptablen Wirkstoff, und
c) gegebenenfalls wenigstens einen von den Komponenten a) und b) verschiedenen kosmetisch oder pharmazeutisch akzeptablen Hilfsstoff.

Bevorzugt umfasst die Komponente c) wenigstens einen kosmetisch oder pharmazeutisch akzeptablen Träger.

Vorzugsweise ist die Träger-Komponente c) ausgewählt unter
i) Wasser,
ii) wassermischbaren organischen Lösungsmitteln, vorzugsweise C₂-C₄-Alkanolen, insbesondere Ethanol,
iii) Ölen, Fetten, Wachsen,
iv) von iii) verschiedenen Estern von C₆-C₃₀-Monocarbonsäuren mit ein-, zwei- oder dreiwertigen Alkoholen,
v) gesättigten acyclischen und cyclischen Kohlenwasserstoffen,
vi) Fettsäuren,
vii) Fettalkoholen,
viii) Treibgasen,
und Mischungen davon.

Geeignete hydrophile Komponenten c) sind die zuvor genannten organischen Lösungsmittel, Öle und Fette.

Speziell geeignete kosmetisch verträgliche Öl- bzw. Fettkomponenten c) sind in Karl-Heinz Schrader, Grundlagen und Rezepturen der Kosmetika, 2. Auflage, Verlag Hüthig, Heidelberg, S. 319 - 355 beschrieben, worauf hier Bezug genommen wird. Bei den erfindungsgemäßen kosmetischen Mitteln kann es sich um hautkosmetische, haarkosmetische, dermatologische, hygienische oder pharmazeutische Mittel handeln. Die erfindungsgemäße Polymerzusammensetzung eignet sich speziell auch zur Formulierung von Gelen.

Vorzugsweise liegen die erfindungsgemäßen Mittel in Form eines Gels, Schaums, Sprays, einer Salbe, Creme, Emulsion, Suspension, Lotion, Milch oder Paste vor. Gewünschtenfalls können auch Liposomen oder Mikrosphären eingesetzt werden. Die erfindungsgemäße Polymerzusammensetzung eignet sich speziell zur Formulierung von Gelen

Die erfindungsgemäßen kosmetischen Mittel können zusätzlich kosmetisch und/oder dermatologisch aktive Wirk- und Effektstoffe sowie Hilfsstoffe enthalten. Vorzugsweise enthalten die erfindungsgemäßen kosmetischen Mittel wenigstens eine erfindungsgemäße Polymerzusammensetzung, wie vorstehend definiert, wenigstens einen wie vorstehend definierten Träger C) und wenigstens einen davon verschiedenen Bestandteil, der vorzugsweise ausgewählt ist unter kosmetisch aktiven Wirkstoffen, Emulgatoren, Tensiden, Konservierungsmitteln, Parfümölen, zusätzlichen Verdickern, Haarpolymeren, Haar- und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Tönungsmitteln, Bräunungsmitteln, Farbstoffen, Pigmenten, Konsistenzgebern, Feuchthaltemitteln, Rückfettern, Collagen, Eiweißhydrolysaten, Lipiden, Antioxidantien, Entschäumern, Antistatika, Emollienzien und Weichmachern.

Zusätzlich zu den erfindungsgemäßen Polymerzusammensetzungen können die kosmetischen Mittel wenigstens ein konventionelles Verdickungsmittel enthalten. Dazu zählen z.B. Polysaccharide und organische Schichtmineralien wie Xanthan Gum® (Kelzan® der Fa. Kelco), Rhodopol® 23 (Rhone Poulenc) oder Veegum® (Firma R. T. Vanderbilt) oder Attaclay® (Firma Engelhardt). Geeignete Verdickungsmittel sind auch organische natürliche Verdickungsmittel (Agar-Agar, Carrageen, Tragant, Gummi arabicum, Alginate, Pektine, Polyosen, Guar-Mehl, Johannisbrotbaumkernmehl, Stärke, Dextrine, Gelatine, Casein) und anorganische Verdickungsmittel (Polykieselsäuren, Tonmiheralien wie Montmorillonite, Zeolithe, Kieselsäuren). Weitere Verdickungsmittel sind Polysaccharidgummen, beispielsweise Gummi arabicum, Agar, Alginate, Carrageene und ihre Salze, Guar, Guaran, Traganth, Gellan, Ramsan, Dextran oder Xanthan und ihre Derivate, z. B. propoxyliertes Guar, sowie ihre Mischungen. Andere Polysaccharidverdicker sind beispielsweise Stärken verschiedensten Ursprungs und Stärkederivate, z. B. Hydroxyethylstärke, Stärkephosphat- ester oder Stärkeacetate, oder Carboxymethylcellulose bzw. ihr Natriumsalz, Methyl-, Ethyl-, Hydroxyethyl-, Hydroxypropyl-, Hydroxypropyl-methyl- oder Hydroxyethyl-methyl- cellulose oder Celluloseacetat. Als Verdickungsmittel können weiterhin Schichtsilikate eingesetzt werden. Hierzu zählen beispielsweise die unter dem Handelsnamen Laponite® erhältlichen Magnesium-oder Natrium-Magnesium-Schichtsilikate der Firma Solvay Alkali sowie die Magnesiumsilikate der Firma Süd-Chemie.

Geeignete kosmetisch und/oder dermatologisch aktive Wirkstoffe sind z. B. Haut- und Haarpigmentierungsmittel, Bräunungsmittel, Bleichmittel, Keratin-härtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, desodorierende Wirkstoffe, sebostatische Wirkstoffe, Pflanzenextrakte, antierythimatös oder antiallergisch aktive Wirkstoffe und Mischungen davon.

Künstlich hautbräunende Wirkstoffe, die geeignet sind, die Haut ohne natürliche oder künstliche Bestrahlung mit UV-Strahlen zu bräunen, sind z. B. Dihydroxyaceton, Alloxan und Walnussschalenextrakt. Geeignete Keratin-härtende Stoffe sind in der Regel Wirkstoffe, wie sie auch in Antitranspirantien eingesetzt werden, wie z. B. Kaliumaluminiumsulfat, Aluminiumhydroxychlorid, Aluminiumlactat, etc. Antimikrobielle Wirkstoffe werden eingesetzt, um Mikroorganismen zu zerstören bzw. ihr Wachstum zu hemmen und dienen somit sowohl als Konservierungsmittel als auch als desodorierend wirkender Stoff, welcher die Entstehung oder die Intensität von Körpergeruch vermindert. Dazu zählen z. B. übliche, dem Fachmann bekannte Konservierungsmittel, wie p-Hydroxybenzoesäureester, Imidazolidinyl-Harnstoff, Formaldehyd, Sorbinsäure, Benzoesäure, Salicylsäure, etc. Derartige desodorierend wirkende Stoffe sind z. B. Zinkricinoleat, Triclosan, Undecylensäurealkylolamide, Citronensäuretriethylester, Chlorhexidin etc. Geeignete Lichtfilterwirkstoffe sind Stoffe, die UV-Strahlen im UV-Bund/oder UV-A-Bereich absorbieren. Geeignete UV-Filter sind die zuvor genannten. Geeignet sind weiterhin p-Aminobenzoesäureester, Zimtsäureester, Benzophenone, Campherderivate sowie UV-Strahlen abhaltende Pigmente, wie Titandioxid, Talkum und Zinkoxid. Geeignete Repellentwirkstoffe sind Verbindungen, die in der Lage sind, bestimmte Tiere, insbesondere Insekten, vom Menschen abzuhalten oder zu vertreiben. Dazu gehört z. B. 2-Ethyl-1,3-hexandiol, N,N-Diethyl-m-toluamid etc. Geeignete hyperemisierend wirkende Stoffe, welche die Durchblutung der Haut anregen, sind z. B. ätherische Öle, wie Latschenkiefer, Lavendel, Rosmarin, Wacholderbeere, Rosskastanienextrakt, Birkenblätterextrakt, Heublumenextrakt, Ethylacetat, Campher, Menthol, Pfefferminzöl, Rosmarinextrakt, Eukalyptusöl, etc. Geeignete keratolytisch und keratoplastisch wirkende Stoffe sind z. B. Salicylsäure, Kalziumthioglykolat, Thioglykolsäure und ihre Salze, Schwefel, etc. Geeignete Antischuppen-Wirkstoffe sind z. B. Schwefel, Schwefelpolyethylenglykolsorbitanmonooleat, Schwefelricinolpolyethoxylat, Zinkpyrithion, Aluminiumpyrithion, etc. Geeignete Antiphlogistika, die Hautreizungen entgegenwirken, sind z. B. Allantoin, Bisabolol, Dragosantol, Kamillenextrakt, Panthenol, etc.

Die erfindungsgemäßen kosmetischen Mittel können als kosmetischen Wirkstoff (wie auch gegebenenfalls als Hilfsstoff) wenigstens ein kosmetisch oder pharmazeutisch akzeptables Polymer enthalten. Dazu zählen ganz allgemein anionische, kationische, amphotere und neutrale Polymere.

Beispiele für anionische Polymere sind Copolymere von Acrylsäure und Acrylamid und deren Salze; Natriumsalze von Polyhydroxycarbonsäuren, wasserlösliche oder wasserdispergierbare Polyester, Polyurethane, z. B. Luviset PUR® der Fa. BASF, und Polyharnstoffe. Besonders geeignete Polymere sind Copolymere aus t-Butylacrylat, Ethylacrylat, Methacrylsäure (z. B. Luvimer® 100P), Copolymere aus Ethylacrylat und Methacrylsäure (z. B. Luvimer® MAE), Copolymere aus N-tert.-Butylacrylamid, Ethylacrylat, Acrylsäure (Ultrahold® 8, strong), Copolymere aus Vinylacetat, Crotonsäure und gegebenenfalls weitere Vinylester (z. B. Luviset®-Marken), Maleinsäureanhydridcopolymere, gegebenenfalls mit Alkohol umgesetzt, anionische Polysiloxane, z. B. carboxyfunktionelle, t-Butylacrylat, Methacrylsäure (z. B. Luviskol® VBM), Copolymere von Acrylsäure und Methacrylsäure mit hydrophoben Monomeren, wie z. B. C₄-C₃₀-Alkylester der (Meth)acrylsäure, C₄-C₃₀-Alkylvinylester, C₄-C₃₀-Alkylvinylether und Hyaluronsäure. Ein Beispiel für ein anionisches Polymer ist weiterhin das unter der Bezeichnung Luviset® Shape (INCI Name: Polyacrylate-22) erhältliche Methylmethacrylat/Methacrylsäure/Acrylsäure/Urethanacrylat-Copolymer. Beispiele für anionische Polymere sind weiterhin Vinylacetat/Crotonsäure-Copolymere, wie sie beispielsweise unter den Bezeichnungen Resyn® (National Starch) und Gafset® (GAF) im Handel sind und Vinylpyrrolidon/Vinylacrylat-Copolymere, erhältlich beispielsweise unter dem Warenzeichen Luviflex® (BASF). Weitere geeignete Polymere sind das unter der Bezeichnung Luviflex® VBM-35 (BASF) erhältliche Vinylpyrrolidon/Acrylat-Terpolymer und Natriumsulfonat-haltige Polyamide oder Natriumsulfonat-haltige Polyester. Weiterhin geeignet sind Vinylpyrrolidon/Ethylmethacrylat/Methacrylsäure-Copolymere wie sie von der Fa. Stepan unter den Bezeichnungen Stepanhold-Extra und -R1 vertrieben werden und die Carboset®-Marken der Fa. BF Goodrich.

Geeignete kationische Polymere sind z. B. kationische Polymere mit der Bezeichnung Polyquaternium nach INCI, z. B. Copolymere aus Vinylpyrrolidon/N-Vinylimidazoliumsalzen (Luviquat® FC, Luviquat® HM, Luviquat® MS, Luviset Clear®, Luviquat Supreme®, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidocopolymere (Polyquaternium-7) und Chitosan. Geeignete kationische (quaternisierte) Polymere sind auch Merquat® (Polymer auf Basis von Dimethyldiallylammoniumchlorid), Gafquat® (quaternäre Polymere, die durch Reaktion von Polyvinylpyrrolidon mit quaternären Ammoniumverbindungen entstehen), Polymer JR (Hydroxyethylcellulose mit kationischen Gruppen) und kationische Polymere auf pflanzlicher Basis, z. B. Guarpolymere, wie die Jaguar®-Marken der Fa. Rhodia.

Ganz besonders geeignete Polymere sind neutrale Polymere, wie Polyvinylpyrrolidone, Copolymere aus N-Vinylpyrrolidon und Vinylacetat und/oder Vinylpropionat, Polysiloxane, Polyvinylcaprolactam und andere Copolymere mit N-Vinylpyrrolidon, Polyethylenimine und deren Salze, Polyvinylamine und deren Salze, Cellulosederivate, Polyasparaginsäuresalze und Derivate. Dazu zählt beispielsweise Luviflex® Swing (teilverseiftes Copolymerisat von Polyvinylacetat und Polyethylenglykol, Fa. BASF).

Geeignete Polymere sind auch nichtionische, wasserlösliche bzw. wasserdispergierbare Polymere oder Oligomere, wie Polyvinylcaprolactam, z. B. Luviskol® Plus (BASF SE), oder Polyvinylpyrrolidon und deren Copolymere, insbesondere mit Vinylestern, wie Vinylacetat, z. B. Luviskol® VA 37, VA 55, VA 64, VA 73 (BASF SE); Polyamide, z. B. auf Basis von Itaconsäure und aliphatischen Diaminen, wie sie z. B. in der DE-A-43 33 238 beschrieben sind.

Geeignete Polymere sind auch amphotere oder zwitterionische Polymere, wie die unter den Bezeichnungen Amphomer® (National Starch) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. - Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette® (AMERCHOL) im Handel erhältlich sind, und Copolymere aus Hydroxyethylmethacrylat, Methylmethacrylat, N,N-Dimethylaminoethylmethacrylat und Acrylsäure (Jordapon®).

Geeignete Polymere sind auch nichtionische, siloxanhaltige, wasserlösliche oder -dispergierbare Polymere, z. B. Polyethersiloxane, wie Tegopren® (Fa. Goldschmidt) oder Belsil® (Fa. Wacker).

In einer speziellen Ausführung enthalten die erfindungsgemäßen Zusammensetzungen wenigstens ein Polymer, das als Verdicker wirkt.

Geeignete polymere Verdicker sind beispielsweise gegebenenfalls modifizierte polymere Naturstoffe (Carboxymethylcellulose und andere Celluloseether, Hydroxyethyl- und -propylcellulose und dergleichen) sowie synthetische polymere Verdicker (Polyacryl- und Polymethacryl-Verbindungen, Vinylpolymere, Polycarbonsäuren, Polyether, Polyimine, Polyamide). Dazu zählen die zum Teil bereits zuvor genannten Polyacryl- und Polymethacryl-Verbindungen, beispielsweise die hochmolekularen mit einem Polyalkenylpolyether, insbesondere einem Allylether von Saccharose, Pentaerythrit oder Propylen, vernetzten Homopolymere der Acrylsäure (INCI-Bezeichnung: Carbomer). Solche Polyacrylsäuren sind u. a. von der Fa. BF Goodrich unter dem Handelsnamen Carbopol® erhältlich, z. B. Carbopol 940 (Molekulargewicht ca. 4000000 Dalton), Carbopol 941 (Molekulargewicht ca. 1250000 Dalton) oder Carbopol 934 (Molekulargewicht ca. 3000000 Dalton). Weiterhin fallen darunter Acrylsäure-Copolymere, die beispielsweise von der Fa. Rohm & Haas unter den Handelsnamen Aculyn® und Acusol® erhältlich sind, z. B. die anionischen, nichtassoziativen Polymere Aculyn 22, Aculyne 28, Aculyn 33 (vernetzt), Acusol 810, Acusol 823 und Acusol 830 (CAS 25852-37-3). Speziell geeignet sind auch assoziative Verdicker, z.B. auf Basis modifizierter Polyurethane (HEUR) oder hydrophob modifizierter Acryl- oder Methacrylsäure-Copolymere (HASE-Verdicker, High Alkali Swellable Emulsion).

Nach einer bevorzugten Ausführungsform handelt es sich bei den erfindungsgemäßen Mitteln um ein Hautreinigungsmittel.

Bevorzugte Hautreinigungsmittel sind Seifen von flüssiger bis gelförmiger Konsistenz, wie Transparentseifen, Luxusseifen, Deoseifen, Cremeseifen, Babyseifen, Hautschutzseifen, Abrasivseifen und Syndets, pasteuse Seifen, Schmierseifen und Waschpasten, flüssige Wasch-, Dusch- und Badepräparate, wie Waschlotionen, Duschbäder und -gele, Schaumbäder, Ölbäder und Scrub-Präparate, Rasierschäume, -lotionen und -cremes.

Nach einer weiteren bevorzugten Ausführungsform handelt es sich bei den erfindungsgemäßen Mitteln um kosmetische Mittel zur Pflege und zum Schutz der Haut, Nagelpflegemittel oder Zubereitungen für die dekorative Kosmetik.

Geeignete hautkosmetische Mittel sind z. B. Gesichtswässer, Gesichtsmasken, Deodorantien und andere kosmetische Lotionen. Mittel für die Verwendung in der dekorativen Kosmetik umfassen beispielsweise Abdeckstifte, Theaterfarben, Mascara und Lidschatten, Lippenstifte, Kajalstifte, Eyeliner, Rouges, Puder und Augenbrauenstifte.

Außerdem können die erfindungsgemäßen Polymerzusammensetzungen verwendet werden in Nose-Strips zur Porenreinigung, in Antiaknemitteln, Repellents, Rasiermitteln, Haarentfernungsmitteln, Intimpflegemitteln, Fußpflegemitteln sowie in der Babypflege.

Bei den erfindungsgemäßen Hautpflegemitteln handelt es sich insbesondere um W/O- oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen.

Hautkosmetische und dermatologische Mittel auf Basis der zuvor beschriebenen erfindungsgemäßen Polymerzusammensetzungen zeigen vorteilhafte Wirkungen. Die Polymere können unter anderem zur Feuchthaltung und Konditionierung der Haut und zur Verbesserung des Hautgefühls beitragen. Durch Zusatz der erfindungsgemäßen Polymere kann in bestimmten Formulierungen eine erhebliche Verbesserung der Hautverträglichkeit erreicht werden.

Hautkosmetische und dermatologische Mittel enthalten vorzugsweise wenigstens eine erfindungsgemäße Polymerzusammensetzung in einem Anteil von etwa 0,001 bis 30 Gew.-%, vorzugsweise 0,01 bis 20 Gew.-%, ganz besonders bevorzugt 0,1 bis 12 Gew.-%, bezogen auf das Gesamtgewicht des Mittels.

Je nach Anwendungsgebiet können die erfindungsgemäßen Mittel in einer zur Hautpflege geeigneten Form, wie z. B. als Creme, Schaum, Gel, Stift, Mousse, Milch, Spray (Pumpspray oder treibmittelhaltiger Spray) oder Lotion appliziert werden.

Die hautkosmetischen Zubereitungen können neben den erfindungsgemäßen Polymerzusammensetzungen und geeigneten Trägern noch weitere in der Hautkosmetik übliche Wirkstoffe und Hilfsstoffe, wie zuvor beschrieben, enthalten. Dazu zählen vorzugsweise Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Bräunungsmittel, Collagen, Eiweißhydrolysate, Stabilisatoren, pH-Wert-Regulatoren, Farbstoffe, Salze, Verdicker, Gelbildner, Konsistenzgeber, Silicone, Feuchthaltemittel, Rückfetter und weitere übliche Additive.

Bevorzugte Öl- und Fettkomponenten der hautkosmetischen und dermatologischen Mittel sind die zuvor genannten mineralischen und synthetischen Öle, wie z. B. Paraffine, Siliconöle und aliphatische Kohlenwasserstoffe mit mehr als 8 Kohlenstoffatomen, tierische und pflanzliche Öle, wie z. B. Sonnenblumenöl, Kokosöl, Avocadoöl, Olivenöl, Lanolin, oder Wachse, Fettsäuren, Fettsäureester, wie z. B. Triglyceride von C₆-C₃₀-Fettsäuren, Wachsester, wie z. B. Jojobaöl, Fettalkohole, Vaseline, hydriertes Lanolin und acetyliertes Lanolin sowie Mischungen davon.

Man kann die erfindungsgemäße Polymerzusammensetzung auch mit herkömmlichen Polymeren, wie zuvor beschrieben, abmischen, falls spezielle Eigenschaften eingestellt werden sollen.

Zur Einstellung bestimmter Eigenschaften wie z. B. Verbesserung der Haptik, des Spreitverhaltens, der Wasserresistenz und/oder der Bindung von Wirk- und Hilfsstoffen, wie Pigmenten, können die hautkosmetischen und dermatologischen Zubereitungen zusätzlich auch konditionierende Substanzen auf Basis von Siliconverbindungen enthalten. Geeignete Siliconverbindungen sind beispielsweise Polyalkylsiloxane, Polyarylsiloxane, Polyarylalkylsiloxane, Polyethersiloxane oder Siliconharze.

Die Herstellung der kosmetischen oder dermatologischen Zubereitungen erfolgt nach üblichen, dem Fachmann bekannten Verfahren.

Bevorzugt liegen die kosmetischen und dermatologischen Mittel in Form von Emulsionen insbesondere als Wasser-in-Öl-(W/O)- oder Öl-in-Wasser-(O/W)-Emulsionen vor. Es ist aber auch möglich, andere Formulierungsarten zu wählen, beispielsweise Hydrodispersionen, Gele, Öle, Oleogele, multiple Emulsionen, beispielsweise in Form von W/O/W- oder O/W/O-Emulsionen, wasserfreie Salben bzw. Salbengrundlagen, usw.

Die Herstellung von Emulsionen erfolgt nach bekannten Methoden. Die Emulsionen enthalten neben wenigstens einer erfindungsgemäßen Polymerzusammensetzung in der Regel übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser. Die Auswahl der Emulsionstyp-spezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, dritter Teil, worauf hiermit ausdrücklich Bezug genommen wird.

Eine geeignete Emulsion, z. B. für eine Hautcreme etc., enthält im Allgemeinen eine wässrige Phase, die mittels eines geeigneten Emulgatorsystems in einer Öl- oder Fettphase emulgiert ist.

Bevorzugte Fettkomponenten, welche in der Fettphase der Emulsionen enthalten sein können, sind: Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Ricinusöl, Sesamöl, Olivenöl, Jojobaöl, Karite-Öl, Hoplostethus-Öl; mineralische Öle, deren Destillationsbeginn unter Atmosphärendruck bei ca. 250°C und deren Destillationsendpunkt bei 410°C liegt, wie z. B. Vaselinöl; Ester gesättigter oder ungesättigter Fettsäuren, wie Alkylmyristate, z. B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Siliconöle, wie Dimethylpolysiloxan, Methylphenylpolysiloxan und das Siliconglykol-Copolymer, Fettsäuren und Fettalkohole enthalten.

Es können auch Wachse verwendet werden, wie z. B. Carnaubawachs, Candilillawachs, Bienenwachs, mikrokristallines Wachs, Ozokeritwachs und Ca-, Mg- und Al-Oleate, -Myristate, -Linoleate und -Stearate.

Weiterhin kann eine erfindungsgemäße Emulsion als O/W-Emulsion vorliegen. Eine derartige Emulsion enthält üblicherweise eine Ölphase, Emulgatoren, die die Ölphase in der Wasserphase stabilisieren, und eine wässrige Phase, die üblicherweise verdickt vorliegt. Als Emulgatoren kommen vorzugsweise O/W-Emulgatoren, wie Polyglycerinester, Sorbitanester oder teilveresterte Glyceride, in Betracht.

Nach einer weiteren bevorzugten Ausführungsform handelt es sich bei den erfindungsgemäßen Mitteln um ein Duschgel, eine Shampoo-Formulierung oder ein Badepräparat.

Solche Formulierungen enthalten wenigstens eine erfindungsgemäße Polymerzusammensetzung sowie üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, Parfümölen, Farbstoffen, organischen Säuren, Konservierungsstoffen und Antioxidantien sowie Verdickern/Gelbildnern, Hautkonditioniermitteln und Feuchthaltemitteln.

Diese Formulierungen enthalten vorzugsweise 2 bis 50 Gew.-%, bevorzugt 5 bis 40 Gew.-%, besonders bevorzugt 8 bis 30 Gew.-% Tenside, bezogen auf das Gesamtgewicht der Formulierung.

In den Wasch-, Dusch- und Badepräparaten können alle in Körperreinigungsmitteln üblicherweise eingesetzten anionischen, neutralen, amphoteren oder kationischen Tenside verwendet werden.

Geeignete Tenside sind die zuvor genannten.

Weiterhin können die Duschgel-/Shampoo-Formulierungen zusätzliche Verdicker, wie z. B. Kochsalz, PEG-55, Propylenglykol-Oleat, PEG-120-Methylglucosedioleat und andere, sowie Konservierungsmittel, weitere Wirk- und Hilfsstoffe und Wasser enthalten.

Nach einer weiteren bevorzugten Ausführungsform handelt es sich bei den erfindungsgemäßen Mitteln um ein Haarbehandlungsmittel.

Erfindungsgemäße Haarbehandlungsmittel enthalten vorzugsweise wenigstens eine erfindungsgemäße Polymerzusammensetzung in einer Menge im Bereich von etwa 0,1 bis 30 Gew.-%, bevorzugt 0,5 bis 20 Gew.-%, bezogen auf das Gesamtgewicht des Mittels.

Vorzugsweise liegen die erfindungsgemäßen Haarbehandlungsmittel in Form eines Schaumfestigers, Haarmousses, Haargels, Shampoos, Haarsprays, Haarschaums, Spitzenfluids, Egalisierungsmittels für Dauerwellen oder "Hot-Oil-Treatments" vor. Je nach Anwendungsgebiet können die haarkosmetischen Zubereitungen als (Aerosol-)Spray, (Aerosol-)Schaum, Gel, Gelspray, Creme, Lotion oder Wachs appliziert werden. Haarsprays umfassen dabei sowohl Aerosolsprays als auch Pumpsprays ohne Treibgas. Haarschäume umfassen sowohl Aerosolschäume wie auch Pumpschäume ohne Treibgas. Haarsprays und Haarschäume umfassen vorzugsweise überwiegend oder ausschließlich wasserlösliche oder wasserdispergierbare Komponenten. Sind die in den erfindungsgemäßen Haarsprays und Haarschäumen eingesetzten Verbindungen wasserdispergierbar, können sie in Form von wässrigen Mikrodispersionen mit Teilchendurchmessern von üblicherweise 1 bis 350 nm, bevorzugt 1 bis 250 nm, zur Anwendung gebracht werden. Die Feststoffgehalte dieser Präparate liegen dabei üblicherweise in einem Bereich von etwa 0,5 bis 20 Gew.-%. Diese Mikrodispersionen benötigen in der Regel keine Emulgatoren oder Tenside zu ihrer Stabilisierung.

Die erfindungsgemäßen haarkosmetischen Formulierungen enthalten in einer bevorzugten Ausführungsform
a) 0,05 bis 5 Gew.-%, vorzugsweise 0,1 bis 3 Gew.-%, wenigstens einer erfindungsgemäßen Polymerzusammensetzung,
b) 20 bis 99,95 Gew.-% Wasser und/oder Alkohol,
c) 0 bis 50 Gew.-% wenigstens eines Treibgases,
d) 0 bis 5 Gew.-% wenigstens eines Emulgators,
e) 0,05 bis 5 Gew.-% wenigstens eines kosmetisch aktiven Wirkstoffs, sowie
f) 0 bis 20 Gew.-%, vorzugsweise 0,1 bis 10 Gew.-%, wenigstens eines von a) bis e) und g) verschiedenen wasserlöslichen oder wasserdispergierbaren Polymers,
g) 0 bis 45 Gew.-%, vorzugsweise 0,05 bis 25 Gew.-%, weitere Bestandteile,
wobei sich die Komponenten a) bis g) zu 100 Gew.-% addieren.

Unter Alkohol sind alle in der Kosmetik üblichen Alkohole zu verstehen, z. B. Ethanol, Isopropanol, n-Propanol.

Unter weiteren Bestandteilen sind die in der Kosmetik üblichen Zusätze zu verstehen, beispielsweise Treibmittel, Entschäumer, grenzflächenaktive Verbindungen, d. h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z. B. Konservierungsmittel, Parfümöle, Trübungsmittel, Wirkstoffe, UV-Filter, Pflegestoffe wie Panthenol, Collagen, Vitamine, Eiweißhydrolysate, Alpha- und Beta-Hydroxycarbonsäuren, Stabilisatoren, pH-Wert-Regulatoren, Farbstoffe, Viskositätsregulierer, Gelbildner, Salze, Feuchthaltemittel, Rückfetter, Komplexbildner und weitere übliche Additive.

Weiterhin zählen hierzu alle in der Kosmetik bekannten Styling- und Conditionerpolymere, die in Kombination mit den erfindungsgemäßen Polymerisaten eingesetzt werden können, falls ganz spezielle Eigenschaften eingestellt werden sollen.

Als herkömmliche Haarkosmetik-Polymere eignen sich beispielsweise die zuvor genannten kationischen, anionischen, neutralen, nichtionischen und amphoteren Polymere, auf die hier Bezug genommen wird.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon Copolyole (CTFA) und aminofunktionelle Silikonverbindungen wie Amodimethicone (CTFA).

Die erfindungsgemäßen Polymerzusammensetzungen eignen sich insbesondere als Verdicker in Haarstyling-Zubereitungen, insbesondere Haarschäumen und Haargelen. Als Emulgatoren können alle in Haarschäumen üblicherweise eingesetzten Emulgatoren verwendet werden. Geeignete Emulgatoren können nichtionisch, kationisch bzw. anionisch oder amphoter sein.

Die erfindungsgemäßen Polymerzusammensetzungen eignen sich auch für Styling-Gele. Als zusätzliche Gelbildner können alle in der Kosmetik üblichen Gelbildner eingesetzt werden. Hierzu wird auf die zuvor genannten konventionellen Verdicker Bezug genommen.

Die erfindungsgemäßen Polymerzusammensetzungen eignen sich auch für Shampooformulierungen, die zusätzlich übliche Tenside enthalten.

In den Shampooformulierungen können zur Erzielung bestimmter Effekte übliche Konditioniermittel in Kombination mit den erfindungsgemäßen Polymerzusammensetzungen eingesetzt werden. Hierzu zählen beispielsweise die zuvor genannten kationischen Polymere mit der Bezeichnung Polyquaternium nach INCI, insbesondere Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidcopolymere (Polyquaternium-7). Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon Copolyole (CTFA) und aminofunktionelle Silikonverbindungen wie Amodimethicone (CTFA). Ferner können kationische Guarderivate wie Guarhydroxypropyltrimoniumchlorid (INCI) verwendet werden.

Die erfindungsgemäßen Polymerzusammensetzungen eignen sich ebenso für die Verwendung in pharmazeutischen Zubereitungen jeder Art und im Überzug solcher pharmazeutischer Zubereitungen.

Ein weiterer Gegenstand der Erfindung ist die Verwendung einer Verbindung der Formel (I) oder der Formel (I.1), wie zuvor definiert, als Hilfsstoff in der Pharmazie.

Typische pharmazeutische Zusammensetzungen enthalten
A) wenigstens eine erfindungsgemäße Polymerzusammensetzung, wie zuvor definiert,
B) wenigstens einen pharmazeutisch akzeptablen Wirkstoff und
C) gegebenenfalls wenigstens einen weiteren, von A) und B) verschiedenen pharmazeutisch akzeptablen Hilfsstoff.

Pharmazeutisch akzeptable Hilfsstoffe C) sind die im Bereich der Pharmazie, der Lebensmitteltechnologie und angrenzenden Gebieten bekanntermaßen verwendbaren Hilfsstoffe, insbesondere die in einschlägigen Arzneibüchern (z. B. DAB, Ph. Eur., BP, NF) gelisteten sowie andere Hilfsstoffe, deren Eigenschaften einer physiologischen Anwendung nicht entgegenstehen.

Geeignete Hilfsstoffe C) können sein: Gleitmittel, Netzmittel, emulgierende und suspendierende Mittel, konservierende Mittel, Antioxidantien, Antireizstoffe, Chelatbildner, Emulsionsstabilisatoren, Filmbildner, Gelbildner, Geruchsmaskierungsmittel, Harze, Hydrokolloide, Lösemittel, Lösungsvermittler, Neutralisierungsmittel, Permeationsbeschleuniger, Pigmente, quaternäre Ammoniumverbindungen, Rückfettungs- und Überfettungsmittel, Salben-, Creme- oder Öl-Grundstoffe, Siliconderivate, Stabilisatoren, Sterilantien, Treibmittel, Trocknungsmittel, Trübungsmittel, zusätzliche Verdicker, Wachse, Weichmacher, Weißöle. Eine diesbezügliche Ausgestaltung beruht auf fachmännischem Wissen, wie sie beispielsweise in Fiedler, H. P. Lexikon der Hilfsstoffe für Pharmazie, Kosmetik und angrenzende Gebiete, 4. Aufl., Aulendorf: ECV-Editio-Kantor-Verlag, 1996, dargestellt sind.

Zur Herstellung von erfindungsgemäßen pharmazeutischen Mitteln können die Wirkstoffe mit einem geeigneten Exzipienten vermischt oder verdünnt werden. Exzipienten können feste, halb feste oder flüssige Materialien sein, die als Vehikel, Träger oder Medium für den Wirkstoff dienen können. Die Zumischung weiterer Hilfsstoffe erfolgt gewünschtenfalls in der dem Fachmann bekannten Weise. Insbesondere handelt es sich dabei um wässrige Lösungen bzw. Solubilisate zur oralen oder zur parenteralen Applikation. Des Weiteren eignen sich die erfindungsgemäß zu verwendenden Copolymere auch zum Einsatz in oralen Darreichungsformen wie Tabletten, Kapseln, Pulvern, Lösungen. Hier können sie den schwerlöslichen Arzneistoff mit einer erhöhten Bioverfügbarkeit zur Verfügung stellen. Bei der parenteralen Applikation können neben Solubilisaten auch Emulsionen, beispielsweise Fettemulsionen eingesetzt werden.

Pharmazeutische Formulierungen der oben genannten Art können durch Verarbeiten der erfindungsgemäßen Polymerzusammensetzung mit pharmazeutischen Wirkstoffen nach herkömmlichen Methoden und unter Einsatz bekannter und neuer Wirkstoffe erhalten werden.

Der Gehalt an wenigstens einer erfindungsgemäßen Polymerzusammensetzung in den pharmazeutischen Mitteln liegt, abhängig vom Wirkstoff, im Bereich von 0,01 bis 50 Gew.-%, bevorzugt 0,1 bis 40 Gew.-%, besonders bevorzugt 1 bis 30 Gew.-%, bezogen auf das Gesamtgewicht des Mittels.

Zur Herstellung der erfindungsgemäßen pharmazeutischen Mittel eignen sich prinzipiell alle pharmazeutischen Wirkstoffe und Pro-Drugs. Hierzu zählen Benzodiazepine, Antihypertensiva, Vitamine, Cytostatika - insbesondere Taxol, Anästhetika, Neuroleptika, Antidepressiva, Antibiotika, Antimykotika, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, durchblutungsfördernde Mittel, Diuretika, Diagnostika, Corticoide, Cholinergika, Gallenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel. Beispiele für geeignete pharmazeutische Wirkstoffe sind die insbesondere die in den Absätzen 0105 bis 0131 der US 2003/0157170 genannten Wirkstoffe.

Neben der Anwendung in der Kosmetik und in der Pharmazie eignen sich die erfindungsgemäßen Polymerzusammensetzungen auch im Lebensmittelbereich. Zu den Lebensmittelzubereitungen sind im Rahmen der vorliegenden Erfindung auch Nahrungsergänzungsmittel wie z. B. Lebensmittelfarbstoffe enthaltende Zubereitungen und diätetische Lebensmittel zu verstehen. Darüber hinaus eigenen sich die erfindungsgemäßen Polymerzusammensetzungen auch für Futtermittelzusätze für die Tierernährung.

Außerdem eignen sich die erfindungsgemäßen Polymerzusammensetzungen zur Herstellung von Zubereitungen von Nahrungsergänzungsmitteln wie wasserunlöslichen Vitaminen und Provitaminen wie Vitamin A, Vitamin A-Acetat, Vitamin D, Vitamin E, Tocopherol-Derivate wie Tocopherolacetat und Vitamin K.

### Pflanzenschutzmittel

Die erfindungsgemäße Polymerzusammensetzung eignet sich weiterhin vorteilhaft zur Herstellung von Pflanzenschutzmitteln. Überraschenderweise wurde gefunden, dass die erfindungsgemäße gelförmige Polymerzusammensetzung eine hohe Verträglichkeit mit einer Vielzahl an Pflanzenschutzwirkstoffen und Adjuvantien aufweist.

Ein bevorzugtes Pflanzenschutzmittel umfasst vorzugsweise folgende Bestandteile:
- wenigstens eine erfindungsgemäße gelförmige Polymerzusammensetzung,
- wenigstens einen Wirkstoff,
- gegebenenfalls wenigstens ein Adjuvans,
- gegebenenfalls Wasser,
- gegebenenfalls wenigstens einen Verdicker, und
gegebenenfalls wenigstens einen weiteren Zusatzstoff,

Geeignete Wirkstoffe zur Anwendung in den erfindungsgemäßen Pflanzenschutzmitteln sind beispielsweise solche, die zur Gruppe der Herbizide, Fungizide, Insektizide, Wachstumsregulatoren, Safener, Acarizide, Molluskizide und Nematizide gehören.

In vielen Fällen ist der Zusatz von Adjuvantien, beispielsweise von Ölen, speziellen Solventien, Tensiden oder Tensidgemischen vorteilhaft. Adjuvantien sind im Pflanzenschutz Hilfsstoffe, welche die Aktivität eines Wirkstoffs (hier: Pflanzenschutzmittel) und/oder seine Selektivität in Bezug auf die erwünschte Wirkung (z.B. fungizide, insektizide, herbizide, bakterizide, nematizide, wachstumsregulatorische Wirkung) erhöhen, die jedoch für sich genommen keine oder nur eine sehr geringe Wirkung bezüglich der Pflanzenschutzwirkung aufweisen. Die Wirkung von Adjuvantien beruht vielfach auf ihrer Grenzflächenaktivität, die den Kontakt der Applikationsform des Wirkstoffs mit der Pflanzenoberfläche verbessert sowie durch Verringerung der Oberflächenspannung das Eindringen der Applikationsform und damit des Wirkstoffs in das Erdreich verbessert.

In einer bevorzugten Ausführungsform liegt das erfindungsgemäße Pflanzenschutzmittel selbst gelförmig vor.

Wenn der wenigstens eine Wirkstoff in der gelförmigen Phase vorliegt, so ist er in der Phase in einer Gesamtmenge von vorzugsweise 2 bis 60 Gew.-%, besonders bevorzugt von 5 bis 50 Gew.-% und insbesondere von 10 bis 40 Gew.-%, bezogen auf das Gesamtgewicht der Phase, enthalten.

Wenn das wenigstens eine Adjuvans in der gelförmigen Phase vorliegt, so ist es in der Phase in einer Gesamtmenge von vorzugsweise 2 bis 50 Gew.-%, besonders bevorzugt von 5 bis 40 Gew.-% und insbesondere von 10 bis 30 Gew.-%, bezogen auf das Gesamtgewicht der Phase, enthalten.

Durch die Einbettung in ein Gel kann der Wirkstoff und/oder das Adjuvans in deutlich größeren Mengen in der erfindungsgemäßen Gelformulierung enthalten sein als dies in üblichen Flüssigformulierungen möglich ist.

Das erfindungsgemäße Pflanzenschutzmittel kann zusätzlich wenigstens einen Verdicker enthalten. Geeignete Verdicker sind die zuvor als Komponente f) genannten, worauf hier in vollem Umfang Bezug genommen wird. Das erfindungsgemäße Pflanzenschutzmittel enthält Verdicker vorzugsweise in einer Menge von 0,002 bis 10 Gew.-%, besonders bevorzugt von 0,02 bis 10 Gew.-%, stärker bevorzugt von 0,04 bis 2 Gew.-%, insbesondere von 0,1 bis 1 Gew.-% und speziell von 0,1 bis 0,6 Gew.-%, bezogen auf das Gesamtgewicht des Pflanzenschutzmittels.

Neben der erfindungsgemäßen gelförmigen Polymerzusammensetzung, wenigstens einem Wirkstoff, gegebenenfalls wenigstens einem Adjuvans und gegebenenfalls wenigstens einem Verdicker kann das erfindungsgemäße Pflanzenschutzmittel weitere Komponenten enthalten. Hierzu gehören beispielsweise Dispergiermittel, Entschäumer, Konservierungsmittel, Biozide, organische Lösungsmittel, Frostschutzmittel und dergleichen.

Geeignete Dispergiermittel oder oberflächenaktive Mittel sind vorzugsweise nicht ionisch. Grundsätzlich können sie von ähnlicher Natur wie die die oben beschriebenen Adjuvantien sein, wobei sie sich von diesen durch ein in der Regel höheres Molekulargewicht unterscheiden. Geeignete Molekulargewichte für Dispergiermittel bewegen sich im Rahmen von ca.1000 g/mol (z.B. phosphatiertes Tristyrylphenolethoxylat, wie Soprophor 3D33 von Rhodia) bis zu mehreren tausend g/mol bei Kammpolymeren (z.B. Atlox 4913 von Croda). Entschäumer sind vorzugsweise Silikon-basierte Entschäumer. Ein geeignetes Konservierungsmittel ist beispielsweise Dichlorophen. Geeignete Biozide sind beispielsweise Isothiazolinone, wie Benzylisothiazolinon und Methylisothiazolinon. Geeignete Frostschutzmittel sind beispielsweise Glycerin, Ethylenglycol und Propylenglycol.

In gleicher Weise können auch veterinärmedizinische Darreichungsformen, insbesondere Rumen-stabile Formen, sowie Darreichungsformen mit Vitaminen, Carotinoiden, Spurenelementen, Nutraceuticals, Aminosäuren und Nahrungsergänzungsstoffen hergestellt werden. Letztere können auch als Lebensmittel oder Supplements gelten.

Ein weiterer Gegenstand der Erfindung ist die Verwendung einer erfindungsgemäßen Polymerzusammensetzung , wie zuvor definiert, zur Membranherstellung, in der Kosmetik, im Pflanzenschutz, für Saatgutcoating, in Lebensmitteln, in Tiernahrung, als Klebrohstoffe, zu Papierherstellung, als Binde- oder Hilfsmittel für Leder und Textil, als mikrobizide Oberflächenbeschichtung, im Vliesstoffbereich, in Wasch- und Reinigungsmittel, zur Herstellung von Anstrichen, im Bausektor.

Die Erfindung wird anhand der im Folgenden beschriebenen Figuren Beispiele näher erläutert. Dabei sollen die Figuren und Beispiele nicht als einschränkend für die Erfindung verstanden werden.

In den nachfolgenden Figuren werden folgende Abkürzungen verwendet:
EO: Ethylenoxid
PO: Propylenoxid
BO: Butylenoxid
Mₙ: Zahlenmittleres Molekulargewicht
M_{w}: Gewichtsmittleres Molekulargewicht
PET: Polyethylenterephthalat
T_{L}-Wert: Wert für die Lichtdurchlässigkeit
n.b.: nicht bestimmt
rad/s: Radiant pro Sekunde (radiant per second)
pphm: Gewichtsteile pro 100 Gewichtsteile Monomer (parts per hundred monomer).

### FIGURENBESCHREIBUNG

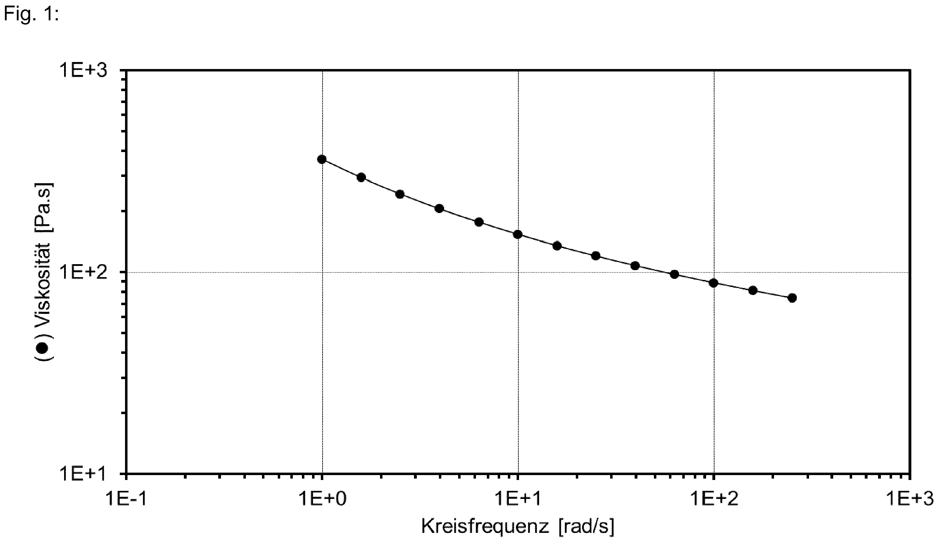

Figur 1 zeigt die Viskosität einer Probe der Polymerzusammensetzung aus Beispiel 19 der Tabelle 1 in Abhängigkeit von der Kreisfrequenz.

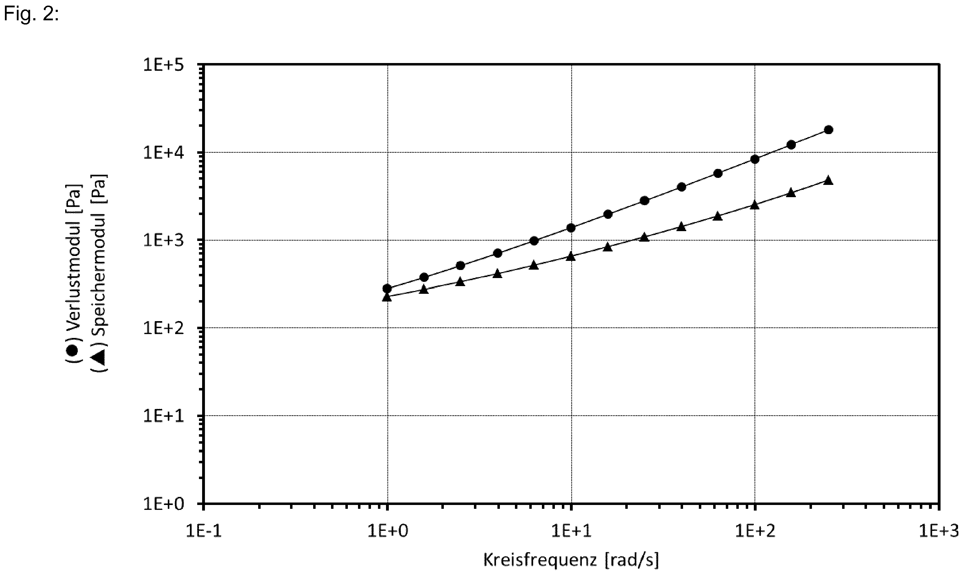

Figur 2 zeigt das Verlust- und Speichermodul einer Probe der Polymerzusammensetzung aus Beispiel 19 der Tabelle 1 in Abhängigkeit von der Kreisfrequenz.

### BEISPIELE

### I) Analytik:

### I.a) Bestimmung der Wasserlöslichkeit

Zur Bestimmung der Wasserlöslichkeit wurden 5 g der jeweiligen Polymerzusammensetzung in ein 1 L Becherglas gegeben und 900 mL Wasser, welches zuvor auf 40°C erhitzt wurde, zugegeben. Die Mischung wurde 20 Minuten lang mit einem Magnetrührer bei 40°C gerührt und der pH-Wert mit Natronlauge auf 8 eingestellt. Wasserlösliche Polymergele führten zu transparenten oder leicht trüben Lösungen.

### I.b) Bestimmung des gewichtsgemittelten Molekulargewichts (M_{w}):

Das gewichstsgemittelte Molekulargewicht des Polymers wurde durch Gelpermeationschromatographie (GPC) bestimmt. Dazu wurden die folgenden Geräte und Chromatographiemethoden verwendet:

Standard: Polyacrylsäure, neutralisiert
Elutionsmittel: 0,08 Mol/L Tris, pH 7,0, + 0,15 Mol/L Na Cl + 0,01 Mol/L NaN₃ in
deionisiertem Wasser
Fluss: 0,8 mL/min
Säulensatz: 1 Vorsäule (l = 5 cm), 2Trennsäulen (l = je 30 cm)
Säulentemperatur: 35°C
Detektor: DRI (Refractive Index Detector) Agilent 1100

### I.c) Viskositätsmessungen

Messgerät: Rotationsrheometer DHR-1 der Firma TA-Instruments mit Peltiersystem
Messgeometrie : Platte-Platte, Ø 40 mm, h ∼1,0 mm
Test: Frequenz-Sweep @ 25°C
Messtemperatur(en) : 25°C
Messzeit : ca. 20 min

### II) Herstellungsbeispiele:

### Allgemeine Herstellungsvorschrift:

In einem Glasreaktor, ausgestattet mit drei Zuläufen, Stickstoffeinlass und einem Ankerrührer, wurde die Polyetherkomponente (PE) und das Lösungsmittel (LM) in einer Menge gemäß Tabelle 1 vorgelegt, für ein paar Minuten mit Stickstoff gespült und auf 75°C erwärmt. Zur Vorlage wurden anschließend bei 75°C und unter Rühren bei 100 Umdrehungen/Minute innerhalb von 4 Stunden die Zuläufe 1 bis 3 simultan zugegeben. Zulauf 1 enthielt Monomer (M), Zulauf 2 enthielt einen Starter (S), gelöst in einer kleinen Menge Lösungsmittel (LM) und der Zulauf 3 enthielt den Regler (R) und gegebenenfalls weiteres Lösungsmittel. Nach der Zugabe der Zuläufe 1, 2 und 3 wurde die Mischung zur Nachpolymerisation für eine weitere Stunde bei 75°C und bei 100 Umdrehungen/Minute gerührt. Anschließend wurde das Polymerisat in ein Becherglas gegossen und sofort auf Raumtemperatur abgekühlt. Die Herstellung der erfindungsgemäßen Polymerzusammensetzungen der Beispiele 1 bis 19 erfolgte nach dieser allgemeinen Herstellungsvorschrift.

Zur Herstellung der erfindungsgemäßen Polymerzusammensetzungen wurden folgende Einsatzstoffe verwendet:
- M1:: Acrylsäure
- M2:: Methacrylsäure
- M3:: Ethyldiglycolacrylat
- M4:: 4-Hydroxybutylacrylat
- M5:: Acrylamid

- PE1:: beidseitig endständig CₓH₂ₓ₊₁/C_{y}H_{2y+1}-terminiertes Polyethylenoxid mit einer freien OH-Gruppe und x, y = 6 - 14
- PE2:: C₁₃-Oxoalkoholethoxylat mit 20 EO
- PE3:: propoxyliertes Ethylenglycol mit einem EO-Anteil von 50%
- PE4:: Polyethylenglycol, Molekulargewicht von 400 g/mol
- PE5:: Polypropylenglycol, Molekulargewicht von 400 g/mol
- PE6:: Alkylpolyglucosid

- R1:: 2-Mercaptoethanol
- R2:: Natriumhypophosphit, 55 %ige wässrige Lösung

- LM1:: Wasser
- LM2:: Isopropanol

- S1:: tert-Butyl peroxyneodekanoat (Reinheit: 97%) (CAS-Nr. 26748-41-4)
- S2:: 2,2'-Azobis(2-methylpropionamidine)dihydrochloride (CAS-Nr. 2997-92-4)

Die Polymerzusammensetzung der Beispiele 1 bis 19 wurden in Form von Gelen erhalten. Die Konsistenz und Eigenschaften sind in Tabelle 2 angegeben. Die Beispiele 17 und 18 sind nicht erfindungsgemäß.

**Tabelle 1**

| **Bsp.-Nr.** | **Vorlage** | | **Zulauf 1** | **Zulauf 2** | | **Zulauf 3** | |
|---|---|---|---|---|---|---|---|
| | **PE [g] (PE-Nr.)** | **LM [g] (LM-Nr.)** | **M [g]** | **S [g] (S-Nr.)** | **LM [g] (LM-Nr.)** | **R [g]** | **LM [g] (LM-Nr.)** |
| 1 | 120 (1) | 162 (1) | 360 (1) | 8.45 (1) | 30 (2) | 18 (1) | / |
| 2 | 120 (1) | 162 (1) | 297 (1) + 63 (2) | 9.1 (2) | 41 (1) | 18 (1) | / |
| 3 | 120 (1) | 162 (1) | 252 (1) + 108 (2) | 9.1 (2) | 41 (1) | 18 (1) | / |
| 4 | 120 (1) | 162 (1) | 164 (1) + 196 (2) | 9.1 (2) | 41 (1) | 18 (1) | / |
| 5 | 200 (1) | 263 (1) | 158 (1) + 442 (2) | 15.2 (2) | 75 (1) | 30 (1) | / |
| 6 | 120 (1) | 162 (1) | 360 (2) | 9.1 (2) | 41 (1) | 18 (1) | / |
| 7 | 320 (1) | 162 (1) | 146 (1) + 174 (2) | 8.1 (2) | 42 (1) | 16 (1) | / |
| 8 | 160 (1) | 162 (1) | 146 (1) + 174 (2) | 8.1 (2) | 42 (1) | 16 (1) | / |
| 9 | 150 (1) | 243 (1) | 273 (1) + 327 (2) | 15.2 (2) | 75 (1) | 30 (1) | / |
| 10 | 120 (1) | 231 (1) | 273 (1) + 327 (2) | 15.2 (2) | 75 (1) | 30 (1) | / |
| 11 | 250 (1) | 247 (1) | 387 (1) + 113 (3) | 3.55 (2) | 46 (1) | 10 (1) + 27 (2) | 28(1) |
| 12 | 250 (1) | 247 (1) | 409 (1) + 91 (4) | 3.55 (2) | 46 (1) | 10 (1) + 27 (2) | 28 (1) |
| 13 | 250 (1) | 198 (1) | 451 (1) + 99 (5) | 3.55 (2) | 46 (1) | 10 (1) + 27 (2) | 28 (1) |
| 14 | 250 (2) | 247 (1) | 500 (1) | 3.55 (2) | 46 (1) | 10 (1) + 27 (2) | 28(1) |
| 15 | 250 (3) | 247 (1) | 500 (1) | 3.55 (2) | 46 (1) | 10 (1) + 27 (2) | 28 (1) |
| 16 | 250 (4) | 247 (1) | 500 (1) | 3.55 (2) | 46 (1) | 10 (1) + 27 (2) | 28 (1) |
| 17 | 250 (5) | 247 (1) | 500 (1) | 3.55 (2) | 46 (1) | 10 (1) + 27 (2) | 28 (1) |
| 18 | 250 (6) | 247 (1) | 500 (1) | 3.55 (2) | 46 (1) | 10 (1) + 27 (2) | 28 (1) |
| 19 | 200 (1) | 285 (1) | 600 (1) | 4.26 (2) | 41 (1) | 12 (1) + 33 (2) | 25 (1) |

**Tabelle 2**

| **Bsp.-Nr.** | **Mw [g/mol]** | **Form** | **Optische Beurteilung** |
|---|---|---|---|
| 1 | 5700 | Gel | Transparent |
| 2 | 3800 | Gel | Transparent |
| 3 | 4200 | Gel | Transparent |
| 4 | 3500 | Gel | Transparent |
| 5 | 4100 | Gel | Transparent |
| 6 | 3500 | Gel | Transparent |
| 7 | 3400 | Gel | Transparent |
| 8 | 3700 | Gel | Transparent |
| 9 | 4200 | Gel | Transparent |
| 10 | 4500 | Gel | Transparent |
| 11 | 4800 | Gel | Transparent |
| 12 | 5700 | Gel | Transparent |
| 13 | 5500 | Gel | Transparent |
| 14 | 5400 | Gel | Transparent |
| 15 | 5500 | Gel | Transparent |
| 16 | 5800 | Gel | Transparent |
| 17 | 5700 | Gel | Transparent |
| 18 | 6800 | Gel | Transparent |
| 19 | 6200 | Gel | Transparent |

## Patentansprüche

1. Verfahren zur Herstellung einer gelförmigen Polymerzusammensetzung, bei dem man
a) eine Monomerzusammensetzung M) bereitstellt, die
A) wenigstens eine α,β-ethylenisch ungesättigte Carbonsäure, und
B) weniger als 0,1 Gew.-%, bezogen auf das Gesamtgewicht der Monomerzusammensetzung M) vernetzend wirkende Monomere, die zwei oder mehr als zwei polymerisierbare α,β-ethylenisch ungesättigte Doppelbindungen pro Molekül aufweisen,
enthält,
wobei die Komponente A) teilweise oder vollständig durch C) wenigstens eine ungesättigte Sulfonsäure oder ungesättigte Phosphonsäure ersetzt sein kann,
b) die in Schritt a) bereitgestellte Monomerzusammensetzung M) einer radikalischen Polymerisation in Gegenwart wenigstens einer Polyetherkomponente PE) unterzieht, die ausgewählt ist unter Polyetherolen mit einem zahlenmittleren Molekulargewicht von wenigstens 200 g/mol und deren Mono- und Di-(C₁-C₆-alkylethern), Polyethergruppenhaltigen Tensiden und Mischungen davon, wobei, wenn die Polyetherkomponente PE) ein Polyetherol mit Propylenoxid-Wiederholungseinheiten oder einen Mono- oder Di-(C₁-C₆-alkylether) eines Polyetherols mit Propylenoxid-Wiederholungseinheiten umfasst, der Anteil dieser Propylenoxid-Wiederholungseinheiten im Mittel höchstens 15 Einheiten pro Molekül beträgt, wobei die Polyetherole PE) Ethylenoxid-Homopolymere oder Ethylenoxid/Propylenoxid-Copolymere sind.

2. Verfahren nach Anspruch 1, wobei die gelförmige Polymerzusammensetzung wenigstens 10 Gew.-%, bevorzugt wenigstens 15 Gew.-%, insbesondere wenigstens 20 Gew.-%, bezogen auf das Gesamtgewicht der Polymerzusammensetzung, wenigstens eines Lösungsmittels LM) enthält, das ausgewählt ist unter Wasser, C₁-C₆-Alkanolen, von PE) verschiedenen Polyolen, deren Mono- und Dialkylethern, aprotisch polaren Lösungsmitteln und Mischungen davon.

3. Verfahren nach Anspruch 2, wobei das Lösungsmittels LM) ausgewählt ist unter Propylenglycol, Dipropylenglycol, Wasser und Mischungen davon.

4. Verfahren nach einem der vorhergehenden Ansprüche, wobei die α,β-ethylenisch ungesättigte Carbonsäure A) ausgewählt ist unter Acrylsäure, Methacrylsäure, Ethacrylsäure, α-Chloracrylsäure, Crotonsäure, Maleinsäure, Itaconsäure, Citraconsäure, Mesaconsäure, Glutaconsäure, Aconitsäure, Fumarsäure und Mischungen davon.

5. Verfahren nach einem der vorhergehenden Ansprüche, wobei die Monomerzusammensetzung M) zusätzlich als Komponente C) wenigstens eine ungesättigte Sulfonsäure oder ungesättigte Phosphonsäure enthält, vorzugsweise ausgewählt unter 2-Acrylamido-2-methylpropansulfonsäure, Vinylsulfonsäure, Allylsulfonsäure, Sulfoethylacrylat, Sulfoethylmethacrylat, Sulfopropylacrylat, Sulfopropylmethacrylat, 2-Hydroxy-3-acryloxypropylsulfonsäure, 2-Hydroxy-3-methacryloxypropylsulfonsäure, Styrolsulfonsäure, Vinylphosphonsäure, Allylphosphonsäure sowie Mischungen davon.

6. Verfahren nach einem der vorhergehenden Ansprüche, wobei die Monomerzusammensetzung M) zusätzlich wenigstens ein Monomer D) enthält, ausgewählt unter Verbindungen der allgemeinen Formeln (I.a) und (I.b) 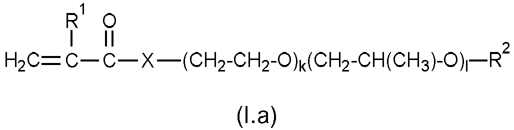
H₂C=CH-CH₂-O-(CH₂-CH₂-O)ₖ(CH₂-CH(CH₃)-O)ₗ-R² (I.b)
worin
die Reihenfolge der Alkylenoxideinheiten beliebig ist,
k und l unabhängig voneinander für eine ganze Zahl von 0 bis 1000 stehen, wobei die Summe aus k und l mindestens 2 beträgt, bevorzugt mindestens 5 beträgt,
R¹ für Wasserstoff oder C₁-C₈-Alkyl steht,
R² für Wasserstoff, C₁-C₃₀-Alkyl, C₂-C₃₀-Alkenyl oder C₅-C₈-Cycloalkyl steht,
X für O oder eine Gruppe der Formel NR³ steht, worin R³ für H, Alkyl, Alkenyl, Cycloalkyl, Heterocycloalkyl, Aryl oder Hetaryl steht.

7. Verfahren nach einem der vorhergehenden Ansprüche, wobei die Monomerzusammensetzung M) zusätzlich wenigstens ein Comonomer enthält, ausgewählt unter
E) Vinylaromaten,
F) C₂-C₈ Monoolefinen, nichtaromatischen Kohlenwasserstoffen mit wenigstens zwei konjugierten Doppelbindungen,
G) Estern α,β-ethylenisch ungesättigter Mono- und Dicarbonsäuren mit C₁-C₃₀-Alkanolen,
H) Verbindungen mit einer radikalisch polymerisierbaren α,β-ethylenisch ungesättigten Doppelbindung und wenigstens einer kationogenen und/oder kationischen Gruppe pro Molekül,
I) Estern von Vinylalkohol oder Allylalkohol mit C₁-C₃₀-Monocarbonsäuren,
K) Amidgruppen-haltigen Monomeren,
L) Estern α,β-ethylenisch ungesättigter Mono- und Dicarbonsäuren mit C₂-C₃o-Alkanediolen, Amiden α,β-ethylenisch ungesättigter Mono- und Dicarbonsäuren mit C₂-C₃₀-Aminoalkoholen mit einer primären oder sekundären Aminogruppe,
M) α,β-ethylenisch ungesättigten Nitrilen,
N) Vinylhalogeniden, Vinylidenhalogeniden,
O) Monomeren mit Harnstoffgruppen,
und Mischungen davon.

8. Verfahren nach einem der vorhergehenden Ansprüche, wobei die Monomerzusammensetzung M) ausschließlich aus Acrylsäure besteht.

9. Verfahren nach einem der vorhergehenden Ansprüche, wobei die Polyetherkomponente PE) wenigstens ein Polyetherol mit einem zahlenmittleres Molekulargewicht im Bereich von etwa 200 bis 100000 g/mol oder einen Mono- oder Di-(C₁-C₂-alkylether) davon umfasst.

10. Verfahren nach einem der vorhergehenden Ansprüche, wobei die Polyetherkomponente PE) wenigstens ein Polyethergruppen-haltiges Tensid umfasst, ausgewählt unter Alkylpolyoxyalkylenethern, Arylpolyoxyalkylenethern, Alkylarylpolyoxyalkylenethern, alkoxylierten tierischen und/oder pflanzlichen Fette und/oder Ölen, Fettaminalkoxylaten, Fettsäureamidalkoxylaten, Fettsäurediethanolamidalkoxylaten, Polyoxyethylensorbitanfettsäureestern, Alkylpolyethersulfaten, Arylpolyethersulfaten, Alkylarylpolyethersulfaten, Alkylpolyethersulfonaten, Arylpolyethersulfonaten, Alkylarylpolyethersulfonaten, Alkylpolyetherphosphaten, Arylpolyetherphosphaten, Alkylarylpolyetherphosphaten, Glycerinethersulfonaten, Glycerinethersulfaten, Monoglycerid(ether)sulfaten, Fettsäureamidethersulfaten, Polyoxyalkylensorbitanfettsäureestern und Mischungen davon.

11. Gelförmige Polymerzusammensetzung, erhältlich durch ein Verfahren wie in einem der Ansprüche 1 bis 10 definiert.

12. Gelförmige Polymerzusammensetzung, erhältlich durch ein Verfahren wie in einem der Ansprüche 1 bis 10 definiert, in Form eines transparenten Gels.

13. Verwendung einer Polymerzusammensetzung, wie in einem der Ansprüche 11 oder 12 definiert oder erhältlich durch ein Verfahren wie in einem der Ansprüche 1 bis 10 definiert,
- in Wasch- und Reinigungsmitteln,
- in Hygieneprodukten,
- in kosmetischen Mitteln,
- in pharmazeutischen Mitteln,
- in Pflanzenschutzmitteln,
- in Netzmitteln,
- in Lacken, Beschichtungsmitteln, Klebstoffen, Lederbehandlungs- oder Textilbehandlungsmitteln, etc.,
- bei der Erschließung und/oder Ausbeutung unterirdischer Erdöl- und/oder Erdgaslagerstätten.

14. Wasch- und Reinigungsmittel, umfassend:
a) wenigstens eine erfindungsgemäße gelförmige Polymerzusammensetzung, wie einem der Ansprüche 11 oder 12 definiert oder erhältlich durch ein Verfahren wie in einem der Ansprüche 1 bis 10 definiert,
b) wenigstens einen Builder (auch als Sequestrierungsmittel, Gerüststoff, Komplexbildner, Chelator, Chelatisierungsmittel oder Enthärter bezeichnet),
c) gegebenenfalls wenigstens ein Enzym,
d) gegebenenfalls wenigstens ein Bleichmittel,
e) gegebenenfalls Wasser,
f) gegebenenfalls wenigstens einen Verdicker, und
g) gegebenenfalls wenigstens einen weiteren Zusatzstoff, der vorzugsweise ausgewählt ist unter von a) verschiedenen Tensiden, Basen, Korrosionsinhibitoren, Entschäumern, Farbstoffen, Duftstoffen, Füllstoffen, Löslichkeitsvermittlern, organischen Lösemitteln, Elektrolyten, pH-Stellmitteln, Parfümträgern, Fluoreszenzmitteln, Hydrotropen, Antiredepositionsmitteln, optischen Aufhellern, Vergrauungsinhibitoren, Einlaufverhinderern, Knitterschutzmitteln, Farbübertragungsinhibitoren, antimikrobiellen Wirkstoffen, Antioxidantien, Korrosionsinhibitoren, Antistatika, Bügelhilfsmitteln, Phobier- und Imprägniermitteln, Quell- und Schiebefestmitteln und UV-Absorbern.

15. Klebstoffzusammensetzung, umfassend oder bestehend aus einer Polymerzusammensetzung, wie in einem der Ansprüche 11 oder 12 definiert oder erhältlich durch ein Verfahren wie in einem der Ansprüche 1 bis 10 definiert.

## Claims

1. A process for preparing a polymer composition in gel form, in which
a) a monomer composition M) is provided, comprising
A) at least one α,β-ethylenically unsaturated carboxylic acid, and
B) less than 0.1% by weight, based on the total weight of the monomer composition M), of crosslinking monomers having two or more than two polymerizable α,β-ethylenically unsaturated double bonds per molecule,
where component A) may be partly or fully replaced by C) at least one unsaturated sulfonic acid or unsaturated phosphonic acid,
b) the monomer composition M) provided in step a) is subjected to a free-radical polymerization in the presence of at least one polyether component PE) selected from polyetherols having a number-average molecular weight of at least 200 g/mol and the mono- and di-(C₁-C₆-alkyl ethers) thereof, surfactants containing polyether groups and mixtures thereof, where, if the polyether component PE) comprises a polyetherol having repeat propylene oxide units or a mono- or di-(C₁-C₆-alkyl ether) of a polyetherol having repeat propylene oxide units, the proportion of these repeat propylene oxide units averages not more than 15 units per molecule, where the polyetherols PE) are ethylene oxide homopolymers or ethylene oxide/propylene oxide copolymers.

2. The process according to claim 1, wherein the polymer composition in gel form comprises at least 10% by weight, preferably at least 15% by weight, especially at least 20% by weight, based on the total weight of the polymer composition, of at least one solvent S) selected from water, C₁-C₆-alkanols, polyols other than PE), the mono- and dialkyl ethers thereof, aprotic polar solvents and mixtures thereof.

3. The process according to claim 2, wherein the solvent S) is selected from propylene glycol, dipropylene glycol, water and mixtures thereof.

4. The process according to any of the preceding claims, wherein the α,β-ethylenically unsaturated carboxylic acid A) is selected from acrylic acid, methacrylic acid, ethacrylic acid, α-chloroacrylic acid, crotonic acid, maleic acid, itaconic acid, citraconic acid, mesaconic acid, glutaconic acid, aconitic acid, fumaric acid and mixtures thereof.

5. The process according to any of the preceding claims, wherein the monomer composition M) additionally comprises, as component C), at least one unsaturated sulfonic acid or unsaturated phosphonic acid, preferably selected from 2-acrylamido-2-methylpropanesulfonic acid, vinylsulfonic acid, allylsulfonic acid, sulfoethyl acrylate, sulfoethyl methacrylate, sulfopropyl acrylate, sulfopropyl methacrylate, 2-hydroxy-3-acryloyloxypropylsulfonic acid, 2-hydroxy-3-methacryloyloxypropylsulfonic acid, styrenesulfonic acid, vinylphosphonic acid, allylphosphonic acid and mixtures thereof.

6. The process according to any of the preceding claims, wherein the monomer composition M) additionally comprises at least one monomer D) selected from compounds of the general formulae (I.a) and (I.b) 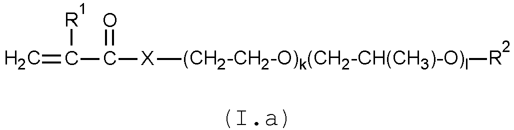
H₂C=CH-CH₂-O-(CH₂-CH₂-O)ₖ(CH₂-CH(CH₃)-O)l-R² (I.b)
in which
the sequence of the alkylene oxide units is arbitrary,
k and l are each independently an integer from 0 to 1000, where the sum of k and l is at least 2, preferably at least 5,
R¹ is hydrogen or C₁-C₈-alkyl,
R² is hydrogen, C₁-C₃₀-alkyl, C₂-C₃₀-alkenyl or C₅-C₈-cycloalkyl,
X is O or a group of the formula NR³ in which R³ is H, alkyl, alkenyl, cycloalkyl, heterocycloalkyl, aryl or hetaryl.

7. The process according to any of the preceding claims, wherein the monomer composition M) additionally comprises at least one comonomer selected from
E) vinylaromatics,
F) C₂-C₈ monoolefins, nonaromatic hydrocarbons having at least two conjugated double bonds,
G) esters of α,β-ethylenically unsaturated mono- and dicarboxylic acids with C₁-C₃₀-alkanols,
H) compounds having one free-radically polymerizable α,β-ethylenically unsaturated double bond and at least one cationogenic and/or cationic group per molecule,
I) esters of vinyl alcohol or allyl alcohol with C₁-C₃₀-monocarboxylic acids,
K) monomers containing amide groups,
L) esters of α,β-ethylenically unsaturated mono- and dicarboxylic acids with C₂-C₃₀-alkanediols, amides of α,β-ethylenically unsaturated mono- and dicarboxylic acids with C₂-C₃₀-amino alcohols having a primary or secondary amino group,
M) α,β-ethylenically unsaturated nitriles,
N) vinyl halides, vinylidene halides,
O) monomers having urea groups,
and mixtures thereof.

8. The process according to any of the preceding claims, wherein the monomer composition M) consists exclusively of acrylic acid.

9. The process according to any of the preceding claims, wherein the polyether component PE) comprises at least one polyetherol having a number-average molecular weight in the range from about 200 to 100 000 g/mol or a mono- or di-(C₁-C₂-alkyl ether) thereof.

10. The process according to any of the preceding claims, wherein the polyether component PE) comprises at least one surfactant containing polyether groups, selected from alkyl polyoxyalkylene ethers, aryl polyoxyalkylene ethers, alkylaryl polyoxyalkylene ethers, alkoxylated animal and/or vegetable fats and/or oils, fatty amine alkoxylates, fatty acid amide alkoxylates, fatty acid diethanolamide alkoxylates, polyoxyethylene sorbitan fatty acid esters, alkyl polyether sulfates, aryl polyether sulfates, alkylaryl polyether sulfates, alkyl polyether sulfonates, aryl polyether sulfonates, alkylaryl polyether sulfonates, alkyl polyether phosphates, aryl polyether phosphates, alkylaryl polyether phosphates, glyceryl ether sulfonates, glyceryl ether sulfates, monoglyceride (ether) sulfates, fatty acid amide ether sulfates, polyoxyalkylene sorbitan fatty acid esters and mixtures thereof.

11. A polymer composition in gel form, obtainable by a process as defined in any of claims 1 to 10.

12. A polymer composition in gel form, obtainable by a process as defined in any of claims 1 to 10, in the form of a transparent gel.

13. The use of a polymer composition as defined in either of claims 11 and 12 or obtainable by a process as defined in any of claims 1 to 10
- in washing and cleaning compositions,
- in hygiene products,
- in cosmetic compositions,
- in pharmaceutical compositions,
- in crop protection compositions,
- in wetting agents,
- in lacquers, coating compositions, adhesives, leather treatment compositions or textile care compositions, etc.,
- in the development and/or exploitation of underground mineral oil and/or natural gas deposits.

14. A washing and cleaning composition comprising:
a) at least one inventive polymer composition in the form of a gel as defined in either of claims 11 and 12 or obtainable by a process as defined in any of claims 1 to 10,
b) at least one builder (also referred to as sequestrant, builder material, complexing agent, chelator, chelating agent or softener),
c) optionally at least one enzyme,
d) optionally at least one bleach,
e) optionally water,
f) optionally at least one thickener, and
g) optionally at least one further additive, preferably selected from the following additives other than a): surfactants, bases, corrosion inhibitors, defoamers, dyes, fragrances, fillers, solubilizers, organic solvents, electrolytes, pH modifiers, perfume carriers, fluorescers, hydrotropes, antiredeposition agents, optical brighteners, graying inhibitors, shrink inhibitors, crease inhibitors, dye transfer inhibitors, antimicrobials, antioxidants, corrosion inhibitors, antistats, ironing aids, hydrophobizing and impregnating agents, antiswell and antislip agents, and UV absorbers.

15. An adhesive composition comprising or consisting of a polymer composition as defined in either of claims 11 and 12 or obtainable by a process as defined in any of claims 1 to 10.

## Revendications

1. Procédé pour la préparation d'une composition de polymère sous forme de gel, dans lequel
a) on met à disposition une composition M) de monomères qui contient
A) au moins un acide carboxylique éthyléniquement α,β-insaturé, et
B) moins de 0,1 % en poids, par rapport au poids total de la composition M) de monomères, de monomères à effet réticulant, qui présentent deux ou plus de deux doubles liaisons éthyléniquement α,β-insaturées polymérisables par molécule,
le composant A) pouvant être remplacé partiellement ou totalement par C) au moins un acide sulfonique insaturé ou au moins un acide phosphonique insaturé,
b) on soumet la composition M) de monomères mise à disposition dans l'étape a) à une polymérisation radicalaire en présence d'au moins un composant de type polyéther PE), qui est choisi parmi des polyétherols dotés d'un poids moléculaire moyen en nombre d'au moins 200 g/mole et leurs mono-(C₁-₆-alkyléthers) et di-(C₁₋₆-alkyléthers), des tensioactifs contenant des groupes polyéther et des mélanges correspondants, où, lorsque le composant de type polyéther PE) comprend un polyétherol comportant des motifs répétitifs de type oxyde de propylène ou un mono- (C₁₋₆-alkyléther) ou un di-(C₁₋₆-alkyléther) d'un polyétherol comportant des motifs répétitifs de type oxyde de propylène, la proportion de ces motifs répétitifs de type oxyde de propylène est en moyenne d'au plus 15 motifs par molécule, les polyétherols PE) étant des homopolymères d'oxyde d'éthylène ou des copolymères oxyde d'éthylène/oxyde de propylène.

2. Procédé selon la revendication 1, la composition de polymère sous forme de gel contient au moins 10 % en poids, préférablement au moins 15 % en poids, en particulier au moins 20 % en poids, par rapport au poids total de la composition de polymère, d'au moins un solvant LM), qui est choisi parmi l'eau, les C₁₋₆-alcanols, des polyols différents de PE), leurs monoalkyléthers et dialkyléthers, des solvants polaires aprotiques et des mélanges correspondants.

3. Procédé selon la revendication 2, le solvant LM) étant choisi parmi le propylèneglycol, le dipropylèneglycol, l'eau et des mélanges correspondants.

4. Procédé selon l'une quelconque des revendications précédentes, l'acide carboxylique éthyléniquement α,β-insaturé A) étant choisi parmi l'acide acrylique, l'acide méthacrylique, l'acide éthacrylique, l'acide α-chloroacrylique, l'acide crotonique, l'acide maléique, l'acide itaconique, l'acide citraconique, l'acide mésaconique, l'acide glutaconique, l'acide aconitique, l'acide fumarique et des mélanges correspondants.

5. Procédé selon l'une quelconque des revendications précédentes, la composition M) de monomères contenant de plus en tant que composant C), au moins un acide sulfonique insaturé ou au moins un acide phosphonique insaturé, de préférence choisi parmi l'acide 2-acrylamido-2-méthylpropanesulfonique, l'acide vinylsulfonique, l'acide allylsulfonique, l'acrylate de sulfoéthyle, le méthacrylate de sulfoéthyle, l'acrylate de sulfopropyle, le méthacrylate de sulfopropyle, l'acide 2-hydroxy-3-acryloxypropylsulfonique, l'acide 2-hydroxy-3-méthacryloxypropylsulfonique, l'acide styrènesulfonique, l'acide vinylphosphonique, l'acide allylphosphonique ainsi que des mélanges correspondants.

6. Procédé selon l'une quelconque des revendications précédentes, la composition M) de monomères contenant de plus au moins un monomère D), choisi parmi des composés de formules générales (I.a) et (I.b) 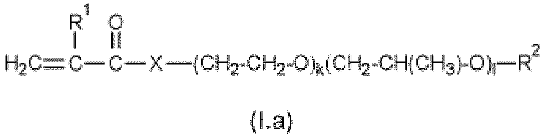
H₂C=CH--CH₂-O-(CH₂-CH₂-O)ₖ(CH₂-CH(CH₃)-O)ₗ-R² (I.b)
dans lesquelles
l'ordre des motifs de type oxyde d'alkylène est arbitraire,
k et l représentent indépendamment l'un de l'autre un nombre entier de 0 à 1 000, la somme de k et l étant d'au moins 2, préférablement d'au moins 5,
R¹ représente hydrogène ou C₁₋₈-alkyle,
R² représente hydrogène, C₁₋₃₀-alkyle, C₂₋₃₀-alcényle ou C₅₋₈-cycloalkyle,
X représente O ou un groupe de formule NR³, dans laquelle R³ représente H, alkyle, alcényle, cycloalkyle, hétérocycloalkyle, aryle ou hétaryle.

7. Procédé selon l'une quelconque des revendications précédentes, la composition M) de monomères contenant de plus au moins un comonomère, choisi parmi
E) des composés aromatiques vinyliques,
F) des monooléfines en C₂-₈, des hydrocarbures non aromatiques comportant au moins deux doubles liaisons conjuguées,
G) des esters d'acides monocarboxyliques et dicarboxyliques éthyléniquement α,β-insaturés avec des C₁₋₃₀-alcanols,
H) des composés comportant une double liaison éthyléniquement α,β-insaturée polymérisable par voie radicalaire et au moins un groupe cationogène et/ou cationique par molécule,
I) des esters d'alcool vinylique ou d'alcool allylique avec des acides monocarboxyliques en C₁₋₃₀,
K) des monomères contenant des groupes amide,
L) des esters d'acides monocarboxyliques et dicarboxyliques éthyléniquement α,β-insaturés avec des C₂₋₃₀-alcanediols, des amides d'acides monocarboxyliques et dicarboxyliques éthyléniquement α,β-insaturés avec des C₂₋₃₀-aminoalcools dotés d'un groupe amino primaire ou secondaire,
M) des nitriles éthyléniquement α,β-insaturés,
N) des halogénures de vinyle, des halogénures de vinylidène,
O) des monomères comportant des groupes urée,
et des mélanges correspondants.

8. Procédé selon l'une quelconque des revendications précédentes, la composition M) de monomères étant exclusivement constituée d'acide acrylique.

9. Procédé selon l'une quelconque des revendications précédentes, le composant de type polyéther PE) comprenant au moins un polyétherol doté d'un poids moléculaire moyen en nombre dans la plage d'environ 200 à 100 000 g/mole ou un mono-(C₁₋₂-alkyléther) ou un di-(C₁₋₂-alkyléther) correspondant.

10. Procédé selon l'une quelconque des revendications précédentes, le composant de type polyéther PE) comprenant au moins un tensioactif contenant des groupes polyéther, choisi parmi les alkylpolyoxyalkylèneéthers, les arylpolyoxyalkylèneéthers, les alkylarylpolyoxyalkylèneéthers, les graisses et/ou les huiles alcoxylées animales et/ou végétales, les alcoxylates d'amines grasses, les alcoxylates d'amides d'acides gras, les alcoxylates de diéthanolamides d'acides gras, les esters d'acides gras de polyoxyéthylènesorbitane, les alkylpolyéthersulfates, les arylpolyéthersulfates, les alkylarylpolyéthersulfates, les alkylpolyéthersulfonates, les arylpolyéthersulfonates, les alkylarylpolyéthersulonfates, les alkylpolyétherphosphates, les arylpolyétherphosphates, les alkylarylpolyétherphosphates, les glycérineéthersulfonates, les glycérineéthersulfates, les monoglycéride(éther)sulfates, les éthersulfates d'amides d'acides gras, les esters d'acides gras de polyoxyalkylènesorbitane et des mélanges correspondants.

11. Composition de polymère sous forme de gel, pouvant être obtenue par un procédé tel que défini selon l'une quelconque des revendications 1 à 10.

12. Composition de polymère sous forme de gel, pouvant être obtenue par un procédé tel que défini dans l'une quelconque des revendications 1 à 10, sous forme d'un gel transparent.

13. Utilisation d'une composition de polymère, telle que définie dans l'une quelconque des revendications 11 et 12 ou pouvant être obtenue par un procédé tel que défini dans l'une quelconque des revendications 1 à 10,
- dans des produits de lavage et de nettoyage,
- dans des produits d'hygiène,
- dans des produits cosmétiques,
- dans des produits pharmaceutiques,
- dans des produits phytosanitaires,
- dans des agents mouillants,
- dans des vernis, des agents de revêtement, des adhésifs, des produits de traitement du cuir ou de traitement de textiles, etc.,
- lors de l'aménagement et/ou de l'exploitation de gisements souterrains de pétrole et/ou de gaz naturel.

14. Produit de lavage et de nettoyage, comprenant :
a) au moins une composition de polymère sous forme de gel selon l'invention, telle que définie dans l'une quelconque des revendications 11 et 12 ou pouvant être obtenue par un procédé tel que défini dans l'une quelconque des revendications 1 à 10,
b) au moins un renforçateur (également désigné comme agent séquestrant, adjuvant, agent complexant, chélateur, agent de chélation ou adoucisseur),
c) éventuellement au moins une enzyme,
d) éventuellement au moins un agent de blanchiment,
e) éventuellement de l'eau,
f) éventuellement au moins un épaississant, et
g) éventuellement au moins un additif supplémentaire, qui est de préférence choisi parmi des tensioactifs, des bases, des inhibiteurs de corrosion, des antimousses, des colorants, des fragrances, des charges, des agents solubilisants, des solvants organiques, des électrolytes, des agents d'ajustement du pH, des supports de parfum, des agents fluorescents, des hydrotropes, des produits anti-redéposition, des azurants optiques, des inhibiteurs de grisage, des agents empêchant le rétrécissement, des agents de protection contre les plis, des inhibiteurs de transfert de couleur, des principes actifs antimicrobiens, des antioxydants, des inhibiteurs de corrosion, des antistatiques, des auxiliaires de repassage, des insectifuges et des agents d'imprégnation, des agents anti-gonflement et anti-éraillants et des absorbants UV différents de a).

15. Composition d'adhésif, comprenant ou étant constituée d'une composition de polymère telle que définie dans l'une quelconque des revendications 11 et 12 ou pouvant être obtenue par un procédé tel que défini dans l'une quelconque des revendications 1 à 10.
